(19) **Europäisches Patentamt / European Patent Office / Office européen des brevets**

(11) **EP 3 262 417 B1**

(12) **EUROPEAN PATENT SPECIFICATION**

(45) Date of publication and mention
of the grant of the patent:
**03.11.2021 Bulletin 2021/44**

(21) Application number: **16756221.4**

(22) Date of filing: **23.02.2016**

(51) Int Cl.:
*G01N 33/574* (2006.01)   *G06N 3/04* (2006.01)
*G06N 5/00* (2006.01)   *G06N 5/04* (2006.01)
*G06N 7/00* (2006.01)   *G01N 33/50* (2006.01)
*G06N 20/20* (2019.01)   *G06K 9/00* (2006.01)
*G06N 3/08* (2006.01)

(86) International application number:
**PCT/US2016/019198**

(87) International publication number:
**WO 2016/138041 (01.09.2016 Gazette 2016/35)**

(54) **CELL IMAGING AND ANALYSIS TO DIFFERENTIATE CLINICALLY RELEVANT SUB-POPULATIONS OF CELLS**

ZELLBILDGEBUNG UND ANALYSE ZUR DIFFERENZIERUNG KLINISCH RELEVANTER SUBPOPULATIONEN VON ZELLEN

ANALYSE ET IMAGERIE DE CELLULES POUR DIFFÉRENCIER CLINIQUEMENT DES SOUS-POPULATIONS IMPORTANTES DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.02.2015 US 201562119726 P**
**08.09.2015 US 201562215654 P**
**18.11.2015 US 201562257154 P**

(43) Date of publication of application:
**03.01.2018 Bulletin 2018/01**

(73) Proprietor: **Cellanyx Diagnostics, LLC
Beverly, Massachusetts 01915 (US)**

(72) Inventors:
• **CHANDER, Ashok**
  **Boston, Massachusetts 02118 (US)**
• **SU, Wendell**
  **Beverly, Massachusetts 01915 (US)**
• **VARSANIK, Jonathan**
  **Brookline, Massachusetts 02446 (US)**

(74) Representative: **Hafner & Kohl PartmbB
Schleiermacherstraße 25
90491 Nürnberg (DE)**

(56) References cited:
WO-A1-2013/181127   WO-A2-2012/048269
WO-A2-2013/030175   US-A1- 2004 153 249
US-A1- 2006 099 624

## Description

### FIELD

Methods related to the field of medical testing/diagnostics, cell-based assays, and compound discovery are provided herein. In various aspects, methods are provided for the determination of the local growth, and/or, oncogenic, and/or local adverse pathology potential, migration rate, and/or, metastatic potential and/or metastatic adverse pathology potential of mammalian cells or patient's cells (e.g., cells obtained from biopsy). In some aspects, microfluidic tissue disassociation, cell, protein, and particle separation, cell manipulation, and assay methods for using the same are provided. Exemplary applications include but are not limited to diagnostic and cell based assays.

### BACKGROUND

Primary cell culture allows for the study of native tissue samples derived from an organism. Culturing cells derived from organisms, can be useful and necessary for applications such as medical diagnostics, cell-based assays, compound discovery and characterization such as stratifying patients during clinical trials.

For example, cancer diagnosis and identification of compounds for treatment of cancer are of great interest due to the widespread occurrence of the diseases, high death rate, and recurrence after treatment. According to National Vital Statistics Reports, from 2002 to 2006 the rate of incidence (per 100,000 persons) of cancer in Caucasians was 470.6, in people of African descent 493.6, in Asians 31 1.1, and Hispanics 350.6, indicating that cancer is wide- spread among all races. Lung cancer, breast cancer and prostate cancer were the three leading causes of death in the US, claiming over 227,900 lives in 2007 according to the NCI.

Survival of a cancer patient depends heavily on detection. As such, developing technologies applicable for sensitive and specific methods to detect cancer is an inevitable task for cancer researchers. Existing cancer screening methods include: (1) the Papanicolau test for women to detect cervical cancer and mammography to detect breast cancer; (2) prostate-specific antigen (PSA) level detection in blood sample for men to detect prostate cancer; (3) occult blood detection for colon cancer; (4) endoscopy, CT scans, X-ray, ultrasound imaging and MRI for various cancer detection; and (5) Gleason score for prostate cancer. These traditional diagnostic methods however are not very powerful, providing only sub-optimal sensitivity and specificity statistics when it comes to cancer detection at very early stages and give little prognostic information. Moreover, some of the screening methods are quite costly and not available for many people. Moreover, detection technologies suffer from a variety of shortcomings such as specificity and sensitivity that leads to overtreatment or late detection. Prostate cancer detection is one example where over-treatment affects 144,000 patients annually in the U.S. due to the lack of clinical tools for risk stratification, costing about $4.9 billion annually in the US alone in overtreatment.

Likewise, existing methods for cancer staging are often qualitative and therefore limited in applicability. For example, diagnoses made by different physicians or of different patients using existing methods such as a Gleason Score for prostate cancer can be difficult to compare in a meaningful manner due to the subjective nature of these methods. As a result, the subjectivity of the existing methods of cancer staging often results in overly aggressive treatment strategies. By way of example, in the absence of better data, the most drastic, potentially invasive, strategy is often recommended, which can lead to overtreatment, poor patient quality of life, and increased medical costs.

One method to detect and/or characterize cancer, for example, is to directly assess living tissue derived from small biopsy samples taken from suspicious tissue. To get a relevant and useful sense of the biological characteristics of tissue, one would be well served by being able to culture biopsy tissue in vitro.

Therefore, the development of technology that is specific and reliable for culturing primary human tissue and/or detecting and characterizing a cancer (e.g., determining the local growth, local adverse pathology, oncogenic, migration rate, and/or metastatic, and/or metastatic adverse pathology potential of cells obtained from a patient) is an area of significant importance. Likewise, there remains a need for improved systems, methods, and devices for diagnostic cell-based assays and compound discovery.

WO 2012/048269 A2 discloses a method for determining a property of a cell.

WO 2013/1811127 A1 discloses a method for providing a composite image of a single tissue sample from a lung cancer patient.

### SUMMARY

The invention is defined by the appended claims. In certain embodiments, a method for evaluating the status of a cell in a sample is provided, comprising: disposing the cell on an extracellular matrix (ECM); capturing multiple images of the cell within a plurality of cells as the cells interact with the ECM over a pre-defined time period in a sample obtained from a subject over a pre-defined time period; evaluating the multiple images of the cell to identify or measure a pre-selected biomarker; identifying the cell as normal or an outlier within the plurality of cells based on the identification or measurement of the pre-selected biomarker; wherein if the cell is identified as an outlier, subjecting the identified cell or measured biomarker in the outlier to a machine learning analysis thereby creating a cell level output indicator; and combining two or more cell level output indicators to create a prognostic indicator for the sample. The sample often comprises a plurality of live cells obtained from culturing live cells present in a sample obtained from the subject. In certain embodiments, the prognostic indicator comprises a single number or indication. The evaluation of the multiple images is, in frequent embodiments, performed utilizing computer or machine vision. Often, the diagnosis or prognosis comprises a cancer diagnosis or prognosis, for example a prostate cancer, bladder cancer, lung cancer, kidney cancer, breast cancer, ovarian cancer, uterine cancer, colon cancer, thyroid cancer, or skin cancer.

In frequent embodiments, the capturing of multiple images is performed with a machine vision system.

In frequent embodiments, the methods described herein are carried out in an automated manner or using automated systems.

In certain embodiments, the images comprise direct images of the cell. Often, the images are captured while the cell is alive and moving. Also often, the images identify cellular or subcellular structures, aspects, or processes measuring about 0.25 micron in size or larger. In certain embodiments, the images identify cellular or subcellular structures, aspects, or processes measuring about 1.0 micron in size or larger.

Often, the pre-selected biomarker comprises a plurality of biomarkers. Also often, two or more of the pre-selected biomarkers are used in the identification of the cell as normal or an outlier. Frequently, two or more of the pre-selected biomarkers are subjected to the machine learning analysis. In certain frequent embodiments, up to five of the pre-selected biomarkers are subjected to the machine learning analysis. In certain embodiments, five or more of the pre-selected biomarkers are subjected to the machine learning analysis. In other certain embodiments, 17 to 26 of the pre-selected biomarkers are subjected to the machine learning analysis. In other certain embodiments, 45 to 65 of the pre-selected biomarkers are subjected to the machine learning analysis. In other certain embodiments, 17 or more of the pre-selected biomarkers are subjected to the machine learning analysis. In other certain embodiments, up to 65 of the pre-selected biomarkers are subjected to the machine learning analysis. In other certain embodiments, 2 to 26 pre-selected biomarkers are subjected to the machine learning analysis. In other certain embodiments, 10 to 20 pre-selected biomarkers are subjected to the machine learning analysis. In other certain embodiments, 4 to 25 pre-selected biomarkers are subjected to the machine learning analysis. In other certain embodiments, 3 to 15 pre-selected biomarkers are subjected to the machine learning analysis. In other certain embodiments, 5 to 10 pre-selected biomarkers are subjected to the machine learning analysis. In other certain embodiments, 17 to 45 pre-selected biomarkers are subjected to the machine learning analysis. In other certain embodiments, 2 to 17 pre-selected biomarkers are subjected to the machine learning analysis. In certain embodiments, the number and type of biomarkers selected are based on a ranking of the biomarker in importance relative to other biomarkers for evaluating a pre-selected adverse pathology predictor. Often, the pre-selected adverse pathology predictor is based on the type of tissue or disorder being evaluated. Often, the pre-selected adverse pathology predictor is vascular invasion, seminal vesicle invasion, positive surgical margin, perineural invasion, lymph node positive, extraprostatic extension, grade, lymph invasion, or a selection or combination thereof.

In certain embodiments, the prognostic indicator comprises a diagnosis of the subject. In other certain embodiments, the prognostic indicator comprises a prognosis for the subject. In other certain embodiments, the prognostic indicator comprises a confirmation or adjustment of a diagnosis of the subject or prognosis for the subject. In other certain embodiments, the prognostic indicator is used to modify or confirm a pathological determination for the sample. The prognostic indicator is often utilized to modify or confirm an established clinical nomogram, tumor grade, cancer staging or grading system, or pathological score used for diagnosis and /or prognosis (e.g., Gleason Score). The prognostic indicator is often used to modify or confirm a Gleason Score determination for the sample. In other certain embodiments, the prognostic indicator is used to modify or confirm a Nottingham Score determination for the sample.

In certain embodiments, the sample comprises a sample of cells from a prostate tissue, a bladder tissue, a lung tissue, a kidney tissue, a breast tissue, an ovarian tissue, a uterine tissue, a colon tissue, a thyroid tissue, a skin tissue. In other certain embodiments, the sample comprises a blood or bone marrow sample. In certain embodiments, the sample comprises a urine sample containing cells of interest. In a related embodiment, the sample is a first-catch post-DRE urine sample. Most frequently, the cell is a live cell. In certain embodiments, the cell is a fixed cell. In certain embodiments, the cell is evaluated in both live and (subsequently) fixed forms.

In frequent embodiments, wherein the evaluating step occurs concurrently or after the contact of a reagent with the cell or medium containing the cell. The reagent often comprises a diagnostic reagent, or a small molecule or large molecule drug. The prognostic indicator in such embodiments often provides an indication of the reaction of the sample to the presence of the small or large molecule drug. In certain embodiments, the method does not include the combining step and the cell level output indicator provides an indication of the reaction of the cell to the presence of the small or large molecule drug.

In certain embodiments, the machine learning analysis comprises a weighted decision tree, a bootstrap aggregated decision tree, a neural network, a linear discriminator, a non-linear discriminator, or a combination thereof of any two or more machine learning analysis. Often, a supervised, a semi-supervised, and/or an unsupervised machine learning method is used to identify the cell as a normal or an outlier. Also often, the machine learning analysis comprises a supervised, a semi-supervised, and/or an unsupervised machine learning method comprising a clustering method. When a clustering method is utilized, it is frequently selected from: k-means, hierarchical (e.g., single linkage, conceptual, etc.) clustering, fuzzy clustering, expectation-maximizing clustering, density-based spatial clustering of applications with noise (DBSCAN), ordering points to identify the clustering structure (OPTICS), or a combination thereof of any two or more supervised, semi-supervised, and/or unsupervised machine learning methods. In certain embodiments, the combining step comprises an application of a machine learning classifier to the identified or measured biomarker of each cell in the plurality of cells. Often, the identifying step comprises an application of a clustering method to an identified or measured biomarker in the cell. In certain embodiments, the machine learning analysis comprises a weighted decision tree, wherein the decision tree comprises nodes and leaves, the nodes containing attributes of a respective biomarker input and the leaves containing a classification function and the connections between the nodes of the decision tree are weighted.

Often, beads are not used when the images are captured.

In certain frequent embodiments, a computer-implemented method is provided, comprising: receiving, by a staging system, a plurality of images for generating predictors, each image specifying a type of biomarker identified in a cell by the staging system and criteria for identifying a biomarker that is normal or an outlier; for each image associated with a type of biomarker, generating, by the staging system, a predictor for the type of biomarker, the generating comprising identifying a training data set comprising a plurality of cells exhibiting biomarkers having both normal and outlier characteristics; training one or more candidate predictors using the identified training data set, wherein each candidate predictor comprises a machine learned model; and optionally evaluating a performance of each candidate predictor by executing each predictor on a test data set comprising live cells exhibiting biomarkers having both normal and outlier characteristics; and returning a designation corresponding to the generated predictor to a requester of the selected predictor.

In certain embodiments, the candidate predictor is a machine learning model of a type based on one of a decision tree, a bootstrap aggregated decision tree, a neural network, a linear discriminator, or a non-linear discriminator. In frequent embodiments, the computer-implemented method further comprises receiving a request for a predictor from a process running in the staging system, the request specifying the designation and an image of a live cell; executing the predictor corresponding to the specified designation on the image of the cell; and returning a result of the predictor to the requesting process.

In frequent embodiments, the identifying step or the evaluating step comprises an application of a clustering method to the biomarkers of the plurality of cells. Often, the staging system comprises an imaging device operably connected with a computer system.

In certain frequent embodiments, a computer-implemented method is provided comprising: storing, by a staging system, a plurality of predictors, each predictor for predicting whether a cell is normal or an outlier, each predictor associated with biomarker criteria for a pre-determined type of normal cell or outlier cell; selecting an existing predictor corresponding to a previously established behavior or characteristic of a source sample; identifying a data set comprising images of a cell on the staging system; evaluating performance of each candidate predictor by executing each predictor on a test data set comprising a plurality of the images of the cell on the staging system; selecting a candidate predictor from the one or more candidate predictors by comparing the performance of the one or more candidate predictors; comparing performance of the selected candidate predictor with performance of the existing predictors; and if the candidate predictor is of a different type than an existing predictor and the performance of the candidate predictor is comparable with or exceeds the performance of one or more existing predictors, adding or replacing the selected candidate predictor to the existing predictors; or if the candidate predictor is of the same type as an existing predictor, reordering the weight of the existing predictor based on the selected candidate predictor responsive to performance of the selected candidate predictor exceeding the performance or inferior to the performance of the existing predictor.

Often, the candidate predictor comprises a machine learning model of a type based on one of a decision tree, a bootstrap aggregated decision tree, a neural network, a linear discriminator, or a non-linear discriminator. Also often, the candidate predictor comprises a clustering method. In certain embodiments, a combination of a clustering method and a machine learning classifier method are utilized in the computer implemented methods described herein.

Also often, the staging system comprises an imaging device operably connected with a computer system.

In frequent embodiments described herein, the behavior of a source sample (or simply a sample) comprises a distinguishable biomarker expression, or expression profile, of the sample. Often, the distinguishable biomarker expression comprises a pathological endpoint in a clinic setting. Frequently, the distinguishable biomarker expression comprises a prognostic indicator. Also frequently, the distinguishable biomarker expression comprises a cell level output or a subject level output.

In frequent embodiments of the computed implemented methods herein, the cell is a live cell. In certain embodiments, the cell is a fixed cell.

Frequently, the imaging device comprises a microscope. Also frequently, the imaging device provides direct imaging a live cell within the internal portion of the microfluidic chamber. Often, wherein the imaging device is capable of identifying and imaging subcellular structures measuring about 1 micron or larger such as a focal adhesion or spreading dynamics.

In certain embodiments, the machine learning algorithm comprises a clustering method. Often, the clustering method is selected from one or more of the following: k-means, hierarchical clustering, fuzzy clustering, expectation-maximizing clustering, DBSCAN, or OPTICS. Also frequently, the computer system further comprises a machine learning classifier or operation thereof in connection with an identified or measured biomarker. The machine learning classifier often comprises a decision tree, a bootstrap aggregated decision tree, a neural network, a linear discriminator, a non-linear discriminator, or a combination of two or more of the foregoing.

Often, the computer system comprises a cell distinguishing and tracking program in operable communication with the imaging output of the imaging device. The cell distinguishing and tracking program is frequently capable of detecting a physical edge of a cell within a population of cells.

Often, the systems described herein are configured to support a chamber comprising cells. Often the cells are live cells. In certain embodiments, the cells are dead or fixed cells.

In frequent embodiments, the systems described herein are used, capable of being used, or configured to be used to image and analyze live cells. In certain embodiments, the cell is a live cell. In certain embodiments, the cell is a fixed cell.

Often the systems are automated systems. Also often, the system comprises computer vision or machine vision.

Often, the cell is obtained from a prostate sample and the prognostic indicator comprises predicting seminal vesicle invasion. Also often, the cell is obtained from a prostate sample and the prognostic indicator, expression profile, or pathology potential determination comprises (predicting) vascular invasion. In frequent embodiments, the cell is obtained from a prostate sample and the prognostic indicator, expression profile, or pathology potential determination comprises (predicting) extra-prostatic extension. Also frequently, the cell is obtained from a prostate sample and the prognostic indicator, expression profile, or pathology potential determination comprises (predicting) positive surgical margins for prostate cancer, often after radical prostatectomy. Often, the cell is obtained from a prostate sample and the prognostic indicator, expression profile, or pathology potential determination comprises (predicting) perineural invasion. Also often, the cell is obtained from a prostate sample and the prognostic indicator, expression profile, or pathology potential determination comprises (predicting) lymph node invasion. The cell in frequent embodiments is obtained from a prostate sample and the prognostic indicator, expression profile, or pathology potential determination comprises (predicting) prostate cancer in tissue adjacent to a tumor site. Also frequently, the cell is obtained from a prostate sample and the prognostic indicator, expression profile, or pathology potential determination comprises (predicting) LAPP and/or MAPP.

The cell also in frequent embodiments is obtained from a breast sample and the prognostic indicator, expression profile, or pathology potential determination comprises (predicting) breast cancer. Often, the sample is evaluated for the presence of HER 2 expression. In frequent embodiments, the cell is obtained from a breast sample and the prognostic indicator, expression profile, or pathology potential determination comprises (predicting) HER 2 expression, grade, lympho-vascular invasion, lymph node invasion, ductal carcinoma *in situ*, lobular carcinoma *in situ*, extra-nodal extension, positive surgical margins, LAPP, and/or MAPP.

Also often, the cell is obtained from a bladder sample and the prognostic indicator, expression profile, or pathology potential determination comprises (predicting) bladder cancer. In frequent embodiments, the cell is obtained from a bladder sample and the prognostic indicator, expression profile, or pathology potential determination comprises (predicting) grade, lymph node invasion, squamous differentiation, glandular differentiation, and/or lymph invasion, LAPP, and/or MAPP.

In certain embodiments, the cell is obtained from a kidney sample and the prognostic indicator, expression profile, or pathology potential determination comprises (predicting) kidney cancer. In frequent embodiments, the cell is obtained from a kidney sample and the prognostic indicator, expression profile, or pathology potential determination comprises (predicting) kidney cancer grade, LAPP, and/or MAPP.

The present methods and systems are most frequently useful for transforming data comprised in an image or depiction of a cell (or population of cells) from or in a sample into one or more metrics useful to determine or adjust a diagnosis, prognosis, or theranosis for a subject. Generally, the cell is removed from its native environment for conducting the present methods and positioned in a fabricated cell chamber on a non-natural substrate. As such, according to the present methods, the analyzed cells are stressed in an unnatural manner to exhibit or express certain predetermined (including newly identified) biomarkers in an unnatural environment. The inventors have identified significant clinical meaning in the identification and measurement of collections of these biomarkers as sets and subsets of data. These data are transformed using methods described herein into clinically actionable metrics that improve patient care. The data transformation described in detail herein was not heretofore possible at least because the raw image data was unknown and/or not accessible apart from methods and devices described herein.

These and other embodiments, features, and advantages will become apparent to those skilled in the art when taken with reference to the following more detailed description of various exemplary embodiments of the present disclosure in conjunction with the accompanying drawings.

**BRIEF DESCRIPTION OF THE DRAWINGS**

The skilled person in the art will understand that the drawings, described below, are for illustration purposes only.

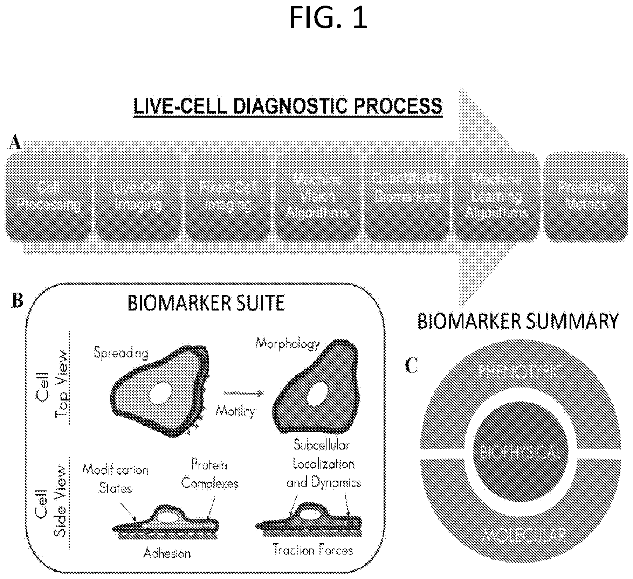

FIG. 1 provides an overview of certain components of the diagnostic platform of the present disclosure, which measures phenotypic, biophysical, and/or molecular biomarkers on live cells harvested from patient tumor samples. FIG. 1A provides a flow diagram outlining a diagnostic process of sample processing, biomarker measurement, algorithmic analysis and generation of predictive measurements. FIG. 1B shows that phenotypic, biophysical, and/or molecular biomarkers are measured on live and fixed samples. FIG. 1C provides a diagram of exemplary biomarkers measured with single cell resolution.

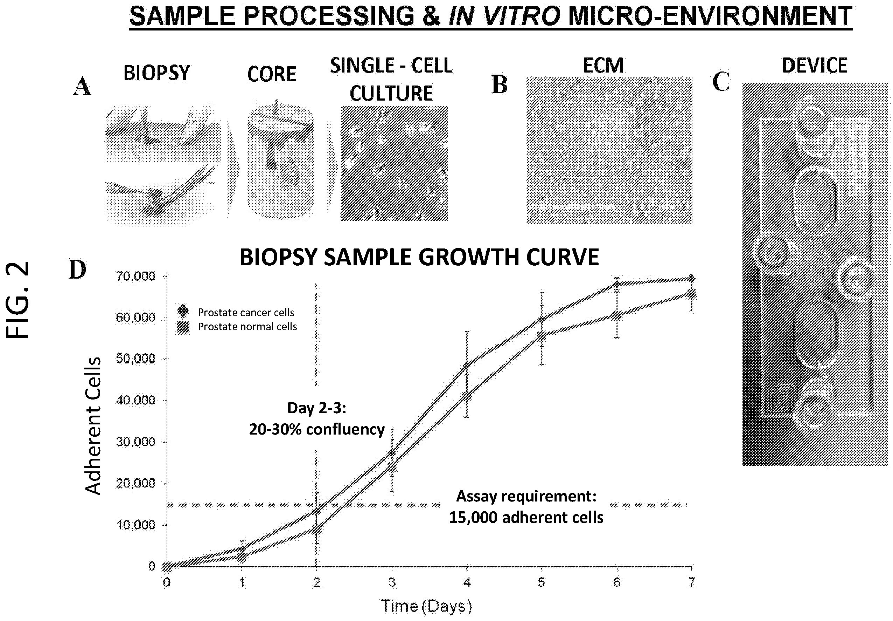

FIG. 2 provides a depiction of certain exemplary procedures conducted on live cells harvested from radical prostatectomy samples prior to cellular analysis according to the processes described herein. FIG. 2A depicts biopsy/surgical samples collected and processed into single cell cultures. FIG. 2B depicts an extra cellular matrix (ECM) formulation used to produce a permissive environment for cell survival and evaluation. FIG. 2C depicts an exemplary microfluidic device used in conjunction with ECM to promote cell survival, as well as automate and standardize biomarker measurement. FIG. 2D depicts an exemplary growth curve of cells derived from patient sample having cells analyzed on day 2.

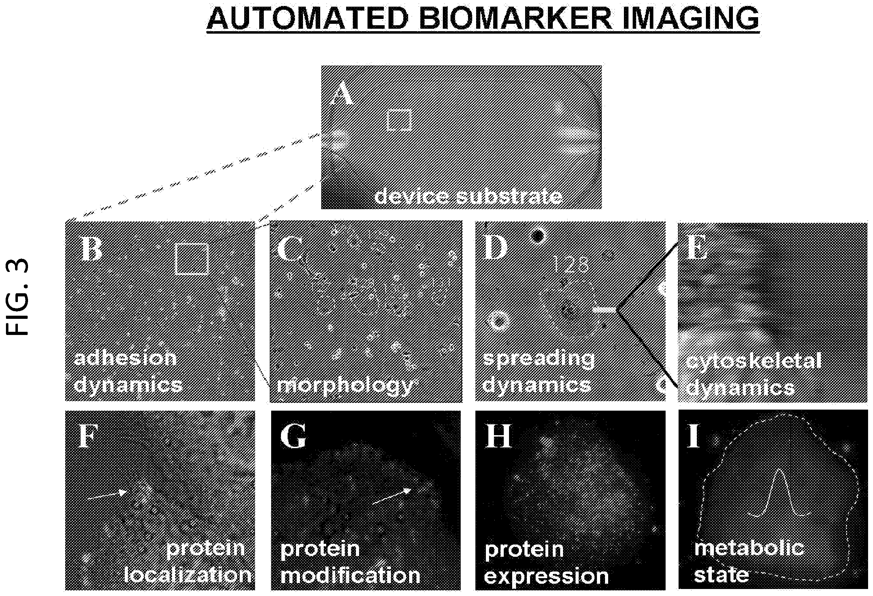

FIG. 3 depicts certain exemplary phenotypic, biophysical, and molecular biomarkers measured using methods and devices of the present disclosure in a microfluidic environment at 20x DIC and 40x fluorescence via an automated fluorescent microscope. FIG. 3A depicts an exemplary cell growth chamber coated with ECM. FIGS. 3B-3I depict the imaging of FIG. 3B - cell adhesion rate to device substrate, FIG. 3C - cellular morphology, FIG. 3D - rate of cell spreading on substrate, FIG. 3E - rapid dynamics of the membrane surface, FIG. 3F - subcellular protein localization, FIG. 3G-subcellular protein modification, FIG. 3H - subcellular protein expression, and FIG. 3I - metabolic activity.

FIG. 4 depicts an exemplary automated process for identifying and tracking cells and biomarkers thereof. FIG. 4A shows a portion of an exemplary procedure where cells are identified and tagged with unique IDs. FIG. 4B depicts the tracking of cell location over time. FIG. 4C depicts the tracking of cell spreading dynamics. FIG. 4D depicts membrane fluctuations measured to identify/measure cytoskeletal dynamics. FIG. 4E depicts the identification and measurement of subcellular protein complexes and protein activation states on fixed, fluorescently stained cells.

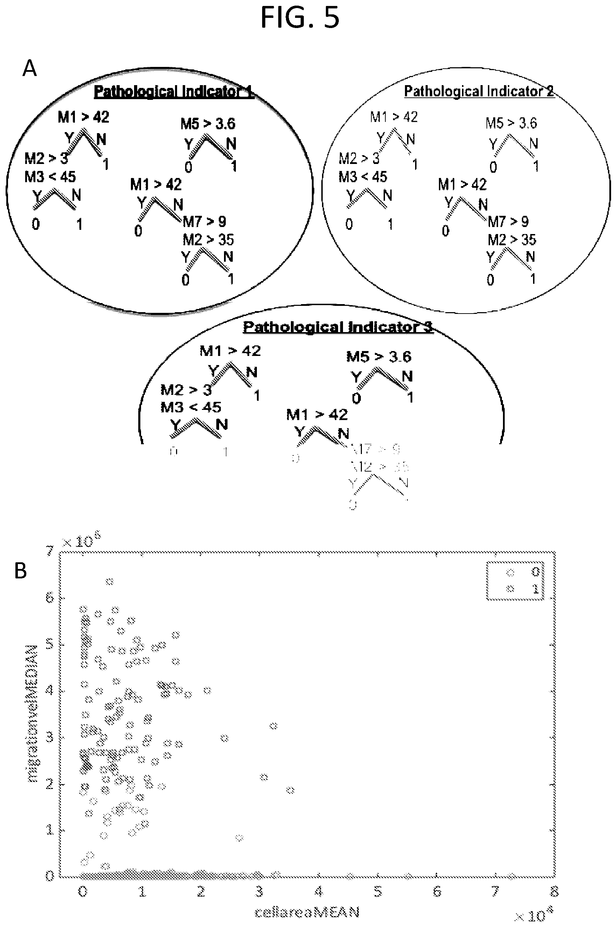

FIG. 5 depicts a table of cells with their corresponding biomarkers. Cells with biomarkers that are outliers (abnormal cells) compared to the norm (or average) are identified (FIG. 5B), isolated, and further analyzed. The abnormal cells are put through a machine learning algorithm, which as depicted here is composed of a collection of previously trained weighted decision trees (FIG. 5A) correlating biomarkers to pathological outcomes. The result is each cell is attributed with a percent likelihood of it having a selected pathological outcome, as further described herein. In practice, as also described herein, these cell-level results are summarized into a patient-level outcome.

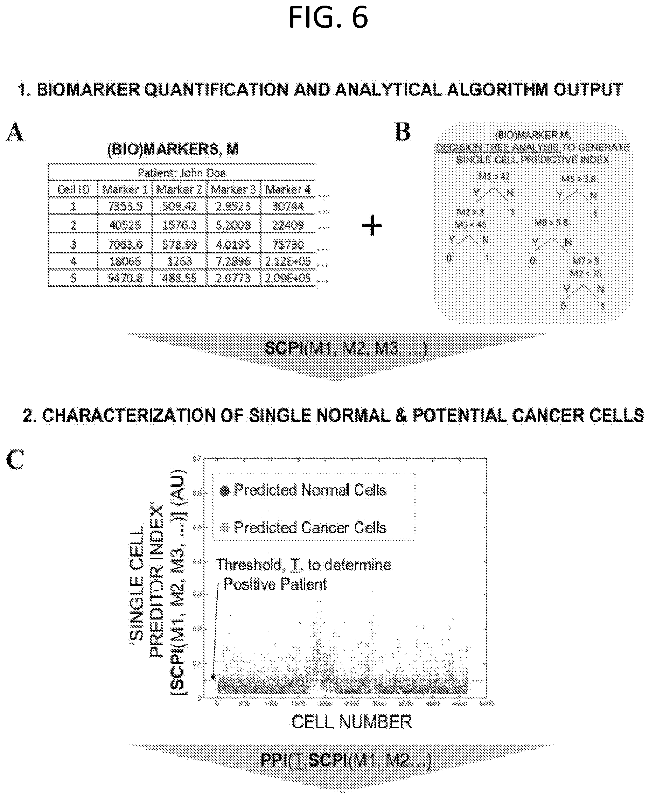

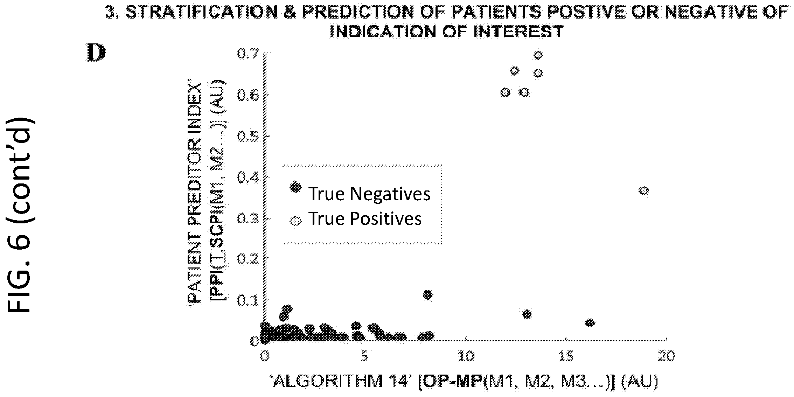

FIG. 6 depicts an exemplary process flow according to the present methods and devices, involving the processing of multiple biomarkers and predicting various pathological outcomes. FIG. 6A depicts a set of four different biomarkers measured for each cell in a patient sample. These marker measurements are input to a machine learning algorithm that generates multiple decision trees (FIG. 6B) that stratifies cells of a negative patient from cells of a positive patient for a given pathological outcome. The decision trees are optionally weighted to optimize algorithm accuracy (FIG. 6B). FIG. 6C depicts a representative plot demonstrating stratification among negative and positive cells utilizing combinations of biomarkers as described by the decision trees. Exemplary patient level results are obtained by summarizing cell level results from FIG. 6C into FIG. 6D, which provides an exemplary plot demonstrating stratification of patients for a given predicted pathology finding.

FIGS. 7A and 7B depict Receiver Operating Characteristics (ROC) curves correlating automated cell analysis according to the methods described herein with clinically relevant pathological indicators.

FIGS. 8A, 8B, and 8C depict exemplary clinical results and comparisons to accepted standards in prostate cancer diagnosis of a number of patients using methods, systems, and devices of the present disclosure.

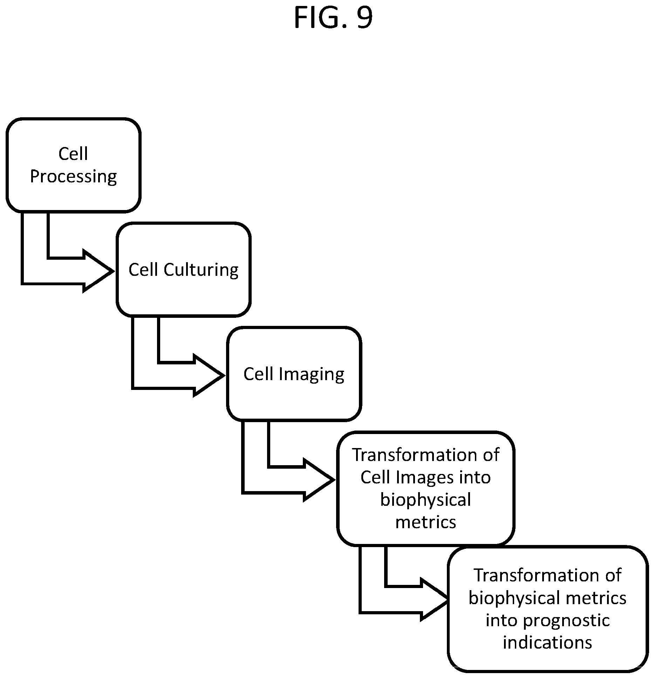

FIG. 9 depicts an example process flowchart for certain aspects of the present disclosure.

FIG. 10 depicts an example imaging process flowchart.

FIG. 11 depicts an example montaging process flowchart.

FIGS. 12A and 12B depict images before and after brightness correction.

FIG. 13 depicts an example cell masking process flowchart.

FIG. 14 depicts the result of a filter process on an image of multiple objects.

FIGS. 15A and 15B depict examples of the application of initial and final thresholds.

FIG. 16 depicts the results of a first stage of a cleanup of invalid objects in the background of an image.

FIG. 17 depicts an example of a final mask prior to being applied to a cell.

FIG. 18 depicts a montaged image having clearly delineated cells after applying the mask to the obj ects.

FIG. 19 depicts an example flowchart of splitting groups of cells apart.

FIG. 20 depicts a continuation of an example flowchart of splitting groups of cells apart.

FIG. 21 depicts a graphical representation of an exemplary watershedding technique.

FIG. 22 depicts a montage having only the nucleus of cells shown.

FIG. 23 depicts segmentation of a group of cells into individually detectable cells.

FIG. 24 depicts an image of segmented cells.

FIG. 25 depicts an exemplary flowchart describing one method of tracking cell movements.

FIG. 26 depicts a continuation of an exemplary flowchart describing one method of tracking cell movements.

FIG. 27 depicts an exemplary flowchart describing a retrograde flow velocity (RFV) measurement.

FIGS. 28A and 28B depict RFV images measurement.

FIG. 29 depicts an exemplary flowchart describing Focal Adhesion measurement.

FIGS. 30A and 30B depict before and after images of FAK analysis.

FIG. 31 depicts an exemplary flowchart describing biomarker analysis.

FIG. 32 depicts a flowchart describing an exemplary abnormal cell identification process flow.

FIG. 33 depicts a flowchart describing an exemplary analysis of abnormal cells with a machine learning method.

FIG. 34 depicts a flowchart describing an exemplary process of combining cell level data to provide a subject level output.

FIG. 35 depicts an exemplary clinical study design and workflow.

FIGS. 36A, 36B, 36C, and 36D depict cell growth, viability and characterization of primary biopsy derived cells.

FIGS. 37A, 37B, 37C, and 37D depict biomarkers quantified to identify and risk stratify tumor cells.

FIGS. 38A, 38B, 38C, and 38D depict risk assessment plots demonstrating an ability to distinctly grade patient samples.

FIG. 39 depicts an exemplary receiver operating characteristic (ROC) curves generated using methods described herein, and numerical representations of accuracy based on the ROC curves.

FIG. 40 depicts another ROC curve, but for a different classification algorithm that can predict adverse pathologies.

FIG. 41 depicts a representation of evaluating suspected cancerous and non-cancerous cells in the sample/analysis.

FIG. 42 depicts a representation of evaluating suspected cancerous and non-cancerous cells in the sample/analysis.

FIG. 43 depicts a representation of evaluating suspected cancerous and non-cancerous cells in the sample/analysis.

FIG. 44 depicts a representation of evaluating suspected cancerous and non-cancerous cells in the sample/analysis.

FIG. 45 depict a ranking of exemplary biomarkers.

FIG. 46 depict classification metrics for multiple biomarkers.

FIG. 47 depicts a ROC curve generated using methods and devices described herein.

FIGS. 48 depicts a ROC curve generated using methods and devices described herein.

FIGS. 49 depicts a ROC curve generated using methods and devices described herein.

FIGS. 50 depicts a ROC curve generated using methods and devices described herein.

FIG. 51 provides an exemplary representation of the present metrics enhancing Gleason score data.

FIG. 52 provides an exemplary representation of the present metrics enhancing Gleason score data.

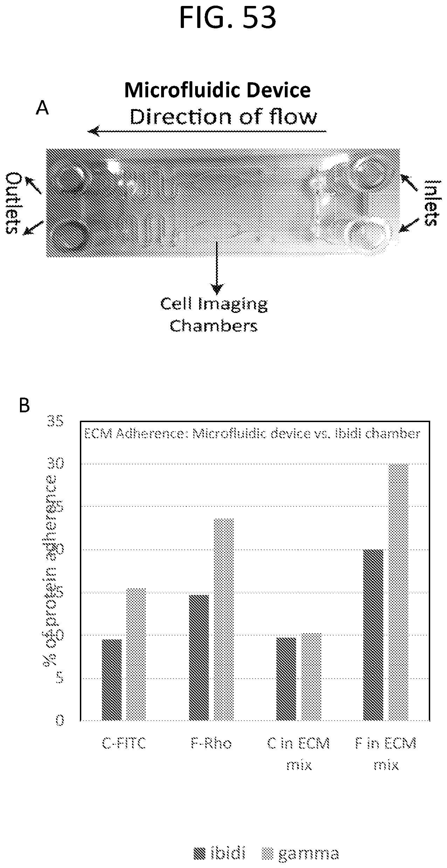

FIGS. 53A and 53B. FIG. 53A depicts an exemplary microfluidic device used in conjunction with ECM to promote cell survival as well as automate and standardize biomarker measurements. FIG. 53B depicts the percentage of ECM protein adhered to the surface of the microfluidic device imaging chamber compared to the ibidi chamber, demonstrating appropriate ECM spreading in the imaging device to support cell growth. FITC conjugated collagen (10 ug/mL) and/or Rhodamine conjugated fibronectin (F-Rho ) (10 $\mu$g/mL)) is added to each chamber. Percentage adherence calculated by comparing fluorescence at the bottom of device after seeding protein (Day 1) vs. after washing protein with PBS (Day 2). In exemplary embodiments, the ECM provides a reference standard by which cellular micro-environment interactions are analyzed.

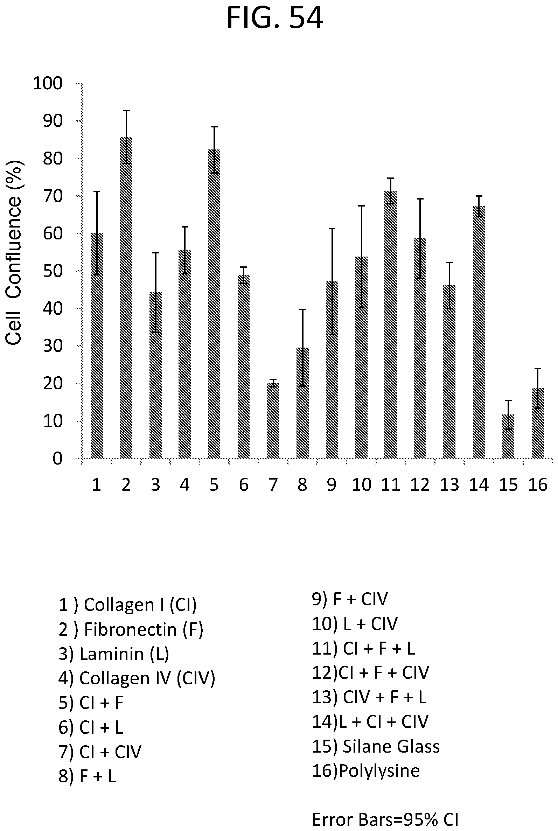

FIG. 54 depicts percentage cell confluence of cells on various ECM surfaces demonstrating that certain ECM formulations (e.g., Collagen I + Fibronectin, 10 $\mu$g/ mL each) allow cell adhesion and robust survival of primary kidney cells compared to other ECM formulations and non-permissive glass surfaces.

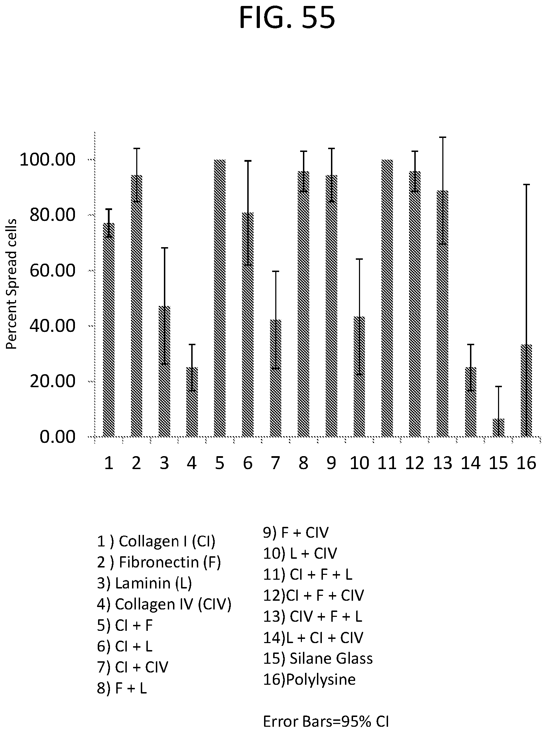

FIG. 55 depicts percentage cell spread on various ECM surfaces demonstrating that certain ECM formulations (e.g., Collagen I + Fibronectin, 10 $\mu$g/ mL each) allow optimal cell adhesion and spread of primary bladder cells.

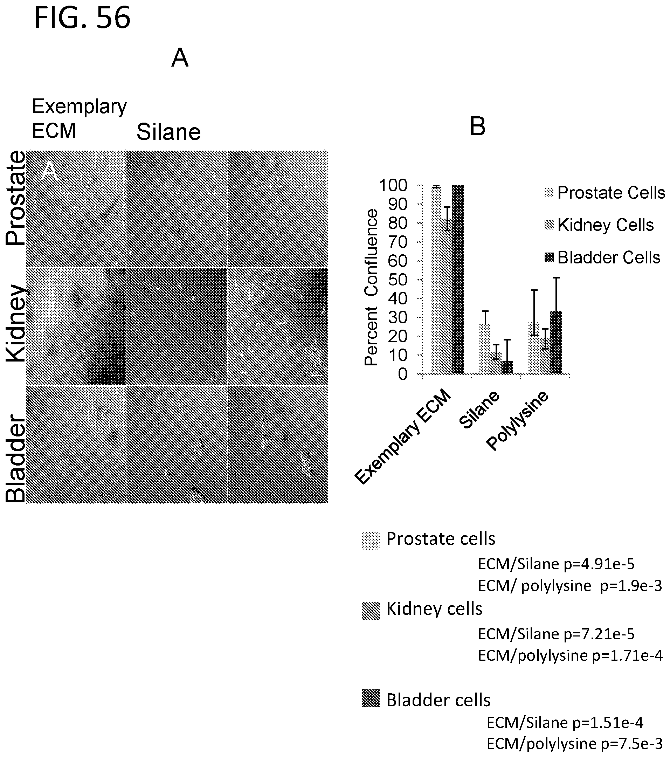

FIG. 56 depicts a comparison of cell confluence/spread of primary prostate, kidney and bladder cells on exemplary ECM (e.g., Collagen I + Fibronectin, 10 $\mu$g/ mL each) vs silane and Poly-L-lysine demonstrating that the exemplary ECM promotes cells spread and growth to confluence.

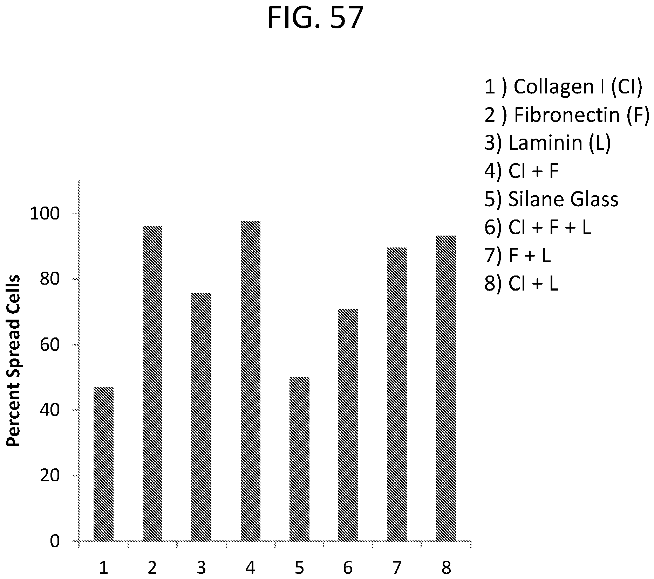

FIG. 57 depicts the percentage of cell spread of primary breast cells on various ECM formulations demonstrating that exemplary ECM formulations (Collagen I + Fibronectin, 10 $\mu$g/ mL each) allow optimal spread of primary breast cells.

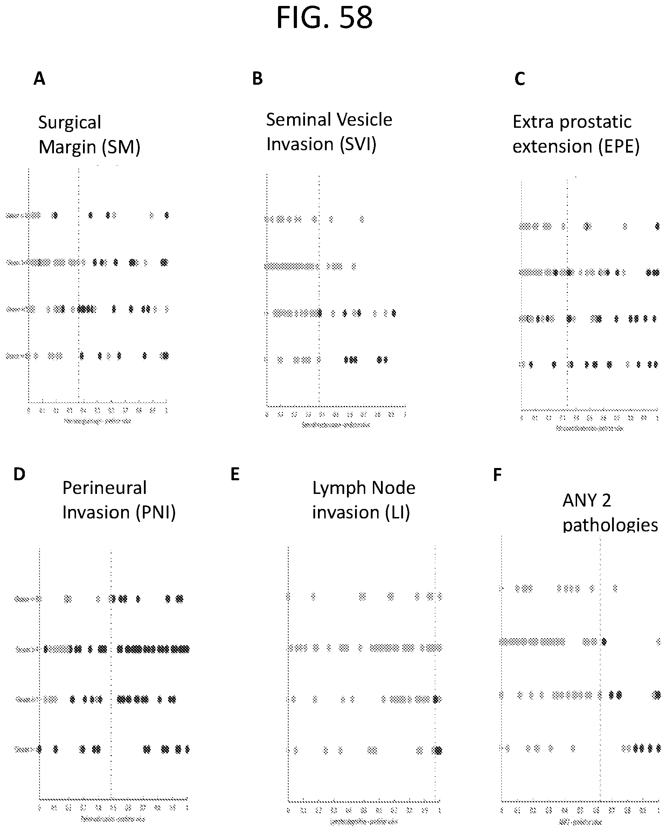

FIG. 58 depicts risk stratification plots showing adverse pathology predictors in patients on the X-axis and clinically assigned Gleason scores on the Y-axis. Each dot represents an individual patient. FIGS. 58A, 58B, 58C, 58D, 58E and 58F are predictor plots for Surgical Margins (SM), Seminal Vesicle Invasion (SVI), Extra Prostatic Extension (EPE), Perineural Invasion (PNI), Lymph Node Invasion (LNI or LI), and ANY 2 pathologies, respectively. Black circles represent individuals tested positive for the pathology, grey circles represent those tested not positive. The dotted grey line is the algorithm-defined operation threshold. Black circles to the right of the threshold are true positives and grey circles to the right are false positives. Black circles to the left of the threshold are false negatives while grey circles are true negatives.

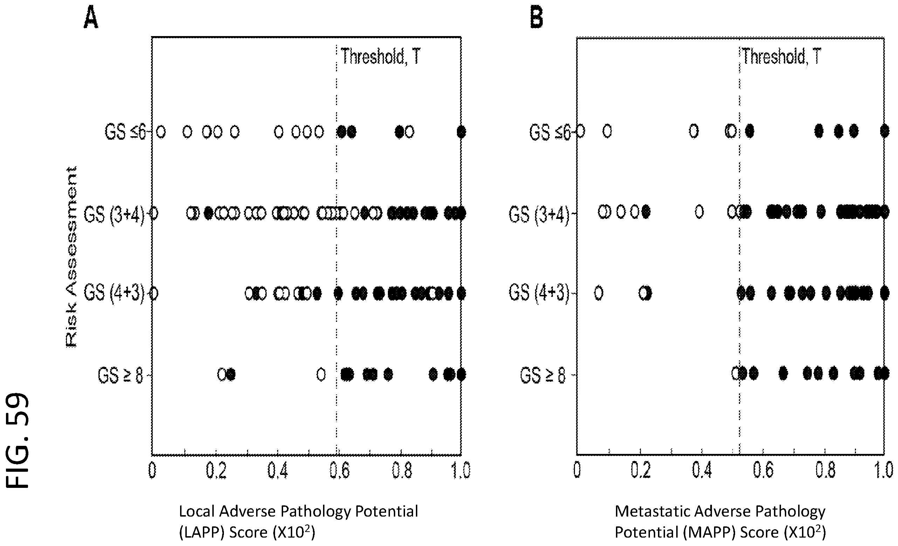

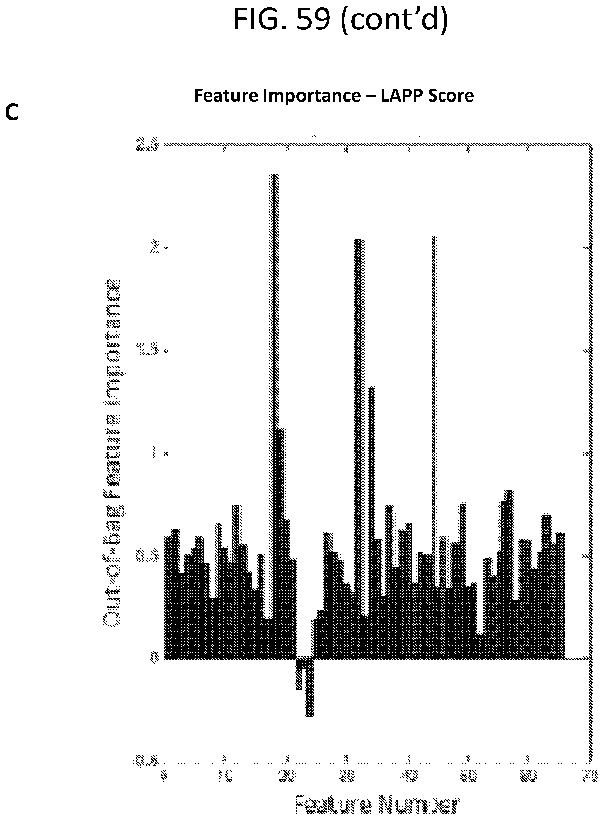

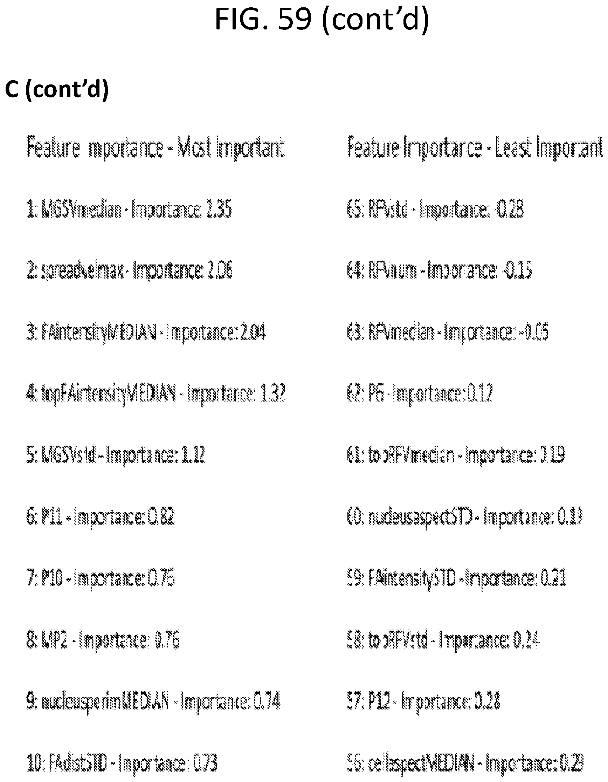

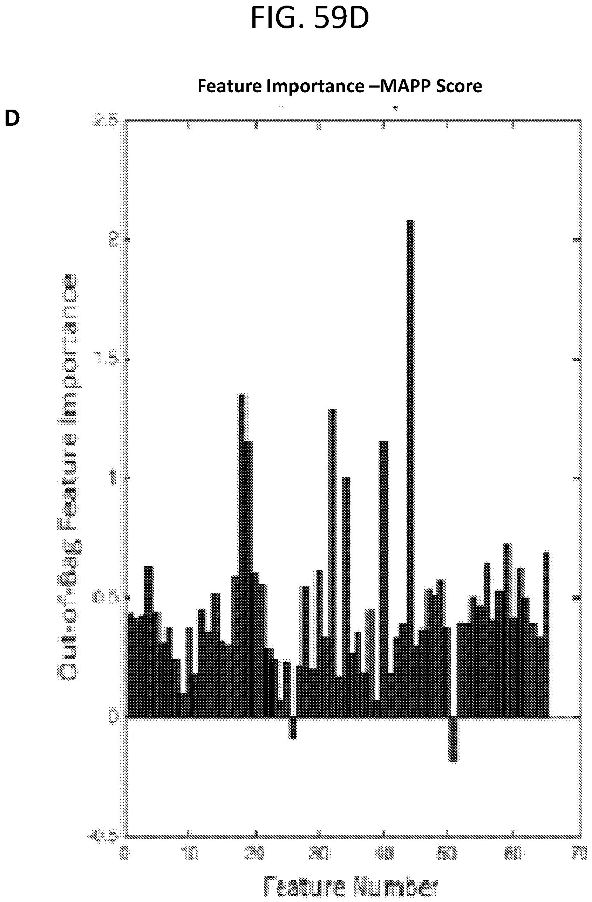

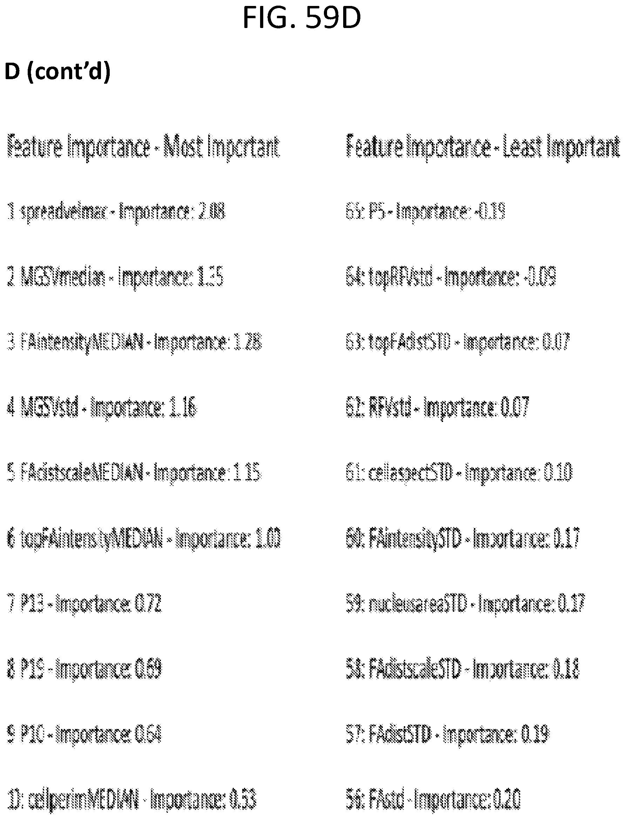

FIGS. 59A & 59B depict risk assessment plots that predict the overall Local Adverse Pathology Potential (LAPP) and Metastatic Adverse Pathology Potential (MAPP) of all samples assayed, grouped by the Gleason score. FIG. 59A shows the LAPP predictor output in each sample, generated by multivariate regression analysis of three adverse pathology predictor outputs for that sample- namely surgical margins, extraprostatic extension and seminal vesicle invasion. The dotted grey line is the algorithm-defined operation threshold. Filled circles to the right of the threshold represent true positives for 'at least one' adverse pathology, while open circles to the right of the threshold are false positives for any adverse pathology. Filled circles to the left of the threshold represent false negatives for 'at least one' adverse pathology which our assay missed, while open circles to the left of the threshold are true negatives for any adverse pathology. FIG. 59B shows a similar plot to FIG. 59A, but depicts the MAPP predictor output in each sample generated by multivariate regression analysis of the following three adverse pathology predictor outputs for that sample- perineural invasion, vascular invasion and lymph node positive.

FIG. 59C depicts an exemplary "feature importance" for LAPP predictor output, which is a rank order of the importance of various biomarkers in generating the algorithm output. The number associated with the biomarker represents the relative importance (1 is the most important, 65 is the least).

FIG. 59D depicts feature importance of a MAPP predictor output.

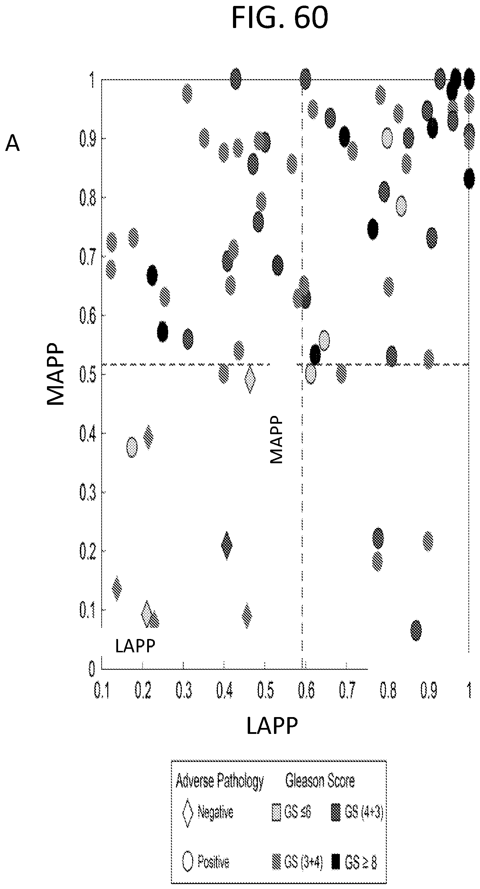

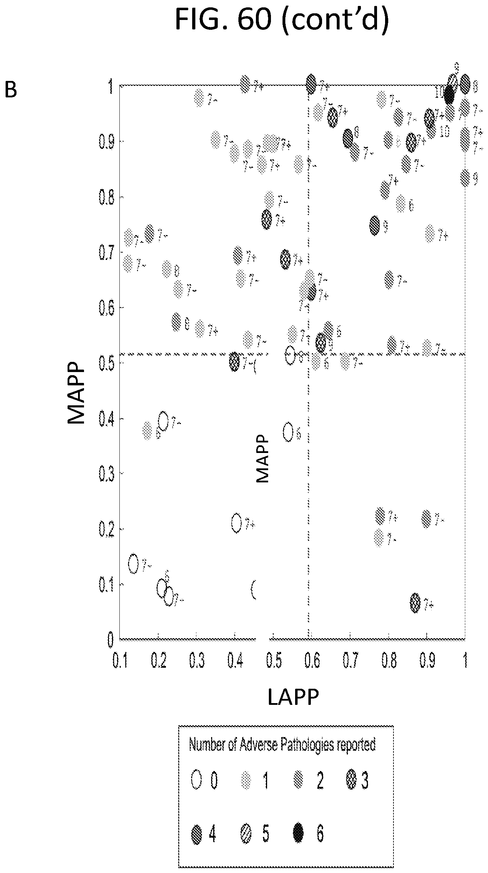

FIGS. 60A and 60B depict scatter plots with MAPP predictor scores on the Y axis and corresponding LAPP predictor scores on X-Axis for prostate samples (n=74). Each data point represents an individual sample. In FIG. 60A data are color coded by Gleason scores (as per the key) and the shape of the data point indicates whether or not an adverse pathology was reported for the sample. Dotted lines represent the algorithm defined thresholds for each predictor. Points above the threshold represent samples predicted positive for at least 'one adverse pathology' for that predictor (SMs, SVIs or EPE for LAPP; PNI, VI, and LNI+ positive for MAPP). FIG. 60B depicts a plot similar to FIG. 60A, except that data are color coded by the number of adverse pathologies reported (as per the key) and Gleason Scores mentioned alongside each data point.

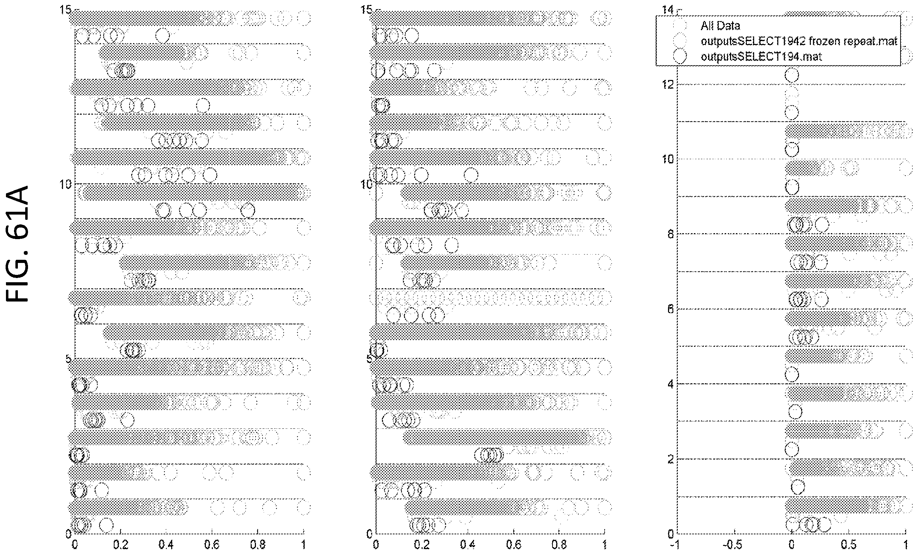

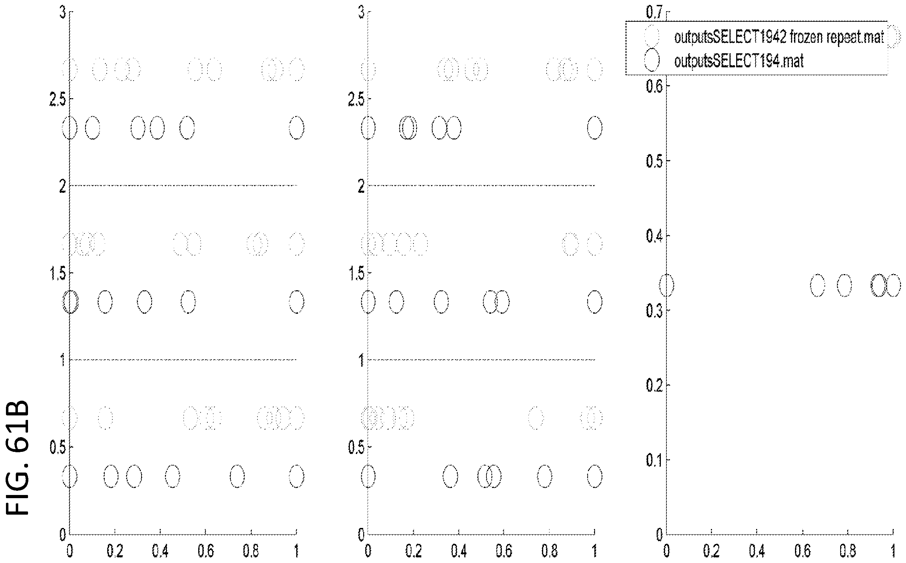

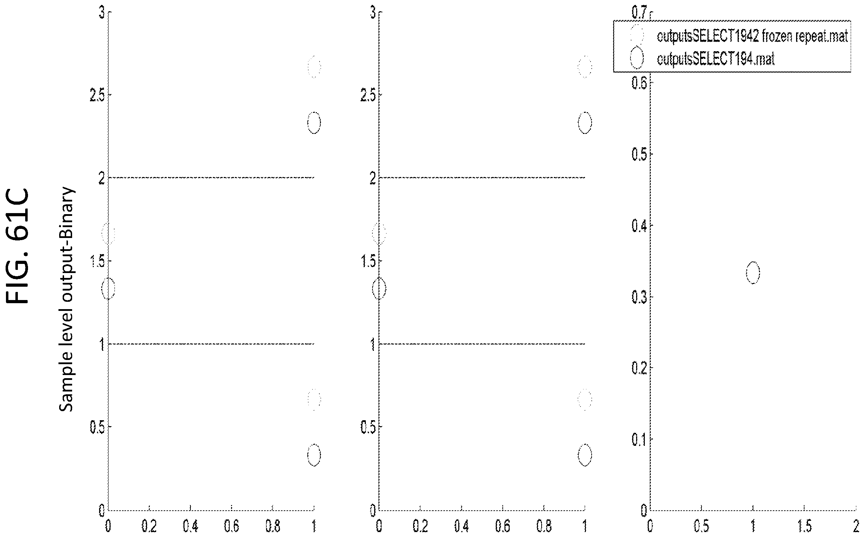

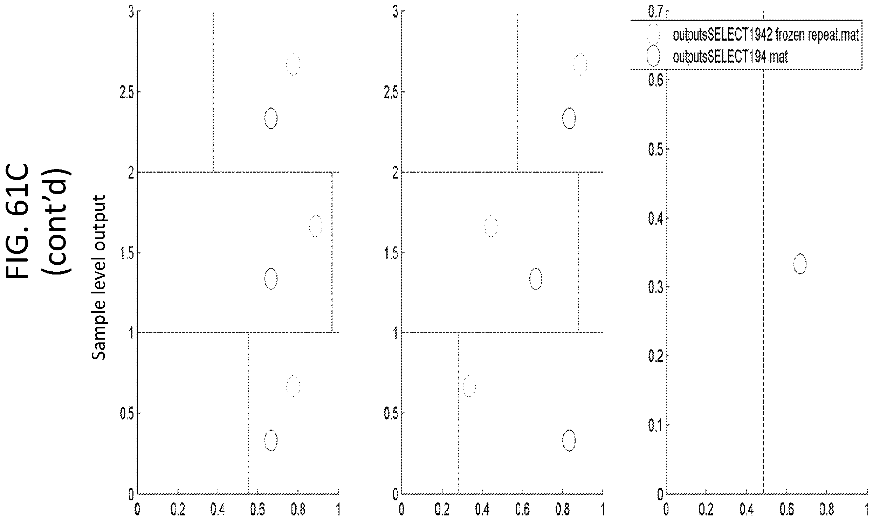

FIGS. 61A, 61B, and61C depict OMAHA robustness, tested by running the sample either fresh or after being frozen once. FIG. 61A depicts a comparison of 44 biomarker outputs (cell level output indicators) in individual cells from one representative sample that were subject to the diagnostic assay either fresh or after one round of freeze (at -80°C) and thaw. The data are compared alongside the total range of biomarker measurements generated by the algorithm (as per the key). FIG. 61B is similar to FIG 61A, except that it depicts a comparison of sample level outputs in fresh vs frozen cells from the given sample. FIG. 61C is similar to FIG 61B, except that it depicts the similarity in the overall sample level output (prognostic indicator or predictor).

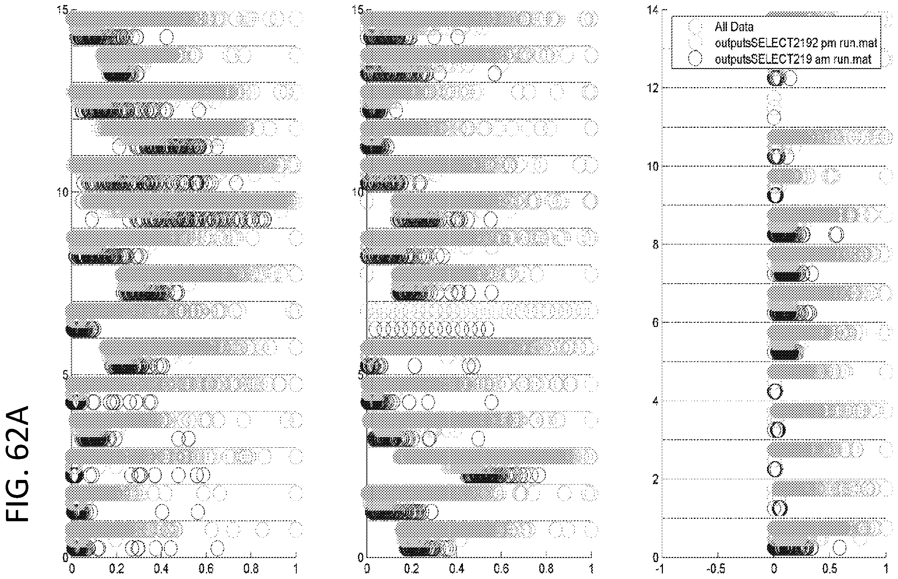

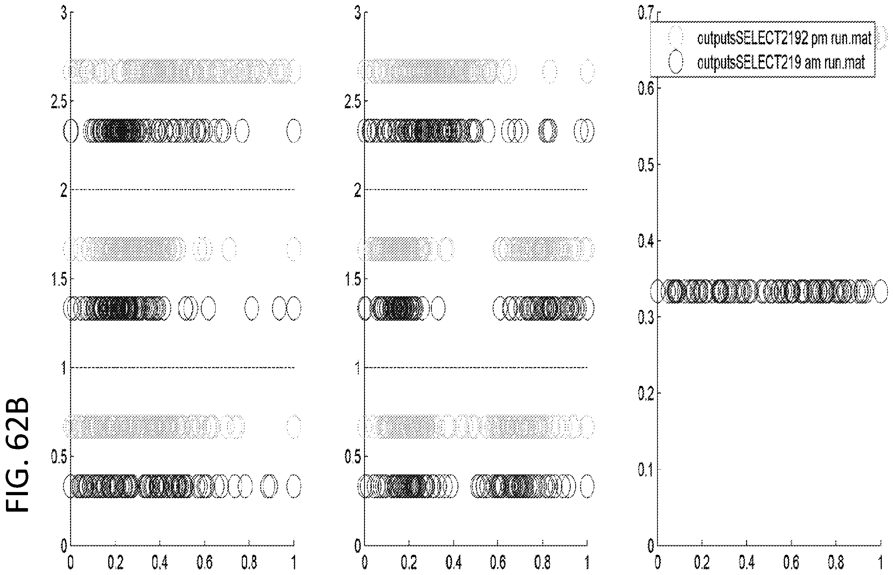

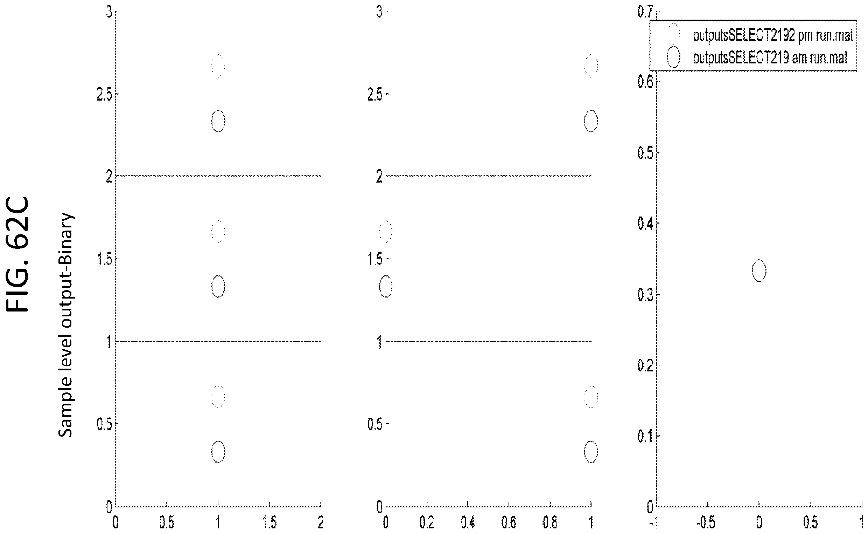

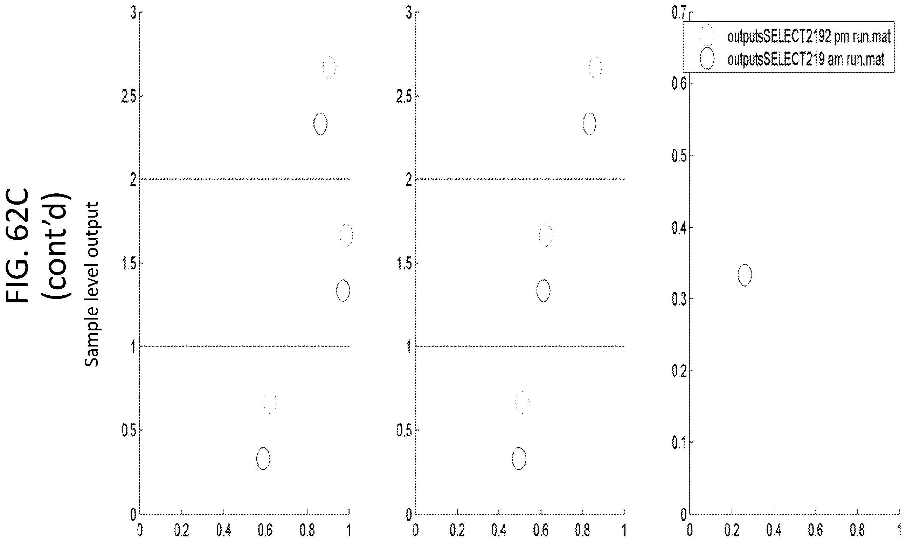

FIGS. 62A, 62B, and 62C depict OMAHA reproducibility, tested by assaying the same sample twice - running the assay with half the cells in the AM and half in the PM. FIG. 62A depicts a comparison of 44 biomarker outputs in individual cells assayed either in the AM or in the PM. The data are compared alongside the total range of biomarker measurements ever generated by the algorithm. FIG. 62B is similar to FIG. 62A, except that it depicts a comparison of sample level outputs in AM vs PM run for the given sample. FIG. 62C is similar to FIG. 62B, except that it depicts the similarity in the overall sample level output.

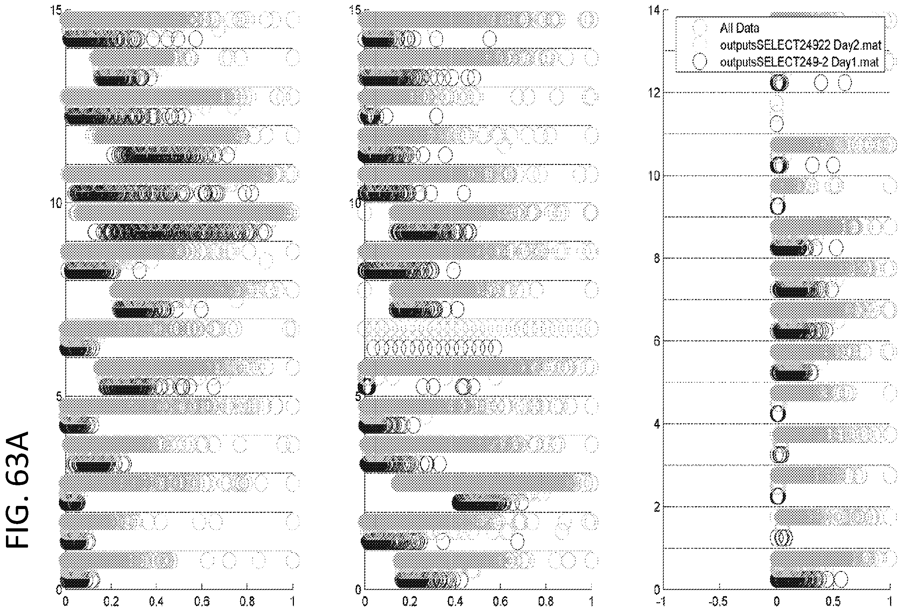

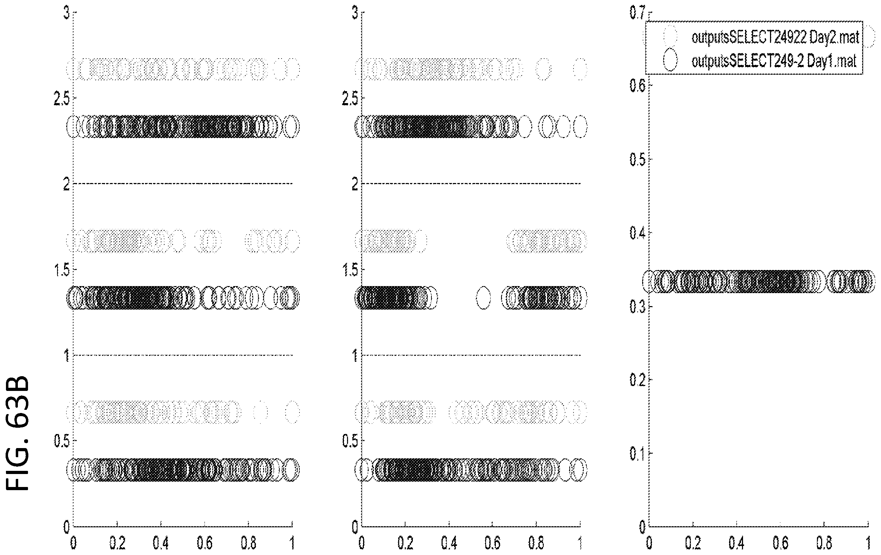

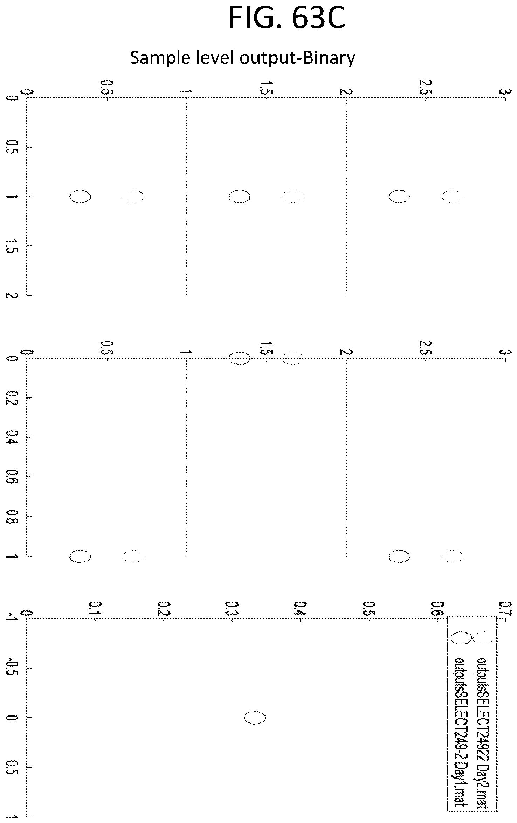

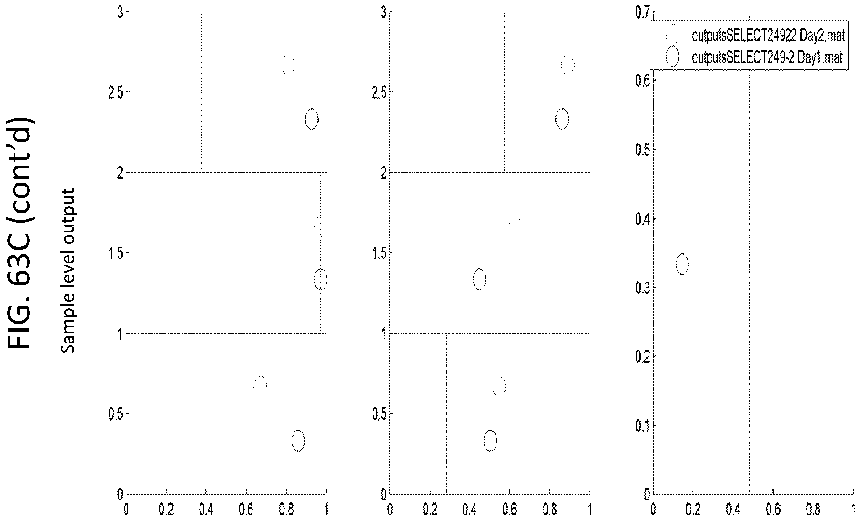

FIG. 63A, 63B, and 63C depict OMAHA day-to-day reproducibility, tested by assaying the same sample twice - running the assay with half the cells on Day 1 and half on Day 2. FIG. 63A depicts a comparison of 44 biomarker outputs in individual cells assayed either on Day 1 or on Day 2. The data are compared alongside the total range of biomarker measurements ever generated by the algorithm. FIG. 63B is similar to FIG. 63A, except that it depicts a comparison of sample level outputs on Day 1 vs Day 2 for the given sample. FIG. 63C is similar to FIG. 63B, except that it depicts the similarity in the overall sample level output.

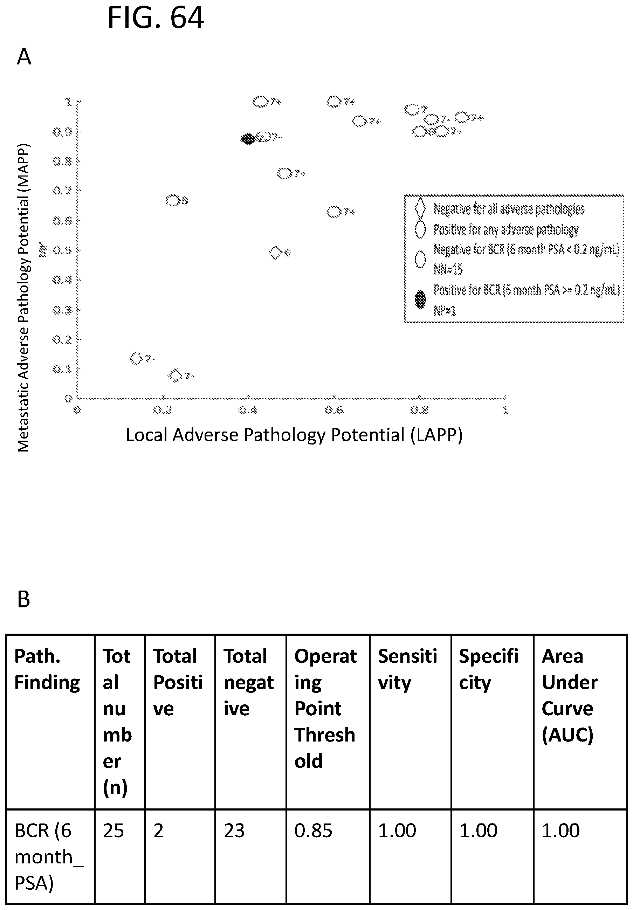

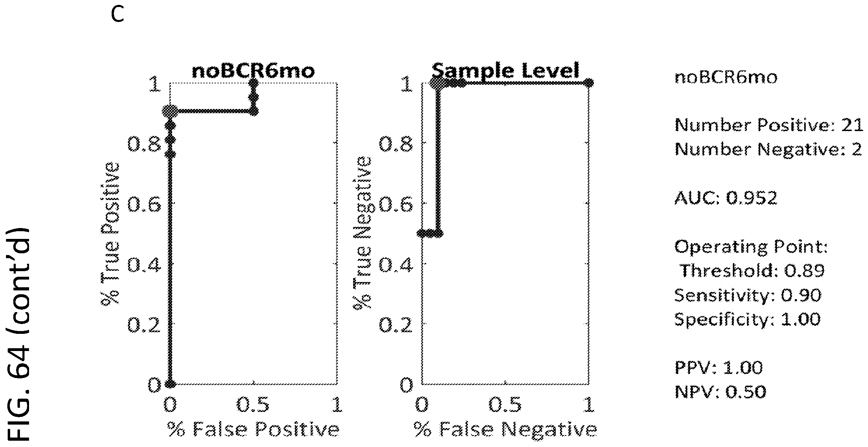

FIGS. 64A, 64B, and 64C relate to BCR (biochemical recurrence) prediction with an algorithm that predicts adverse pathologies using LAPP and MAPP scores. FIG. 64A depicts a scatter plot with MAPP predictor scores on the Y axis and corresponding LAPP predictor scores on X-Axis for prostate samples (n=16). Each data point represents an individual sample, with the Gleason score mentioned alongside. The shape of the data point indicates whether or not an adverse pathology was reported for the sample. Data points in black represent samples with reported 6 month BCR. Patients that exhibit PSA > 0.2 mg/mL at 6 months are defined as BCR positive. FIG. 64B describes an exemplary ROC curve generated by a classification algorithm that can create a single LAPP to predict which patients will have 6 month BCR independent of adverse pathology in prostate tissue (n=25). An AUC of 1.0 was obtained at a selected operating point, achieving a sensitivity and specificity of 1.0. FIG. 64C provides an output related to 23 patient samples assessed for biochemical recurrence (BCR) as defined by a PSA>0.2ng/ml after radical prostatectomy. Quantification of biomarkers provided a statistical algorithm that generated a 'Threshold' value of 0.89, resulting in the prediction of patients that will not experience BCR with a sensitivity of 0.90 and specificity of 1.00.

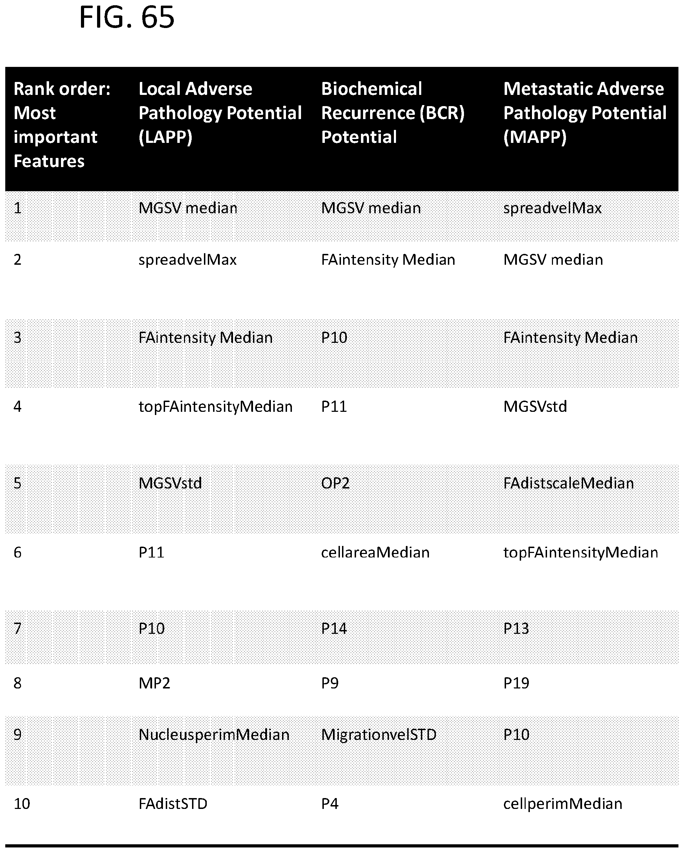

FIG. 65 depicts a comparison of the top 10 exemplary biomarker outputs identified for predicting adverse pathologies vs. BCR in prostate tissue.

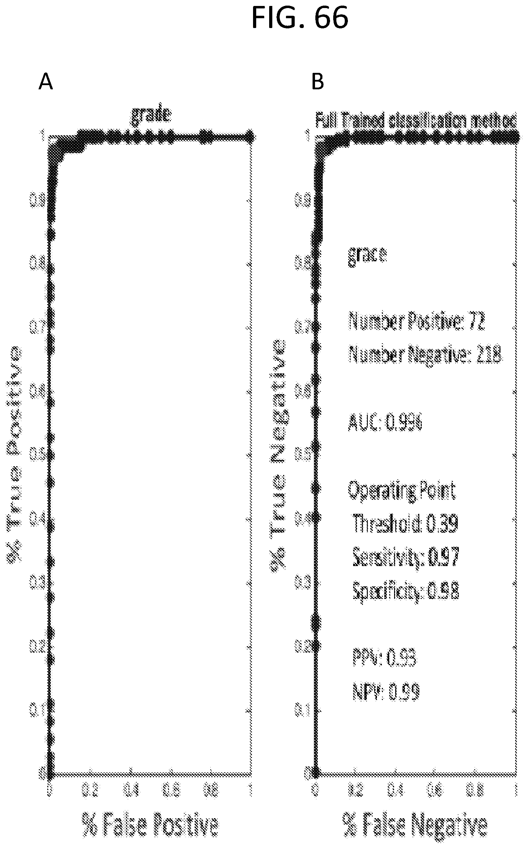

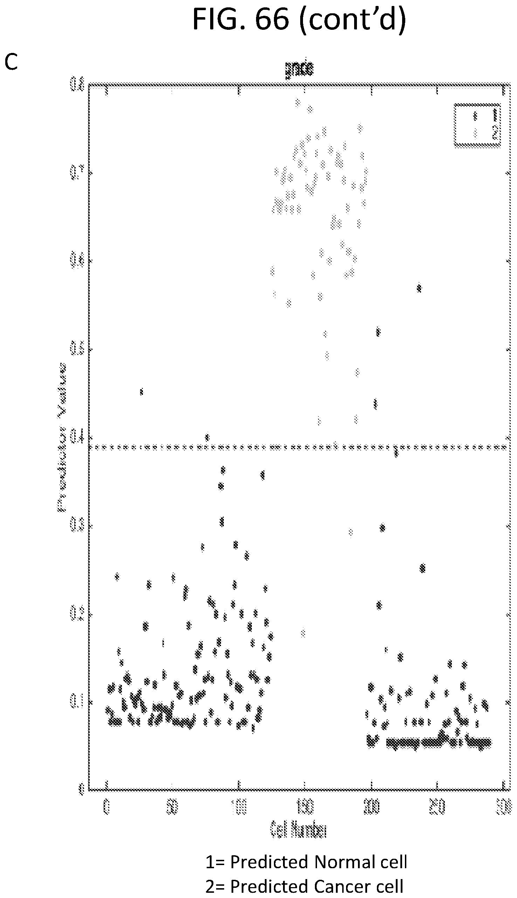

FIGS. 66A, 66B, and 66C depict algorithm generated predictors for tumor 'grade'. FIGS. 66A and 66B depict exemplary sample level ROC curves generated by a classification algorithm that predicts the grade of the tumor (n=290). An AUC of 0.996 was obtained at the selected operating point, achieving a sensitivity of 0.97 and specificity of 0.98. FIG. 66C depicts an exemplary cell level predictor plot for one given sample, with the predictor value for each cell on the Y axis and cell number on X axis. The dotted line represents an algorithm-defined threshold. Cells represented by light grey dots are predicted cancer cells, and cells represented by black dots are predicted normal cells.

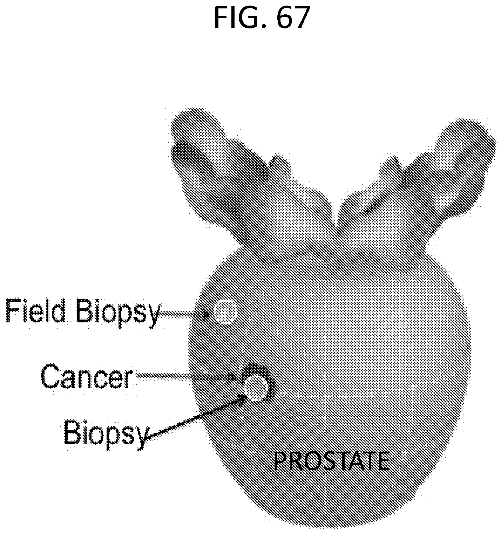

FIG. 67 depicts a prostate gland, showing a location of a malignancy and biopsy locations within the location of the cancer and in a field location (adjacent tissue) outside of the location of a malignancy to depict the manner that biopsies are taken in a clinical setting. A field algorithm is applied to field samples, and a malignant algorithm is applied to samples from the location of the malignancy, and the results from both types of samples result in a prediction of adverse pathology.

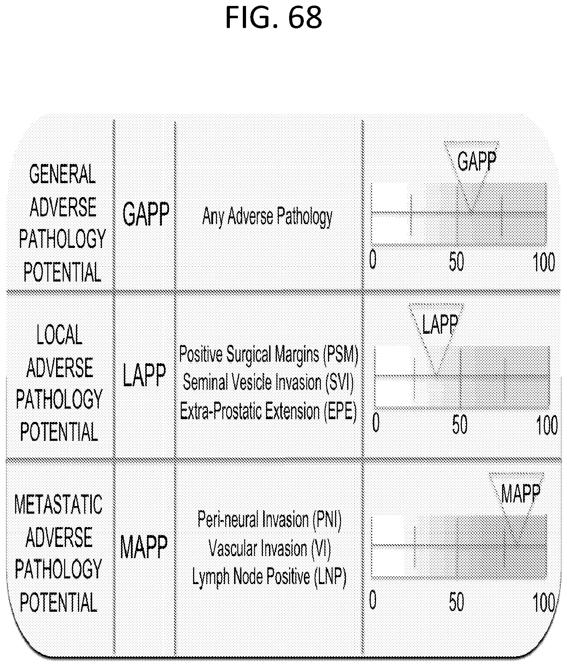

FIG. 68 depicts an overview of an exemplary process involving the evaluation of over ten biomarkers described herein, application of algorithms as described herein, and the generation of 3 predictive metrics - General Adverse Pathology Potential (GAPP), Local Adverse Pathology Potential (LAPP), and Metastatic Adverse Pathology Potential (MAPP). Certain exemplary measures and an exemplary evaluation of GAPP, LAPP, and MAPP outputs are depicted.

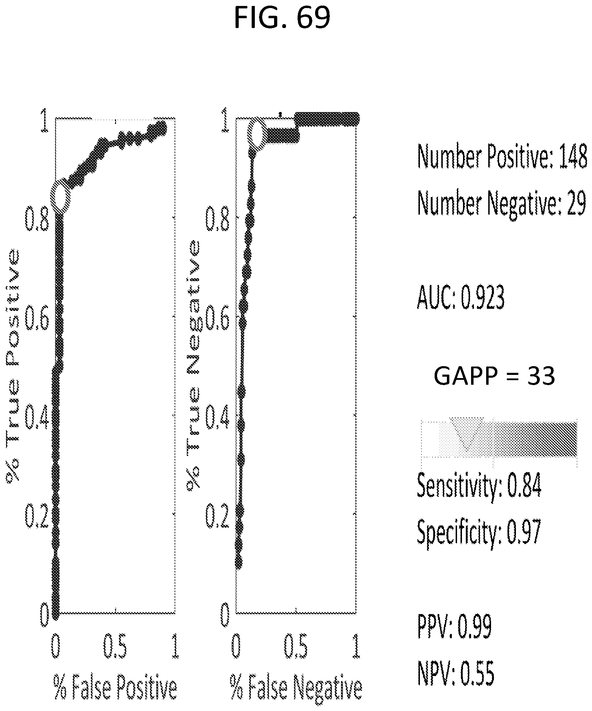

FIG. 69 depicts an exemplary GAPP ROC curve generated by a classification algorithm that can predict any adverse pathology. The large circle depicts the threshold point, or GAPP in this example.

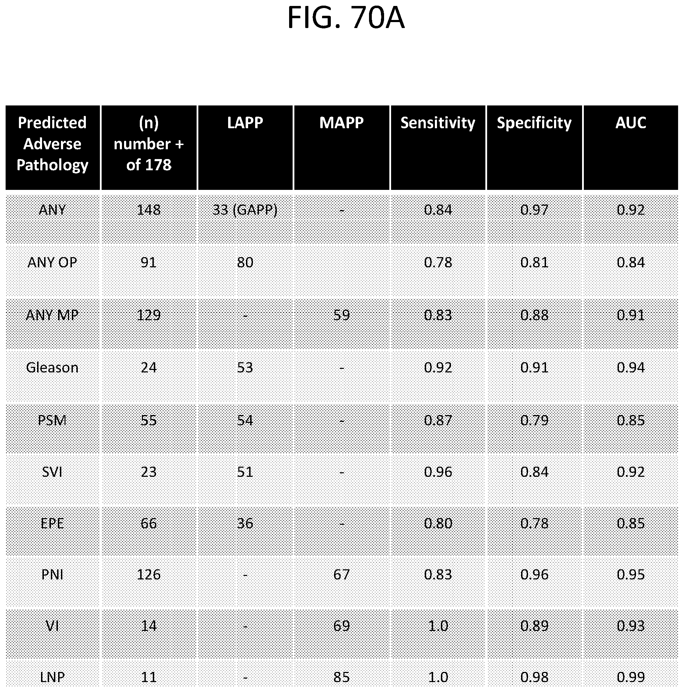

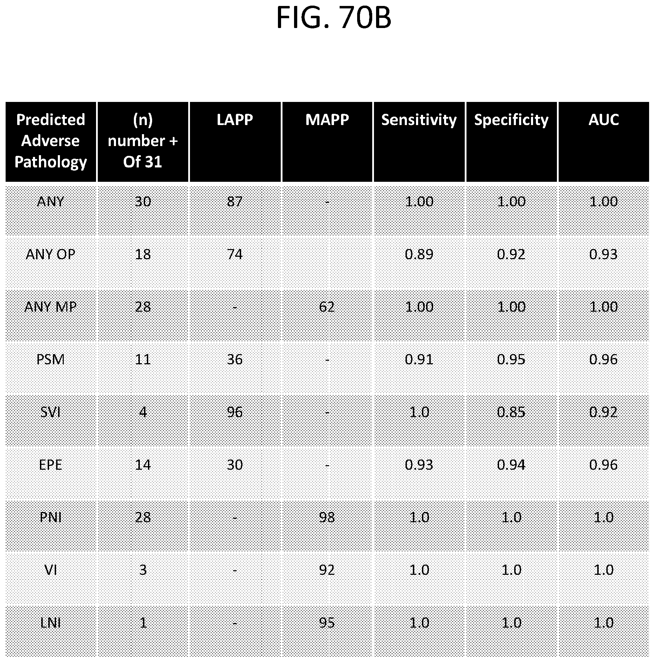

FIGS. 70A and 70B depict clinical validation of an exemplary prostate phenotypic evaluation described herein for a variety of adverse pathologies in patients. LAPP and MAPP outputs are provided, in addition to sensitivity, specificity, and AUC. FIG. 70A refers to samples obtained from a malignancy location, and FIG. 70B refers to samples obtained from a field location (nearby tissue or adjacent tissue).

## DETAILED DESCRIPTION OF THE VARIOUS EMBODIMENTS

For clarity of disclosure, and not by way of limitation, the detailed description of the various embodiments is divided into certain subsections that follow.

Unless defined otherwise, all technical and scientific terms used herein have the same meaning as is commonly understood by one of ordinary skill in the art to which this disclosure belongs.

As used herein, "a" or "an" means "at least one" or "one or more."

As used herein, the term "and/or" may mean "and," it may mean "or," it may mean "exclusive-or," it may mean "one," it may mean "some, but not all," it may mean "neither," and/or it may mean "both."

As used herein, "Local Adverse Pathology Potential" or "LAPP" (also referred to herein as "Oncogenic potential" or OP) refers to a quantitative prediction of a tumor's growth potential, or an algorithmic dynamic biomarker prediction of local adverse pathology.

As used herein, "Metastatic Adverse Pathology Potential" or "MAPP" (also referred to herein as "Metastatic potential" or MP) refers to a quantitative prediction of whether a tumor will invade other tissues, or algorithmic dynamic biomarker prediction of distant adverse pathology.

As used herein, "treatment" means any manner in which the symptoms of a condition, disorder or disease are ameliorated or otherwise beneficially altered. Treatment also encompasses any pharmaceutical use of the compositions herein.

As used herein, "subject" often refers to an animal, including, but not limited to, a primate (e.g., human). The terms "subject" and "patient" are used interchangeably herein.

As used herein, the terms "detect," "detecting," or "detection" may describe either the general act of discovering or discerning or the specific observation of a molecule or composition, whether directly or indirectly labeled with a detectable label.

As used herein, "sensitivity" refers to sensitivity = true positives / (true positives + false negatives).

As used herein, "specificity" refers to specificity = true negatives / (true negatives + false positives).

As used herein, the term "designation" refers to any value that is used to uniquely identify the predictor model. The designation may be a function, a function name or a pointer used to invoke the predictor. The predictor factory may maintain a table mapping designations to predictor models for looking up a predictor model given a designation. The designation may comprise a numeric identifier, a string, a function pointer identifying the predictor model, or a name of a function or method implementing the predictor model. The designation also comprises information identifying coefficient values corresponding to the predictor model, for example, coefficient values used by a machine learning technique.

As used herein, "prognostic indicator" refers to an indicator which predicts the likely outcome of a certain disease, diagnosis, or activity.

As used herein, the phrase "cell level output" refers to the results of an analysis performed using the imaging and machine learning processes described herein with an assumption that each cell within a sample or subject is an independent entity. An exemplary cell level output provides a series of descriptors for various behaviors of interest for a cell.

As used herein, the phrase "sample level output" or "subject level output" refers to an aggregate analysis of a cell level output that describes all evaluated cells belonging to a particular sample or subject. LAPP, MAPP, adverse pathology prediction, and GAPP are included as sample level and subject level outputs.

As used herein, the phrase "predictor" or "predictors" refers to a machine leaning algorithm or machine learned model. LAPP, MAPP, adverse pathology prediction, and GAPP are included as predictors.

As used herein, the phrase "machine learning" refers to the construction and adapting of algorithms based on data with minimal external instructions. *See, e.g.,* C. M. Bishop, Pattern Recognition and Machine Learning (Springer 2007).

As used herein, the phrase "live cell" refers to an intact cell that maintains activity of at least a portion of its typical intracellular processes or extracellular reactions. Typically, "live cell" excludes lysed or fixed cells.

As used herein "diagnosis" refers to the ability of a test to determine, yes or no, if a patient is positive for a disease state.

As used herein "prognosis" refers to the ability of a test to determine how aggressive of indolent a disease state is, in part by predicting specific pathology findings related to the progression of a disease.

As used herein, the term "outlier" or "outlier cell" refers to a cell having a detected or measured biomarker that is distinguishable from that biomarker in one or more other cells in a specific sample or between samples. Often this term refers to a cell having at least one biomarker that is distinguishable, often to a notable degree, from the majority of other cells in the specific sample or between samples.

As used herein, the term "stage of cancer" refers to a qualitative or quantitative assessment of the level of advancement of a cancer. Criteria used to determine the stage of a cancer include, but are not limited to, the size of the tumor and the extent of metastases (e.g., localized or distant).

As used herein, "sample" refers to any substance containing or presumed to contain a cell of interest or a cell for investigation. The term "sample" thus includes a cell, organism, tissue, fluid, or substance including but not limited to, for example, blood, plasma, serum, spinal fluid, lymph fluid, synovial fluid, urine, tears, stool, external secretions of the skin, respiratory, intestinal and genitourinary tracts, saliva, blood cells, tumors, organs, tissue, samples of cell culture constituents, natural isolates (such as drinking water, seawater, solid materials), microbial specimens, cell lines, and plant cells, including processed, purified, isolated, enriched or enhanced versions of these substances. A "tissue sample" refers to a sample having or obtained from a tissue of a subject, including homogenized, disassociated, otherwise processed samples, cellular cultures thereof, and fractions or expression products thereof.

Any sample suspected of containing cells relevant to the therapeutic indication being evaluated can be utilized in the devices and according to the methods of the present disclosure. By way of non-limiting example, the sample may be tissue (e.g., a prostate biopsy sample or a tissue sample obtained by prostatectomy), blood, urine, semen, cells (such as circulating tumor cells), cell secretions or a fraction thereof (e.g., plasma, serum, exosomes, urine supernatant, or urine cell pellet). In the case of a urine sample, such is often collected immediately following an attentive digital rectal examination (DRE), which causes prostate cells from the prostate gland to shed into the urinary tract. The sample may require preliminary processing designed to, purify, isolate, or enrich the sample for cells of interest. A variety of techniques known to those of ordinary skill in the art may be used for this purpose.

The present description should be read with reference to the drawings. The drawings, which are not necessarily to scale, depict selected embodiments and are not intended to limit the scope of the present disclosure. The detailed description illustrates by way of example, and is not intended to limit the scope of the present disclosure.

## Tissue dissociation

After receiving a tissue sample, it is dissacociated according to known methods, devices, and reagents, for example, those set forth in U.S. Patent Application Publication Nos. 20130149724 and 20130237453, and PCT Patent Application No. PCT/US14/61782, filed October 22, 2014 and published as WO2015/061458 A1.

## Perfusion chamber

The disassociated cells can be optionally placed in a perfusion chamber, for example, such as those set forth in U.S. Patent Application Publication Nos. 20130149724 and 20130237453, and PCT Patent Application PCT/US14/61782 filed October 22, 2014 and published as WO2015/061458 A1, including related reagents and methods.

In various embodiments discussed above, given the inputs of mammalian tissue, the device, in an automated, systematic fashion, can dissociate, segregate, sort, enrich, manipulate, and assay cells for biomarker quantification. These quantified biomarkers, which can be based on physical properties of the cells or biochemical / metabolic properties of the cells or associated extracellular components, can then be used as inputs into algorithms to output quantifiable metrics regarding the aggressiveness, or oncogenic potential, of a cancer, or the invasion, motility, or metastatic potential of a cancer. Examples of these algorithms can be found, for example in U.S. Patent Application Publication No. 20130237453.

The present inventors have developed innovative microfluidic devices. Based on the quantification of biomarkers in such devices, metrics of MAPP and LAPP were developed, for example, to aid physicians in treatment decisions and supplement the qualitative Gleason score with a sensitive, specific, and quantitative metrics. MAPP and/or LAPP can be used to modify or confirm an established clinical nomogram, tumor grade, cancer staging or grading system, or pathological score used for diagnosis and /or prognosis. For example, in other certain embodiments, MAPP and/or LAPP is/are used to modify or confirm a Nottingham Score determination for the sample. The devices and methods described and contemplated herein represent an exemplary a personalized diagnostic solution capable of predicting aggressiveness to better guide therapy selection. Moreover, the inventors have also cultured and evaluated prostate cells from clinically relevant patient samples *in vitro* with similar results.

The presently described devices, methods and clinical measures can, in certain embodiments, be utilized along with the traditional Gleason Scores in evaluating patients, which adds critical information to the evaluation of patients having Gleason scores of, for example, 6-9, or higher.

On one exemplary protocol, biopsied cells are introduced (e.g., injected) into microfluidic devices of the present disclosure. The cells are then analyzed on the chip using, for example, automated light/fluorescent microscopy, and images are uploaded to, or accessed in a database by, a program that utilizes machine vision image analysis to calculate and return LAPP and MAPP values. In such an exemplary protocol, the following steps are characterized by the use of one or more technologies selected from the group consisting of ECM formulation, a microfluidic device, a biomarker suite, machine vision software, and prognostic algorithms. Frequently, raw images are generated that require processing. After processing and then analysis, the resulting data is often synthesized into distinct, meaningful outputs that can be delivered to physicians. Though prostate samples are often utilized, the presently described technologies and methods are readily applied to bladder, lung, kidney, breast, ovarian, uterine, colon, thyroid, or skin tissues and cells.

In certain embodiments, the present devices and methods provide the ability to differentiate between low-risk (low-grade) and high-risk (high-grade) prostate cancer as correlated with the reference standard of the Gleason Score. The present devices and methods also often provide a stratification of low-risk, intermediate-risk, and high-risk patients as correlated with the reference to Gleason Score standards, or another established clinical nomogram, tumor grade, cancer staging or grading system, or pathological score. In addition, the present devices and methods provide the ability to differentiate between different types of intermediate risk patients (Gleason 6 or 7) - risk stratifying within the intermediate patient prostate cancer population, segregating patients as having indolent, locally aggressive, or metastatically aggressive types of cancer. Also, the present devices and methods provide the ability to act as a therapy guide, differentiating patients who should be treated via active surveillance, surgery or radiation, and / or adjuvant therapy. In certain embodiments, the present devices and methods also provide the ability to facilitate compound validation and therapeutic pipeline acceleration. In frequent embodiments, the present devices and methods also provide the ability to distinguish between normal and cancer samples, predict aggressive potential of disease, stratify patients by risk category, within patients that are intermediate risk (clinically ambiguous), identify patients with local growth potential and/or metastatic potential, control for biopsy sample heterogeneity, provide high signal to noise biomarker analysis, and return clinically actionable metrics

*Biophysical Metrics and Predictive Indications*

In certain preferred embodiments, the present methods, systems, and devices provide novel phenotypic diagnostic test capabilities that identify and analyze biomarkers that correlate with relevant indicators of cancer pathology (e.g., prostate, bladder, lung, kidney, breast, ovarian, uterine, colon, thyroid, skin). As such, not only does the present disclosure provide the ability to identify and monitor biomarkers in live cells in a manner heretofore not possible or contemplated, but it also provides the capability of at least: identifying novel biomarkers in cell populations; attributing a novel significance to biomarkers relative to diagnoses, therapeutic decisions, or drug monitoring; adjusting or confirming pathological findings obtained via traditional or accepted methodologies (e.g., Gleason Score, Nottingham Score); and/or adjusting or confirming prognoses and therapeutic interventions obtained or designed using traditional or accepted methodologies.

In connection with prostate cancer, the present disclosure provides methods and systems that generate actionable scoring metrics of MAPP and LAPP that distinguish between, for example, Gleason 6 vs. 7, as well as within Gleason 7 (3+4 vs. 4+3) scores. These methods and systems, therefore, will aid physician decision making in the treatment of prostate cancer while patients are on active surveillance. These methods and systems are also useable in connection with other tumor types, for example, kidney, breast, and lung tumors.

In certain embodiments, an automated method of evaluating a cell of a subject for the presence or absence of a pre-determined metric or collection of metrics, as described herein, without additional user input. In such embodiments, the cell is exposed to a visioning system such as magnified imaging system (e.g., a microscope) having machine vision capabilities. The visioning system identifies a metric exhibited by the cell (e.g., migration velocity) to characterize the cell as a cell for further examination based on that metric. The characterization is based on an evaluation or measurement of that metric as falling within the bounds of the exhibition of that metric in normal or non-cancerous cells and/or the exhibition of that metric in cancerous cells. Cells identified as falling outside the bounds of normal measured characteristics relative to others from the same sample are, most frequently, selected for further investigation. These cells are identified in frequent embodiments as outliers. Frequently included in this process is a trained model of cellular examination based on the evaluation of the metric in a population of cells, including mixed populations of similar or the same cell types, or cellular populations obtained from similar tissues, including normal cells, cancerous cells, pre-cancerous cells, and/or mixtures of any two or more of the foregoing.

In a tissue sample obtained from a subject, often only a portion of the heterogenous cell population exhibits outlier characteristics or is actually cancerous. Though not wishing to be bound by any particular theory, selected outlier status appears to be the case typically for only a selected subset of cells even if the tissue is obtained from a patient known to have cancer present in that tissue. As such, the methods and devices described herein are useful to, in a frequently automated manner, identify outlier cells present in a sample for further investigation according to methods and using devices described herein.

Novel biomarker evaluation, such as certain biomarkers described herein, are often included in this process. Cells may be evaluated as bare cells. Cells may also be evaluated after or concurrently with being stained with specific stains (e.g., chemiluminescent, fluorescent, contrast, etc.) that enhance the detectability of pre-determined metrics, such as certain cellular features, or the presence of certain proteins or surface markers. In addition, cells may be evaluated after or concurrently with being exposed to a reagent such as a molecular marker that is detectable in the presence of certain cellular processes or in the presence of certain nucleic acids, polypeptides, or proteins.

The presently described machine learning algorithms have the ability to process multiple biomarkers and accurately predict various pathological outcomes, as outlined in FIG. 6. With regard to FIG. 6, Table A, the values in the table are comprised of measured values of selected biomarkers extracted from live cell imaging as detailed herein. As such, each of the biomarkers contemplated herein is measurable and may be attributed a specific number upon measurement.

With regard to FIG. 6B, multiple exemplary decision trees are shown. These decision trees provide a representation of the specific machine learning algorithm used, e.g., the bootstrap aggregated decision tree. In an exemplary version of this process, each of the measured biomarkers is utilized to create a decision tree that when viewed individually or together with additional biomarker decision trees leads to a positive or negative outcome for a certain pathological output concerning a sample. As an extremely simplified example taken partially from Figure 6B, one of the trees considered biomarker "M1" as a biomarker of significance, or a measured biomarker. It decided that if any cells has an M1 value greater than X, then it is positive for a pathological output; and if MI is smaller than X, then it is negative for a pathological output. More frequently, multiple biomarkers are assessed concurrently or sequentially in this manner to feed the pathological output. Also frequently, a particular weighted significance is attributed to one or more biomarkers such that its evaluation in the decision tree carries either more or less significance in the overall pathological output. In other words, often if multiple biomarkers are assessed for a specific cell or population of cells, each biomarker is not equally weighted with each other biomarker in terms of the ultimate pathological output. As mentioned above, multiple (e.g., up to 25 or more) decision trees may created and included the analysis in the methods described herein. This multiple biomarker evaluation process has proven to be unexpectedly useful, for example when conducting a *de novo* investigation of a sample involving a correlation of biomarkers and pathological outputs that is not known ahead of time. Generation of multiple decision trees permits, in certain embodiments, an aggregation of data from multiple decision trees into an optimized process or algorithm that optimizes the sensitivity and specificity of results based on measured biomarker information of a sample. In certain embodiments, the presently described methods and systems utilize multiple decision trees simultaneously or concurrently, weighting the accuracy of the outcomes of all the decision trees based on previously known information, and then returning a predicted pathological outcome.

Figure 6C provides a representative plot demonstrating stratification among negative and positive cells utilizing combinations of biomarkers as described by the decision trees. The Y axis provides the likelihood of the predicted pathological outcome (or SCPI) between 0 and 1. Each dot represents a cell that has been imaged. Red dots (dark) are cells predicted to be negative for a pathological output (normal) and blue dots (light) are predicted to be positive (abnormal). This plot represents a single pathological outcome. Often, if multiple pathological outcomes are being investigated, individual plots are generated for each outcome.

Figure 6D provides a graph summarizing cell level results into subject level results. In particular, an exemplary plot demonstrating stratification of patients for a given predicted pathological outcome is provided. This graph separates individual subjects based on their pathological output using systems and methods described herein. The location of each dot on the graph is determined based on the predicted pathological outcome for each subject. Hence, the X-axis is one method of segregation between positive and negative patients that has a clinically relevant meaning, and the Y-axis is another method of segregation between positive and negative patients that has a different clinically relevant meaning from the X-axis. These segregation methods (or patient predictor index (PPI)) are based on an extension of the results of the cell level data.

With further reference to Figure 5, the Table below provides a listing of individual cells, and the imaging-related scores of each cell for each of four different biomarkers.

Table 1

| Patient X | | | | |
|---|---|---|---|---|
| Cell ID | Marker 1 | Marker 2 | Marker 3 | Marker 4 |
| 1 | 7353.5 | 509.42 | 2.9523 | 30744 |
| 2 | 40526 | 1576.3 | 5.2008 | 22409 |
| 3 | 7063.6 | 578.99 | 4.0195 | 75730 |
| 4 | 18066 | 1263 | 7.2896 | 2.12E+05 |
| 5 | 9470.8 | 488.55 | 2.0773 | 2.09E+05 |

Cells with biomarkers that were determined to be outliers compared to the norm were isolated and further analyzed. These data are represented in Figure 5. Although migration velocity comprised the biomarker represented in Figure 5, any of the other biomarkers contemplated herein may be plotted in this aspect.

Image data from abnormal cells were subjected to a machine learning algorithm, which is composed of a collection of previously trained weighted decision trees correlating biomarkers to pathological outcomes. *See* Figure 5. The result was each cell was attributed with a percent likelihood of it being of a certain pathological outcome.

**Table 2**

| Patient X | | | | |
|---|---|---|---|---|
| Abnormal Cell ID | Path Indicator 1 | Path Indicator 2 | Path Indicator 3 | Path Indicator 4 |
| 1 | 0.323 | 0.234 | 0.367 | 0.566 |
| 2 | 0.465 | 0.967 | 0.566 | 0.977 |
| 3 | 0.685 | 0.487 | 0.488 | 0.855 |
| 4 | 0.245 | 0.286 | 0.997 | 0.467 |
| 5 | 0.467 | 0.689 | 0.577 | 0.687 |

As an additional step, the cell-level results were summarized into a patient-level outcome, utilizing PPI methods and systems outlined, for example, in and in connection with Figure 6 above. At this stage, the numbers presented are binary (i.e., 0s or Is) and correlated with either a "positive" or a "negative" for a pathological outcome. *See* Table 3 below. This provides a simplified example that can be adapted to provide additional data correlating with additional pathological outcomes. In this example, the subject from whom the sample was obtained may be determined to be positive for a specific indicator of disease or cancer, or may be determined to have a specific stage of disease or cancer, under Patient Indicators 1 and 4 as they each contain the number "1." Patient indicators 2 and 3, being the value "0," most frequently means that the patient is negative for a specific indicator of disease or cancer.

**Table 3**

| Patient X | | | |
| --- | --- | --- | --- |
| Path Indicator 1 | Path Indicator 2 | Path Indicator 3 | Path Indicator 4 |
| 1 | 0 | 0 | 1 |

In addition, though a binary outcome is often desired, numbers falling between 0 and 1 will often provide clinically valuable information regarding an expected clinical pathological outcome, or a confirmation or adjustment of a diagnosis or prognosis.

*Transformation of Cell Images into biophysical metrics*

The transformation of captured cell images into biophysical metrics involves, in certain embodiments, one or more of a variety of processes, including for example: Montaging, Illumination Correction, Edge smoothing/detection, Dynamic Thresholding, Watershedding algorithm, Cell tracking over time, Kymograph analysis, and Signal Crosstalk correction.

In frequent embodiments, a completely automated method of extracting cellular biomarkers, including aspects of cell and nucleus morphology, cell motility, intracellular dynamics, original cell attachment, and adhesion maturation is provided from a diverse set of live cell images is provided. In certain embodiments, the creation and maintenance of a global coordinate and cell tracking system is provided, permitting biomarkers extracted from different imaging magnifications, modalities and time frames to be tied to individual cells. Intracellular motility events such as actin cycling are quantified, for example, by tracking intracellular and cell peripheral features over time. Quantification of biomarkers from fluorescent images is also provided. Image manipulations and computations performed on smaller, subdivided regions of interest is often provided, for example, to improve efficiency. Moreover, refined metrics are synthesized via the condensation of live cell biomarker data into a single framework, having biomarkers attributed to individual live cells. In the related tracking imaging, cell size and shape, nucleus size, edge smoothness, mean grayscale value, and migration velocity are observed, measured or recorded. Cell spreading during tracking is also often quantified in addition to assessment of membrane fluctuations to extract retrograde flow velocity. At the end of tracking, cells may be fixed and stained, which permits one method of focal adhesion identification.

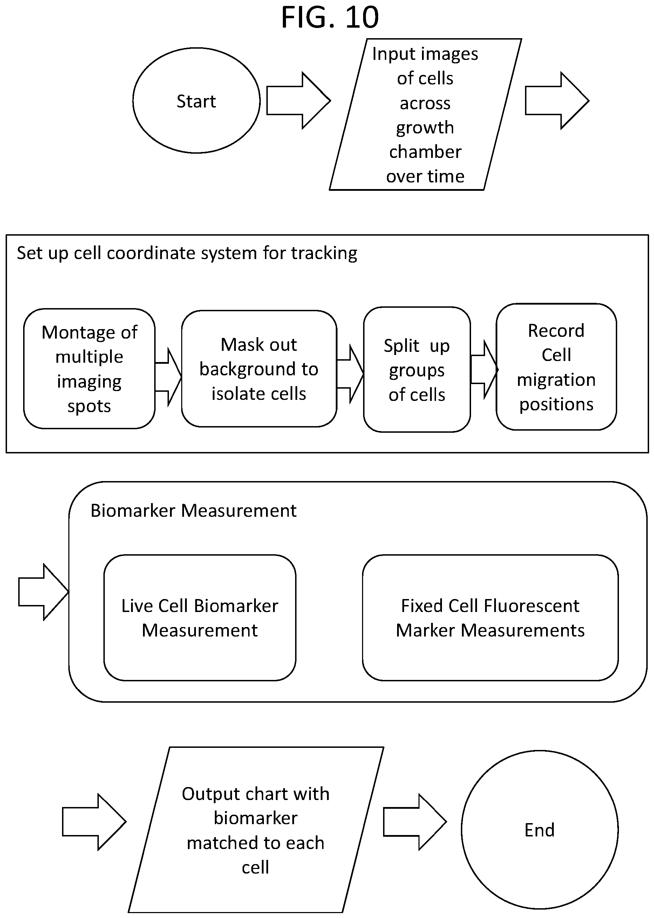

With reference to Figure 10, a variety of images of live cells are obtained in the chamber, which are utilized to obtain and analyze cellular biomarker data. Imaging types such as the following are frequently acquired: Cell Spreading: Timelapse images of a fix location over time, for example, spaced at 3 minutes between images over an hour, resulting in a total of 21 images per location is taken. RFV: Timelapse images of a fix location over time, for example, spaced at 3 second intervals between images over a span of 1.5 minutes, resulting in a total of 31 images per location is taken. Cell Tracking: Timelapse images of a fixed location over time, for example, spaced at about 4 minute intervals between images. In one embodiment, this cycle is performed for every 2 locations the RFV process has cycled through, so the 4 minutes includes the 2X RFV process. Fluorescent images: In one embodiment, 4 images are taken at each location, and each image is taken when being illuminated with a different wavelength of light. Different time intervals for each of the foregoing types of imaging, including both intermediate intervals and total time span, is contemplated and is often optimized for a desired biomarker. Moreover, fluorescent imaging often involves excitation of a fluorescent marker with illumination from one or more excitation signal sources, each excitation signal source having a pre-selected wavelength or spanning over a range of wavelengths. The wavelength of the excitation signal is often correlated with the fluorophore that is to be excited to provide for optimal excitation and emission. One or more detectors are often provided capable of detecting emission signals within the emission wavelength or range of emission wavelengths. Moreover, when multiple different fluorescent targets are illuminated for excitation, the target fluorophores excitation wavelengths, and emission profiles are selected to maximize the wavelength separation of the peak emission profiles to enhance detection of discrete emission signals.

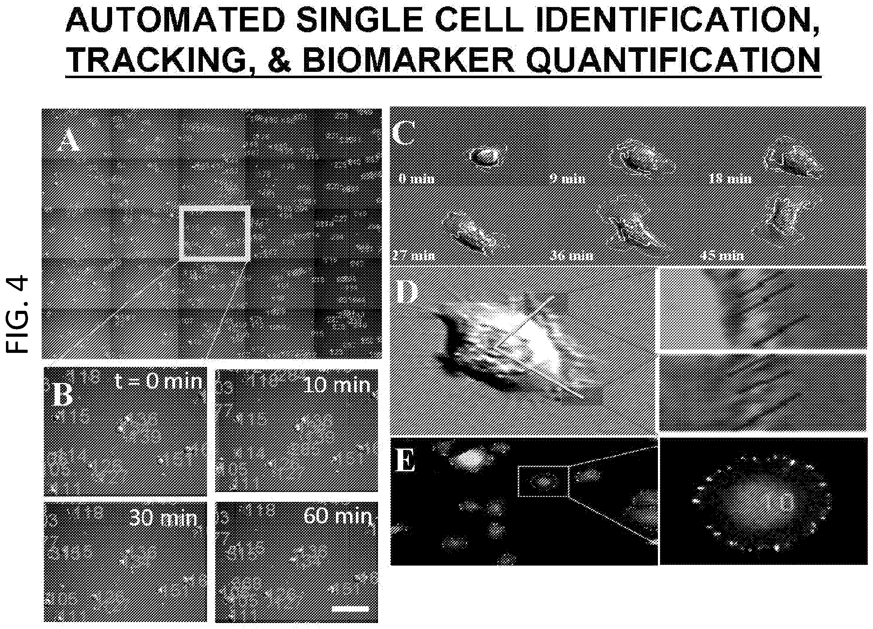

With further reference to Fig. 10, often the imaging chamber is too large to be imaged at the appropriate magnification to identify the desired biomarkers and therefore must be divided into coordinates to provide for imaging of multiple sectors (i.e., imaging spots) that can be montaged to create an image of the whole imaging chamber or selected area of the chamber. *See, e.g.,* Fig. 4A, which provides an exemplary image montage. In this process, a cell coordinate system is often established for tracking (utilize cell tracking images) utilizing one or more of the following procedures:

Montage of multiple imaging spots: In certain embodiments, at any time t, the desired viewing window is subdivided (optimized based on desired or actual cell density) into an m-by-n dimensioned grid. Each of the sectors of the grid is individually imaged, and the image is stitched back together to provide a full field of view of the growing environment of a cell.

Mask out background to isolate cells: In certain embodiments, an image typically consists of cells, some tissue debris, and random artifacts on the substrate. To eliminate non-cell objects, areas outside the cell are blacked out. Doing so focuses the analysis program at the proper locations containing live cells and reduces or prevents artifacts from being misidentified as cells in the downstream process.

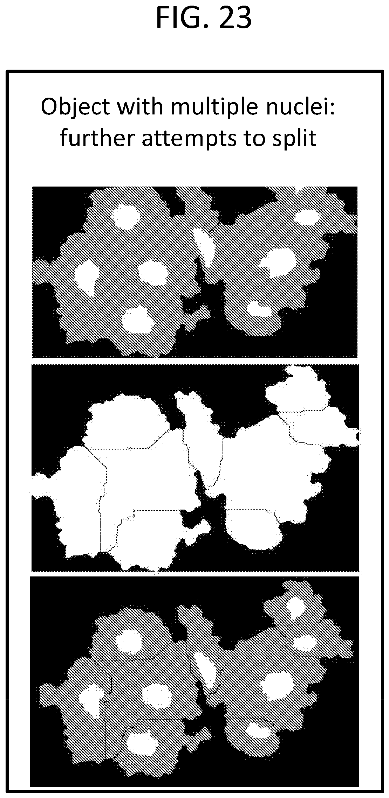

Split up groups of cells: Over the course of the culturing and imaging process, some cells have a tendency to cluster together. Since tying each measured marker to its respective cell is critical to the diagnostic process, it is necessary to segment these cells further and not consider them as a single entity. *See, e.g.,* Fig. 23.

Record cell migration positions: Over the course of the culturing and imaging processes, a cell may migrate across the field of view. The present methods and systems permit tracking of these cellular migration movements and permit accurate measurement of one or more biomarkers over time, even while the cell migrates.

Measurement of Biomarkers: Utilizing the RFV images, cell spreading images, and also cell tracking images, biomarkers tied to each cell's variations in phenotypic behavior over time can be extracted from the images in certain embodiments. In addition, certain protein-based markers can only be visualized when tagged by fluorescent antibodies after fixation of the cells. Each tagged protein is often visualized at a predetermined wavelength, which requires in certain embodiments that each wavelength excitation is cycled through at each location.

Output: In certain frequent embodiments, the output of imaging provides data grouped into the m-by-n array, where the rows include cell IDs (i.e., cells identified during the cell tracking process), and the columns include the individual biomarkers measured for each of those cells.

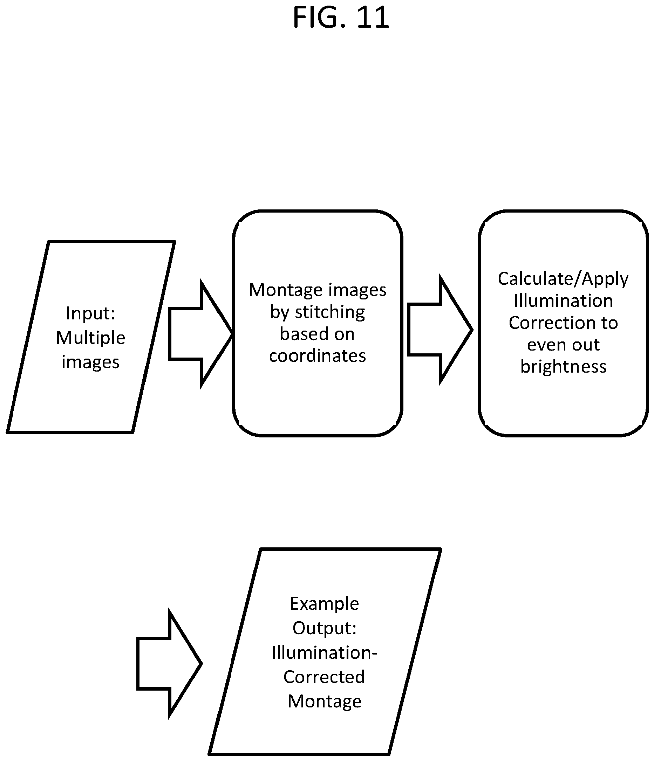

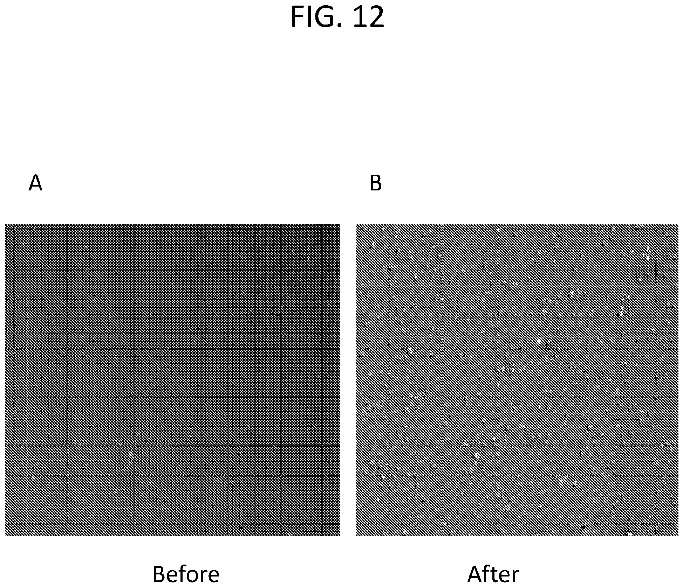

With reference to Fig. 11, an example workflow of a montaging operation is provided. This process involves, as noted for example, combining multiple images or different portions of the imaging chamber into a single larger image, which enhances the ability to track cell movements and the matching of biomarkers to known/identified cells in the chamber. In an exemplary process, cell tracking images are taken and stitched together based on the coordinates of the images. In certain embodiments, illumination across the total field of view (as represented by the montage) is uneven within sections and a correction factor is calculated to smooth out the brightness across the whole montage. *See, e.g.,* Figs. 12A before applying the correction factor versus Fig. 12B after applying the correction factor. The illumination can be corrected for each image over time.

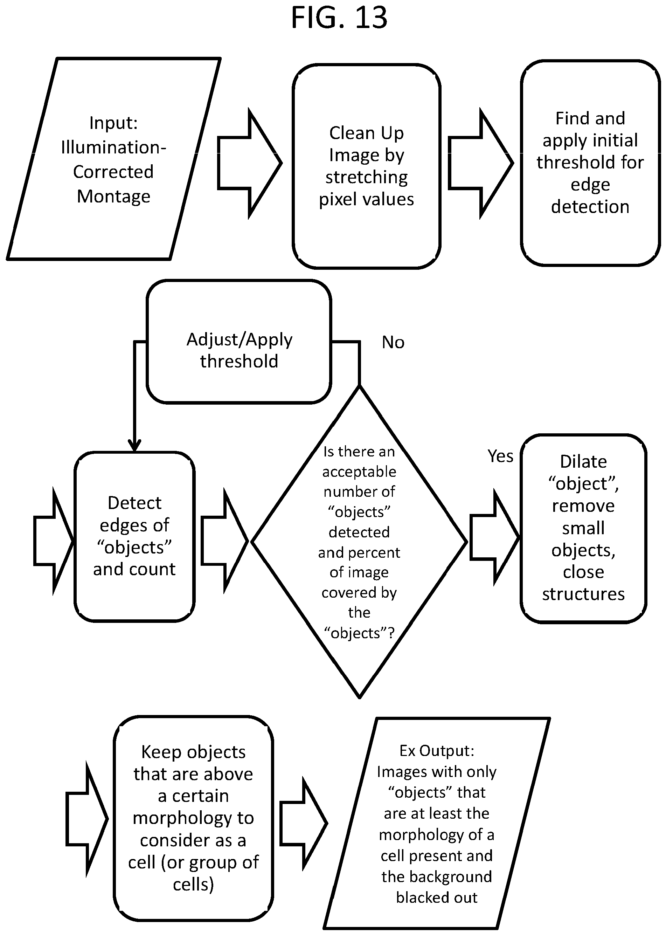

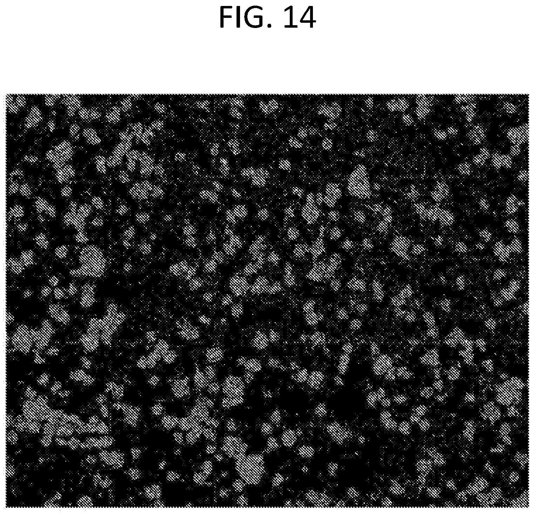

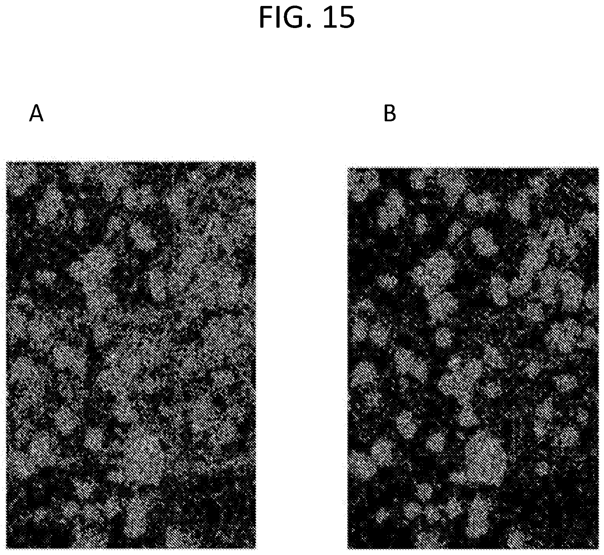

With reference to Fig. 13, a cell masking process flowchart is exemplified to isolate areas of the images that contain cells and thereby enhance cell tracking and analysis accuracy. Programs such as MATLAB and C++ are useful for cell masking, among other imaging procedures detailed herein. For example, illumination is corrected (e.g., at each timepoint of cell tracking), images are cleaned up by stretching pixel values, and an initial threshold for detecting edges of objects within the image are defined and applied across the image. In certain embodiments, a method of detecting the edge of an object (e.g., a cell) is provided, such as a Canny edge detector, to locate a border of an object in the viewing field. Thereafter, after an edge of an object is detected, it and similar objects are counted. As represented, for example, in Fig. 14, a filter process on an image of multiple objects is shown. As a generally expected range cell size is known, and when cells are seeded at a predetermined density, there are a desired or expected number of objects in the viewing area. This often includes a percentage range of the viewing area occupied by the objects as well as an expected level of background image noise. In certain embodiments, if these expectations are met, imaging thresholds are adjusted, reapplied, and object edges are counted again. Fig. 15 provides an image having both initial and final thresholds on an image of an exemplary chamber, demonstrating clear delineations between cells.

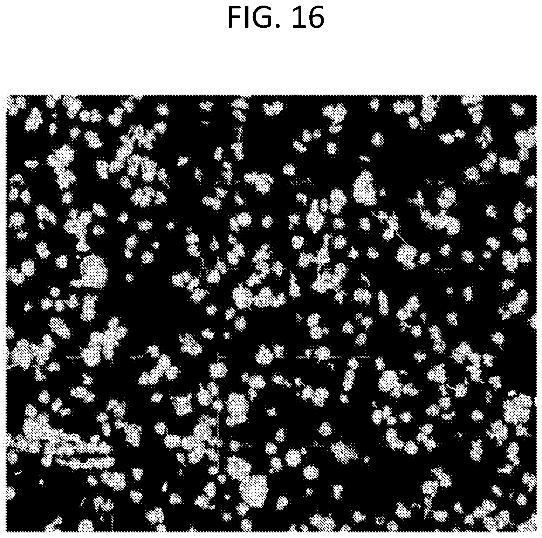

Thereafter, often the object or image thereof is dilated to remove small objects and other non-cell structures from the view. When an acceptable viewing threshold is applied, all identified objects are smoothed and their edges blurred, for example, to connect tightly packed objects to form larger structures. Objects that are isolated from other objects and are of a non-expected cell size are considered noise and removed from the image. Fig. 16, for example, provides the results of a first stage of a cleanup of invalid objects in the background of an image.

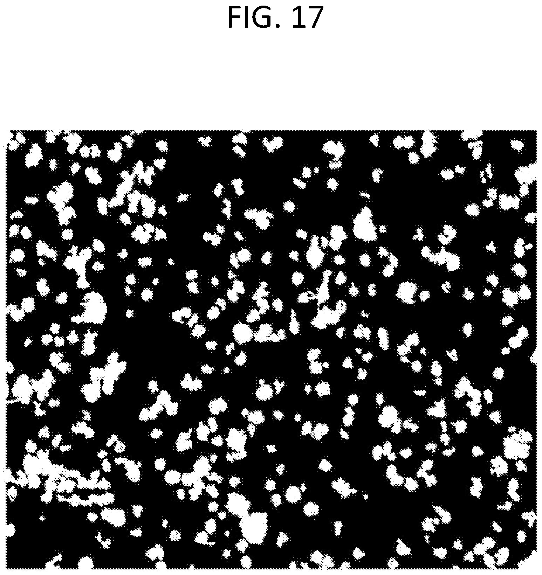

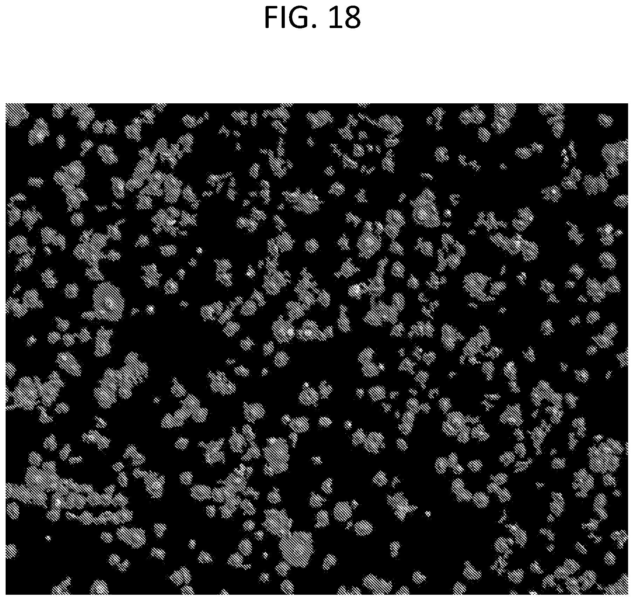

At this stage, images are mostly devoid of noise outside the area of the desired objects, but noise may remain within one or more object since the blurring does not perfectly connect neighboring objects. To remove image noise within the object and provide a continuous and viewable area within the object, the color of the image is optionally inverted in certain embodiments such that the background and noise are white, and the structures are black. Small objects that are noise may be thereafter be removed from the image. This process of inverting the color of the image is similar to the above-noted methods of noise removal to occur within the image of individual objects. Due to the montaging process, if undertaken, edges bordering neighboring images may be misidentified as objects. As such, the regions of white that now define the background is often expanded to fill in those objects and covert them to background noise. At this point in this exemplary process, the image is mostly or completely composed of only white larger structures and a black background. Another inversion of color is thereafter undertaken, and white areas are dilated to fill in holes within the structures. Small objects are then removed to reduce or eliminate lingering artifacts and yield a mask that isolates the areas containing cells. Fig. 17 provides an example of a final mask prior to being applied to a cell. Fig. 18 provides a montaged image having clearly delineated cells after applying the mask to the objects.

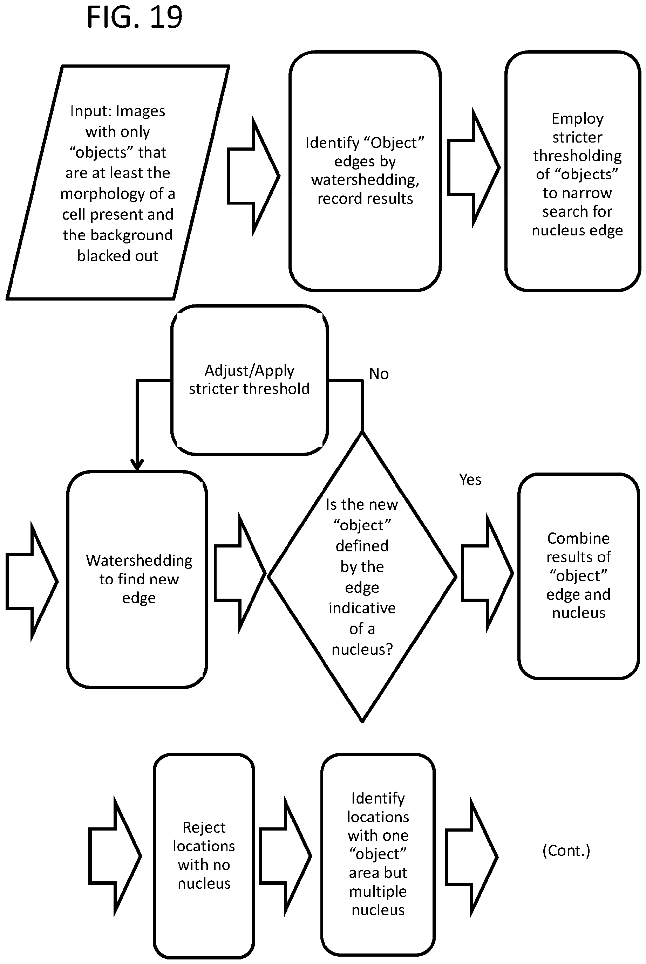

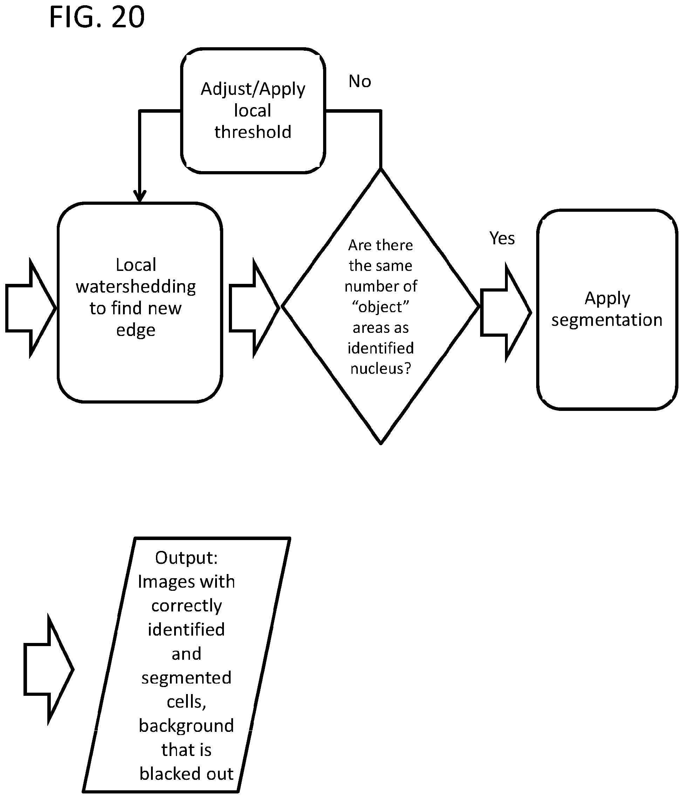

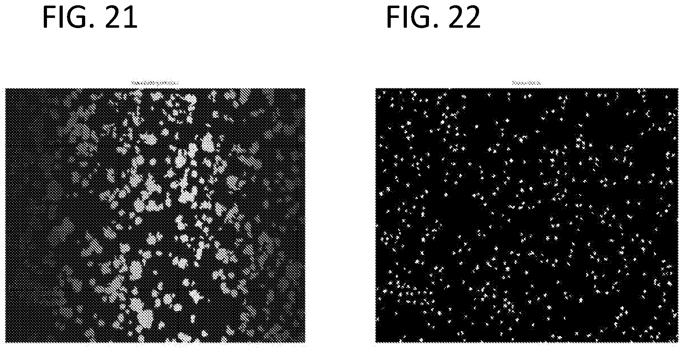

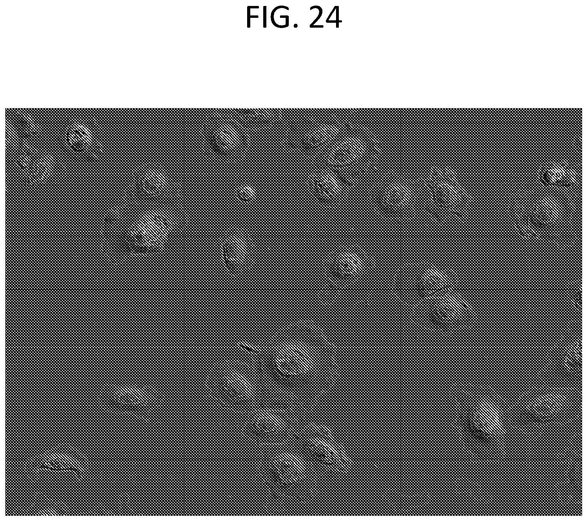

With reference to Figs. 19-23, an exemplary flowchart describing one method of splitting groups of cells is provided. This process is provided in certain imaging embodiments since, over the course of observations, cells may come in contact with one another and become clustered. Identifying locations of clustering and separating the cells increases accuracy of downstream biomarker measurement. In one exemplary process, the edges of objects are identified to find the nucleus of each cell, which is one true indicator of whether an object is a cell or not. In certain embodiments, a watershedding technique is utilized in this process to identify local object edges. Fig. 21 provides a graphical representation of a watershedding technique. A stricter threshold to re-identify object edges is then applied. This process typically yields the identification of object edges that are larger, greater, or more expansive than the area of the cell nucleus. As such, a stricter threshold is often employed to narrow the search for the nucleus edge. An edge detection technique, for example as explained above, may be repeated in this process in a manner that results in the identification of an area matching the morphology of cell nucleus. Fig. 22, for example, provides a montage having only the nucleus of cells shown. The results of structure edge and nucleus location are often combined. Objects in the image having no nucleus may be identified here and removed from the collected data. Often, objects with multiple nuclei are identified. However, as a cell generally only has a single nucleus, an object with multiple nuclei is often interpreted as containing multiple cells packed together. Such multiple-cell objects are often segmented into individually identified cells. One example of a technique use for such segregation is a watershed technique and/or threshold adjustment cycle, applied to these areas containing multiple-cell objects that may be performed or repeated until the number of unique objects equals the number of nuclei. Often, the resulting segmentation is applied and drawn into the image. For example, see Figs. 23 and 24.

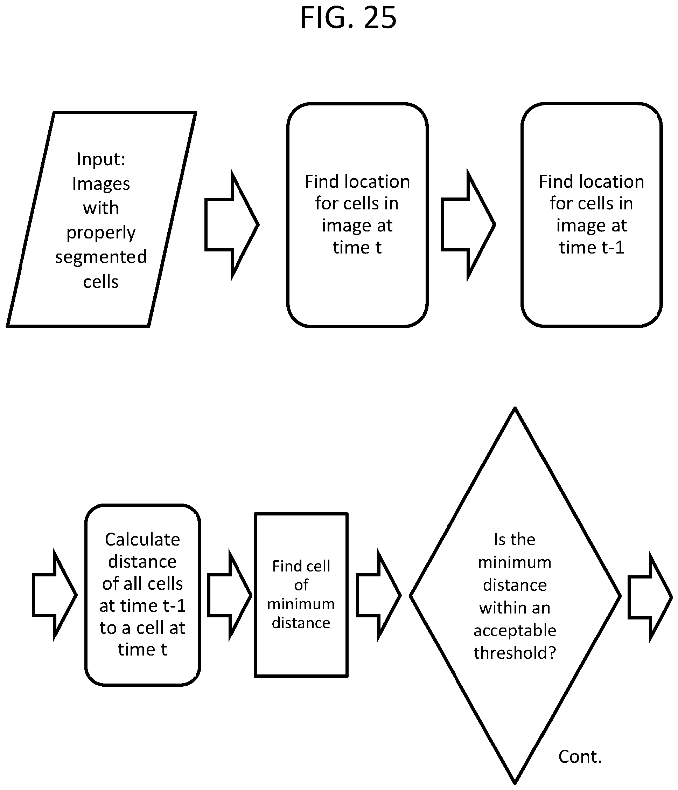

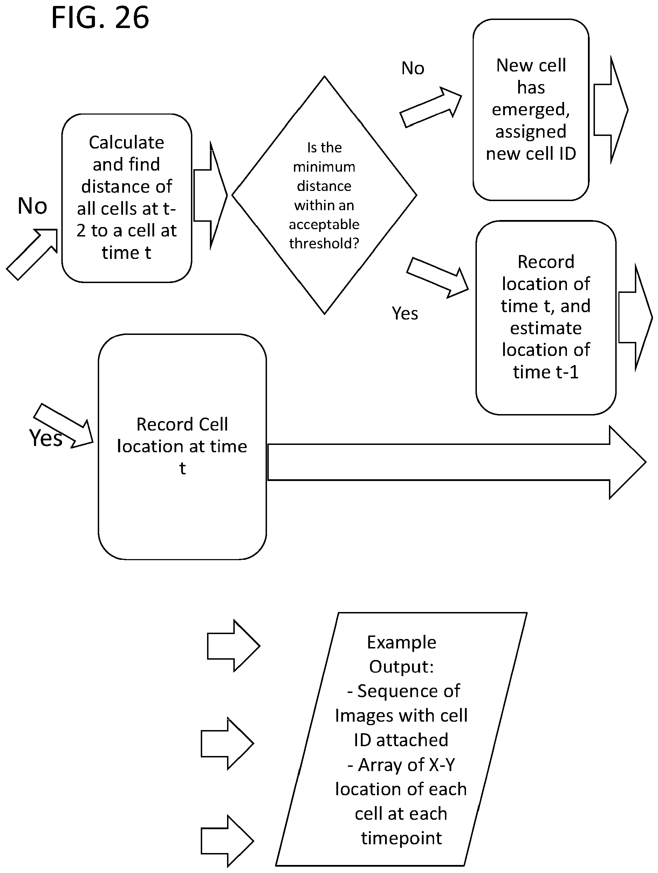

With reference to Figs. 25-26, an exemplary flowchart describing one method of tracking cell movements in an imaging area is provided. The inventors have observed that cells will move over time during the observation process. Tracking cell movements permits markers to be matched to the appropriate cells over time. In certain embodiments, migration velocity is monitored. Migration direction, migration distance, persistence length are related biomarkers that are monitored in certain embodiments. For example, cell locations in an image at time t/t-1 are determined and the distance of cell travel, if any, is calculated from t to t-1. In these embodiments, the absolute position of a cell in the image at time t is determined and recorded. In order to find out where the cells came from, for example, the absolute position of the cell in the image t-1 is determined and recorded. For each cell at time t, the distance of one cell to other cells or another cell, or another reference point, at time t-1 is calculated. In certain embodiments, an inquiry about whether an acceptable a minimum distance threshold is met is provided. If one cell in time t-1 is within the threshold, then the location of the cell at time t is recorded. If more than one cell in time t-1 is within the threshold, then the location of the cell t is recorded into the cell at time t-1 that has the closest match in morphology among all cells meeting the distance threshold. If no cells at time t-1 are found within the threshold: the program optionally looks back at time t-2 and repeats the search. The same decision tree from the step above may then be applied. However, the position at time t-1 will be estimated based on the average movement from t-2 to t. If no cells are found to be matching at t-2, then it is often determined that a new cell has emerged, and a new cell ID may be assigned to that new cell. Two outputs are often provided. One output may be a sequence of images with a cell ID attached to a cell. The other output comprises an m-by-n array in which the rows comprise cell IDs and the columns comprise absolute X and Y axis locations of specific cells.

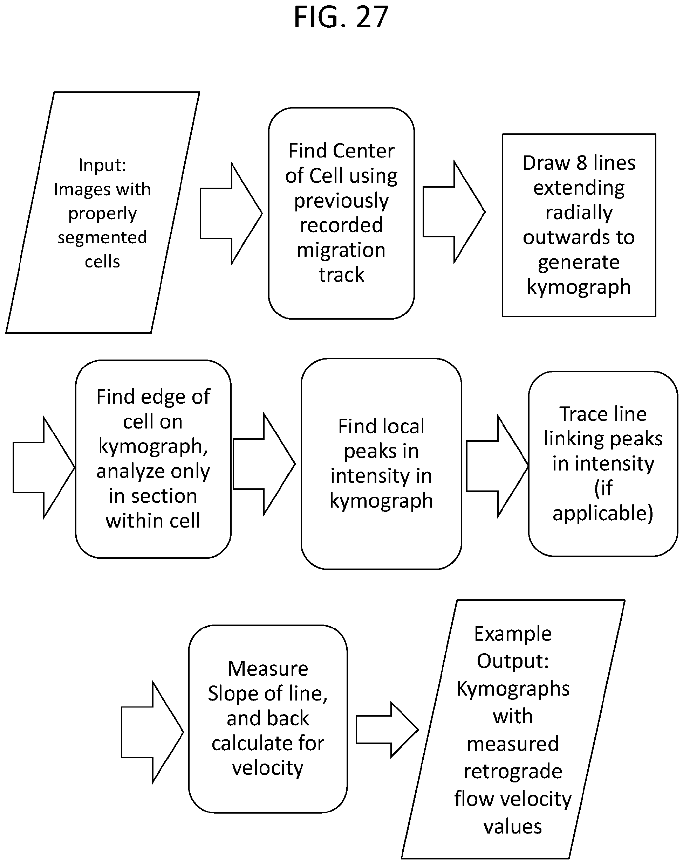

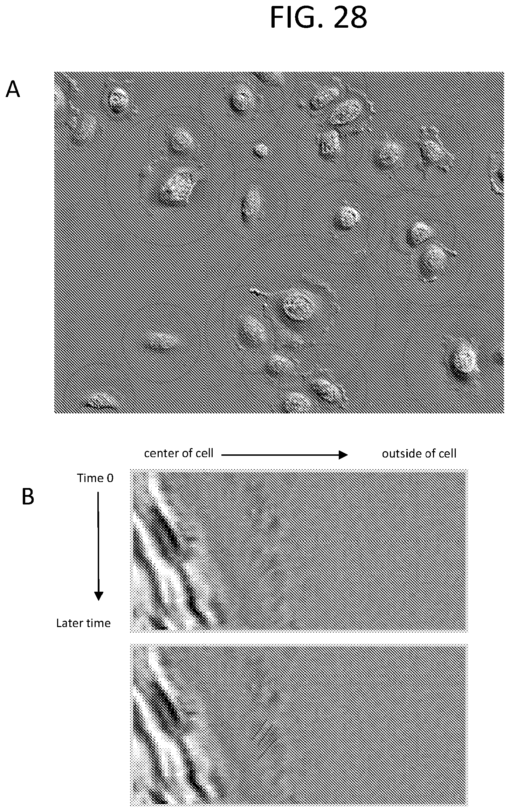

With reference to Figs. 27-28, an exemplary flowchart describing retrograde flow velocity (RFV) measurement is provided. In certain embodiments, from the center of the cell as identified by the cell movement tracking, multiple lines (e.g., up to 8, or more) extending radially outwards from the cell are drawn in the RFV images to generate kymographs, which are graphs with the x axis representing distance from the center, and y axis representing progression of time, from top to bottom. Fig. 28A provides an example of such line drawing on a cell image for RFV measurement. To narrow the search for the retrograde movement in certain embodiments, the areas indicating the nucleus and outside of the cell are cutoff from the kymograph. Often, the nucleus and non-cell area provide distinct grayscale properties versus the cytoplasm of the cell and can be easily identified. From this selected location, local peaks in grayscale intensity in the kymograph can be found. These peaks are often linked together from the top right to the bottom left of the kymograph, which is indicative of a retrograde flow line. If such a line exists, then the slope of the line is measured, and can be back calculated for the velocity of the retrograde flow. One exemplary output includes an m-by-n matrix with the rows being the cell ID and the columns being the retrograde flow velocity values. Fig. 28B provides one example of a kymograph having RFV lines highlighted.

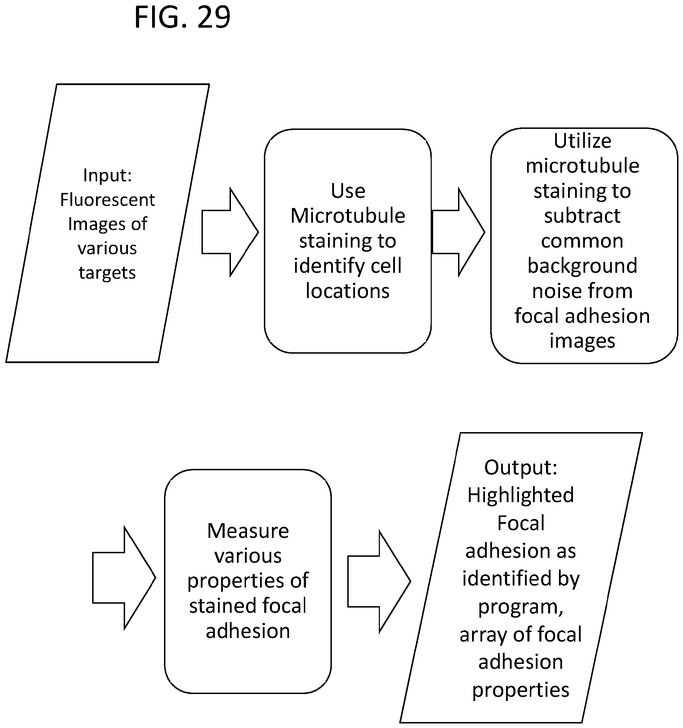

With reference to Figs. 29-30, an exemplary flowchart describing Focal Adhesion measurement is provided.

In certain related embodiments, microtubule staining is utilized to identify cell locations. For example, a fixation step may occur between live cell measurement and cell marker measurement that may slightly alter cell morphology. Using microtubule staining is a good indicator of where the locations of the fixed cells are since microtubules are present throughout a cell body. Beginning with a raw image, the intensity of the whole image is scaled up until a staining signal can be seen. This is preferred because saturated pixels where fluorescent protein aggregates are located may overshadow the actual signal. The pixel intensity is then stretched to set related thresholds. A cleanup of the signal is often performed to reduce noise in the background, for example by using Wiener Filtering. Next, to distinguish the location of the background, the image is often binarized, changing the location of cells to white and the background to black. The areas containing cells are often then subtracted from the image, leaving an image with only background and small artifacts. This image is then subtracted from the noise-reduced (e.g., Wiener Filtered) image, yielding a high contrast image including valid signals. In addition, utilizing a similar method to generate cell masks described above, the image is binarized to separate cells, dilated to smooth edges, small objects removed, and the remaining regions of white will be considered for FAK analysis.

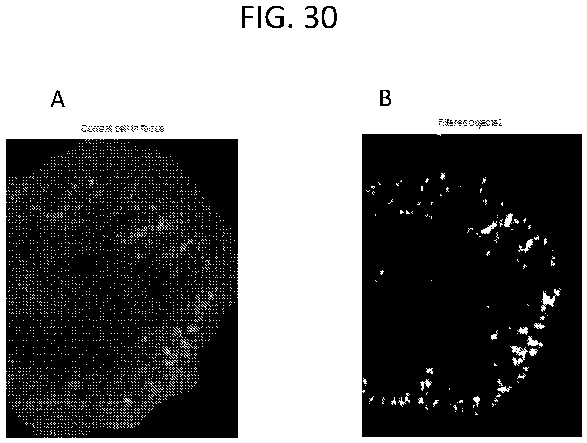

To analyze FAK staining, many similar processes described above may be repurposed for identifying staining location and size within a cell. For example, beginning with a raw image, the intensity is scaled up to increase the signal strength, and the intensity range stretched to set limits. Again, the phenomenon of bright aggregates may be observed. Since bright aggregates may affect an interpretation of FAK staining, these locations are often masked out. As such, the masking procedure similar to that described elsewhere herein may be utilized to cover locations of bright aggregate. The FAK image may be combined with the bright aggregate mask, and its intensity restretched. The FAK image may then be subtracted with the intensity-stretched microtubule staining image to remove any artifacts and background noise common to both images. Since regions with high density signal may appear brighter than low density areas in an image, a Gaussian filter may be used, for example, to correct for any background illumination differences. The image of background illumination may then be subtracted from the FAK image with the bright aggregate mask, and the product provides the basis for further FAK detection. For example, from a full field of view image, each cell may be isolated locally for FAK analysis. Similar processes described herein may often be applied here. For example, the intensity may be stretched, Wiener Filtering used to reduce noise, background illumination corrected by Gaussian filtering, the image is binarized, small objects removed, large structures filled in to have a continuous area, watershedding iterations performed to segment larger FAK stains, and finally various properties of each FAK point measured. One output here is with images having FAK points colored in, and an m-by-n array in which the rows are the cell ID and the columns are the various properties of the FAK stain such as area/size, intensity, number within the cell, distance from center of the cell, etc. Fig. 30A and 30B provide before and after images of FAK analysis.

### *Transformation of biophysical metrics into predictive indications*

In certain embodiments, a representation of a cell or collection of cells from a subject is provided comprising an identification or measurement of a biomarker. More frequently, the identification or measurement of a plurality of biomarkers in each of a plurality of cells is provided through methods described herein. As the behavior and characteristics of a cancer cell can be complicated, processing multiple biomarkers is often preferred since frequently a single biomarker may not capture the complex nature of a cancer cell. Moreover, cancer cell and tissue samples are known to be heterogenous, containing both benign and cancer cells. This complicates the process of identifying cancer cells for observation out of a larger population of benign cells. Overall, therefore, it is a major object of the present disclosure to provide the automated measurement and evaluation of a variety of biomarkers in each of a plurality of cells simultaneously or in sequence. Supervised, semi-supervised, and/or unsupervised machine learning algorithms are provided herein to achieve these objects. Unsupervised learning is, for example, a technique of finding structure in data when you do not necessarily know the desired output. Some examples include clustering, Hidden Markov models, principal component analysis, singular value decomposition, or a Self-organizing map. These methods and systems provide for the ability to automatically identify abnormal cells such that future processing may only occur on these cells. These cell-level results are often combined to provide a patient or test compound level output.

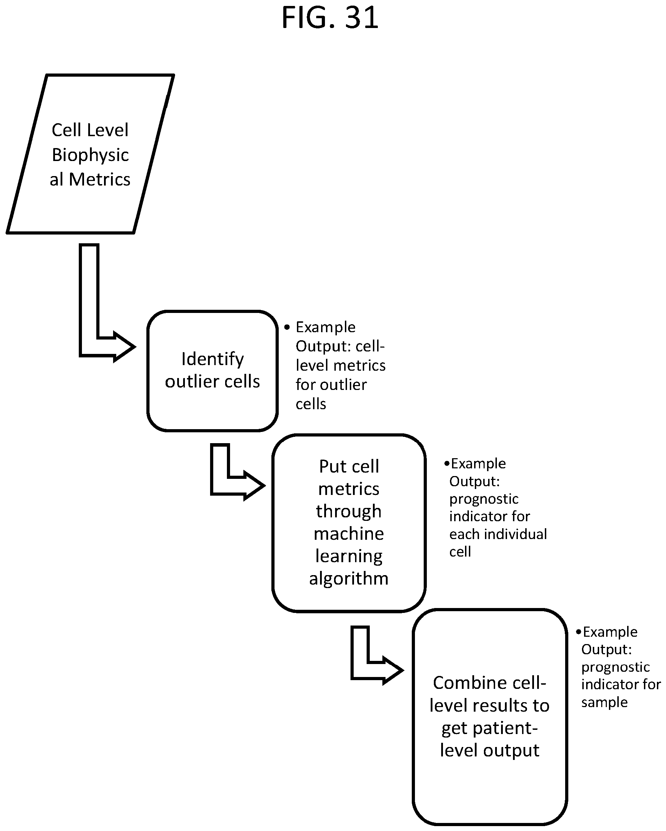

With reference to Fig. 31, an exemplary flowchart describing biomarker analysis is provided. As an exemplary initial step, the m-by-n array output(s) from the imaging process and optionally any pathological data are provided for each sample. Relatively abnormal cells are then identified. For example, in a sample, there may be a mix of normal cells and abnormal cells. In frequent heterogeneous populations of cells, normal cells often outnumber abnormal cells. To enhance the analysis, frequently only cells that are abnormal as compared to the general population are considered in the biomarker analysis, which often provides clarity and differentiation among samples during analysis. In one exemplary output, an m-by-n array in which the rows are comprised or the IDs of the abnormal cells, and the columns are the biomarkers of those cells is provided. Cell metrics are often run through a learning algorithm involving a training process, test process, and an output. In the training process, abnormal or outlier cells isolated from the prior process, the metrics of those abnormal cells are fed into a machine learning process that recognizes patterns within the various biomarkers and creates algorithms tying the cell's biomarkers to the cell's known or expected pathological outcome or another prediction. The algorithm is often the same for all cells within a test set. This process continuously improves the ability of the machine learning process to perform in the test process. In certain embodiments, all the cells from each subject may be assumed to have the same pathological outcome as those that are evaluated. The test process uses abnormal cells isolated from the previous process, and the metrics for each cell are fed into a trained algorithm, which in frequent embodiments returns a likelihood of a cell exhibiting a certain pathological outcome. As one exemplary output of the test process, an m-by-n array is provided in which the rows are comprised of abnormal cell IDs, and the columns comprise predicted pathological outcome of the cell. Thereafter, the cell-level results are often combined to obtain a patient-level output. For example, the results of multiple cells from the previous processes are summarized to reflect the pathological result describing one patient. One exemplary output comprises a 1-by-n array in which the column provides a predicted pathological outcome of the patient.

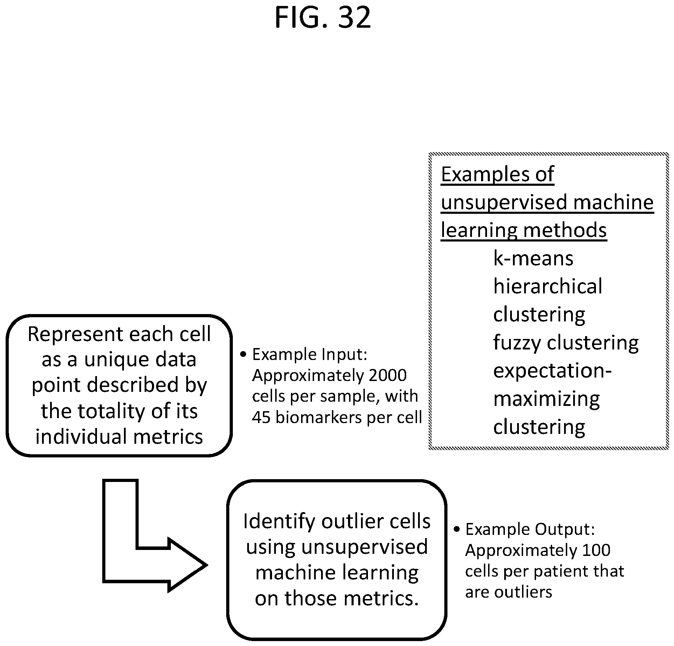

With reference to Fig. 32, a flowchart describing an exemplary abnormal cell identification process flow is provided. For example, in such a process, a population of cells from a subject containing a group of normal cells and abnormal cells is provided. Based on an analysis of biomarkers described herein the inventors have determined that abnormal cells tend to be relatively and detectably different from the normal cells. Each cell, for example, has many (e.g., about 65 or more) biophysical metrics or biomarkers that have been identified and used in calculations described herein. *See, e.g.,* Figs. 3-6. Nevertheless, within subject biopsies for example, the amount of normal cells is greater than that of abnormal cells. As such, methods of separating the abnormal cells from normal cells is provided through supervised, semi-supervised, and/or unsupervised machine learning methods are utilized to enhance signal to noise ratio (the "signal" here representing abnormal cells). Without this type of separation step, most samples will look similar due to the presence of large amounts of normal cells. In certain embodiments therefore, a single heterogenous sample from a subject provides both a control or baseline as well as a test sample. The machine learning methods described herein permit a subject to use her own cells as a baseline for normal vs. abnormal.

Exemplary supervised learning techniques that may be employed include (in addition to others discussed herein) at least the following techniques: averaged one-dependence estimators (AODE), artificial neural network (e.g., backpropagation, autoencoders, Hopfield networks, Boltzmann machines, Restricted Boltzmann Machines, Spiking neural networks), Bayesian statistics (e.g., Bayesian network, Bayesian knowledge base), Case-based reasoning, Gaussian process regression, Gene expression programming, group method of data handling (GMDH), inductive logic programming, instance-based learning, lazy learning, Learning Automata, Learning Vector Quantization, Logistic Model Tree, Minimum message length (decision trees, decision graphs, etc.) (e.g., Nearest Neighbor Algorithm, Analogical modeling), Probably approximately correct learning (PAC) learning, Ripple down rules, a knowledge acquisition methodology, Symbolic machine learning algorithms, Support vector machines, Random Forests, Ensembles of classifiers (e.g., Bootstrap aggregating (bagging), Boosting (meta-algorithm)), Ordinal classification, Information fuzzy networks (IFN), Conditional Random Field, analysis of variance (ANOVA), Linear classifiers (e.g., Fisher's linear discriminant, Logistic regression, Multinomial logistic regression, Naive Bayes classifier, Perceptron, Support vector machines), Quadratic classifiers, k-nearest neighbor, Boosting, Decision trees (e.g., C4.5, Random forests, Iterative Dichotomiser 3 (ID3), Classification And Regression Tree (CART), supervised learning In Quest (SLIQ), SPRINT), and Bayesian networks (e.g., Naive Bayes), and Hidden Markov models.

Semi-supervised learning employs the use of small amount of labeled data together with a large amount of unlabeled data. In certain embodiments, such use of unlabeled data used together with labeled data improves learning accuracy.

Exemplary unsupervised learning techniques that may be employed include (in addition to others discussed herein) at least the following techniques: Expectation-maximization algorithm, Vector Quantization, Generative topographic map, Information bottleneck method, Artificial neural network (e.g., Self-organizing map), Association rule learning (e.g., Apriori algorithm, Eclat algorithm, FP-growth algorithm), Hierarchical clustering (e.g., Single-linkage clustering, Conceptual clustering), Cluster analysis (e.g., K-means algorithm, Fuzzy clustering, DBSCAN, OPTICS algorithm), and Outlier Detection (e.g., Local Outlier Factor).

A variety of exemplary data clustering methods can be utilized here include k-means clustering, hierarchical clustering, fuzzy clustering, expectation-maximizing clustering, density-based spatial clustering of applications with noise (DBSCAN), and ordering points to identify the clustering structure (OPTICS).

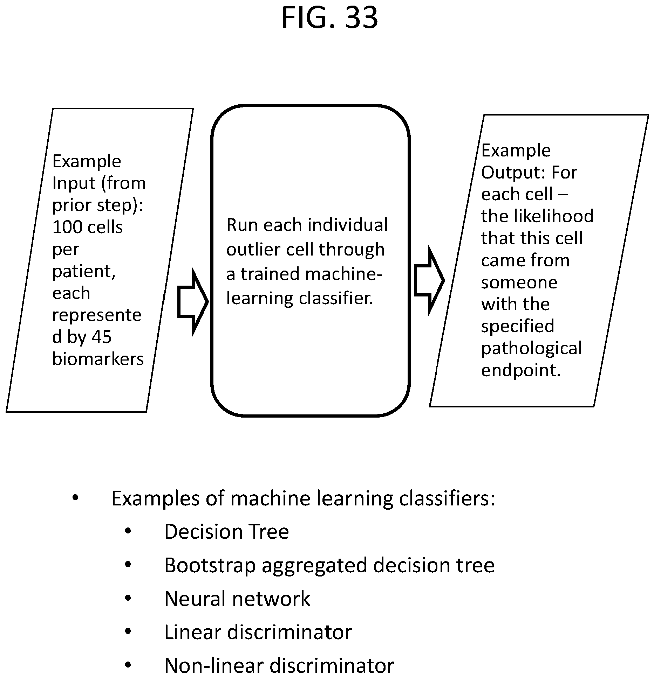

With reference to Fig. 33, a flowchart describing an exemplary analysis of abnormal cells with a machine learning method is provided. In one embodiment, a machine learning classifier is provided based on, for example, a surgical pathology report and associated histological analyses related to tested samples. The sample and results of the methods described herein are processed through this classifier to produce a likelihood that each cell came from a patient with the selected pathological endpoint. Results from imaging abnormal cells may then be fed through a classifying algorithm that correlates each biomarker characteristics of the cell with clinically relevant pathological indicators. The classifying algorithm being frequently previously trained with a training set of samples with known pathological indicators or biomarkers. The algorithm, based on the training samples, often generates a set of equations, rules, and methods that link biomarker patterns with specific pathological indicators. Often, these algorithms are generated through machine learning methods, such as bootstrap aggregated decision tree, neural network, linear discriminator, non-linear discriminator, and/or a Naive Bayes classifier. One exemplary output for each cell after it passes through the classifier is a number describing the likelihood of that particular cell to be positive for a certain pathological indicator. Using this trained machine-learning algorithm, the inventors have been able to take a sample with unknown pathology results and provide a likelihood that it fits the model of samples that have the pathology results in question.

Based on the machine learning tools described herein, methods are provided herein to recognize patterns in the imaged biophysical metrics. This process, for example, associates these patterns with known pathological outputs associated with samples. Certain examples of actual physical endpoints include Lymph Node Positive, Seminal Vesicle Invasion, and Positive Surgical Margin. Using patterns that are associated with known physical endpoints, the methods and systems described herein often provides a confidence that each individual cell input fits the model of the cells that are known to be associated with those endpoints. Moreover, the present methods and systems are capable of generalizing - for each physical endpoint, an output the confidence that an input cell belongs to a patient that has that physical endpoint may be provided.

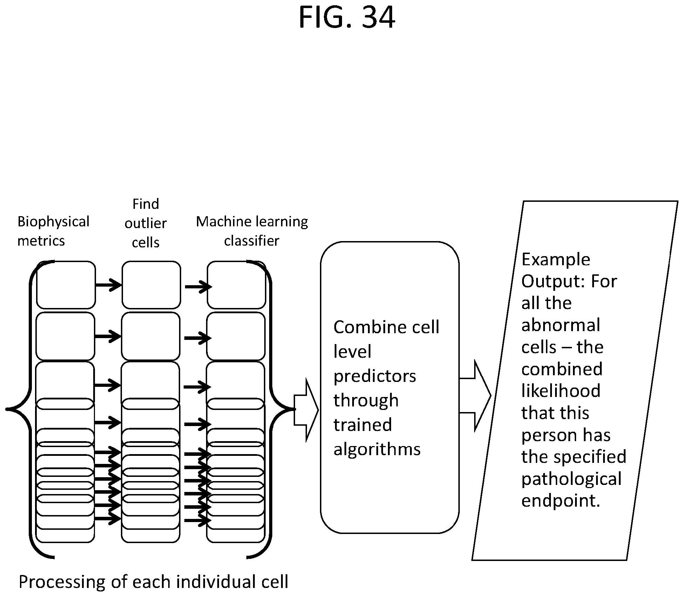

With reference to Fig. 34, a flowchart describing an exemplary process of combining cell level data to provide a subject level output. Overall, this process is done to combine cell-level data in a trained manner to generate sample-level and subject level predictors of pathological output. For example, in certain embodiments, the final step in the process is often the summarization of the data pertaining to all the analyzed cells, each with multiple predicted pathological outcomes, which describes the subject that provided the sample. In certain embodiments, the cell level data may be summarized to provide a single number or term per individual pathological outcome or combination of pathological outcomes as analyzed in the cell level data, per subject. A variety of various methods may be applicable at this step, including manual methods such as thresholding, mean, median, variance, percentage over a threshold, cluster size, etc., and machine learning methods similar to the those described in connection with cell-level analysis.

## Sample Types and Applications

The present methods, systems, and devices are not intended to be limited to specific sample types or tissue types. Live cell analysis methods are presented herein, which may be applied to samples of or derived from tissues or fluids. Both animal and plant cells may be evaluated according to the methods described herein.

For example, prostate tissue or cells derived from prostate tissue may be utilized as described herein. Cells from or derived from bladder, lung, kidney, breast, ovarian, uterine, colon, thyroid, or skin tissue, or tumors associated with the genito-urinary tract or other tumors, may also be analyzed according to the methods described herein. Blood, blood components, urine, bone marrow, bile, lymph, cerebral spinal fluids, among other biological fluids are also candidate samples.

The sensitivity and specificity numbers (as outlined in the equations below) described and obtained using methods and systems described herein, provide a predictive model for cell behavior. In certain frequent embodiments, a diagnostic tool embodied within these systems and methods is provided. In other embodiments, a prognostic tool embodied within these systems and methods is provided. Often, the presently described systems and methods are used to monitor the health or treatment of a subject.

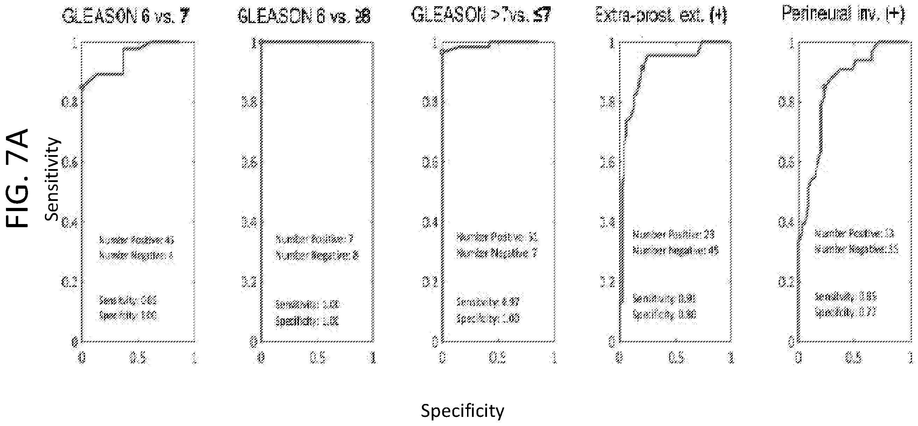

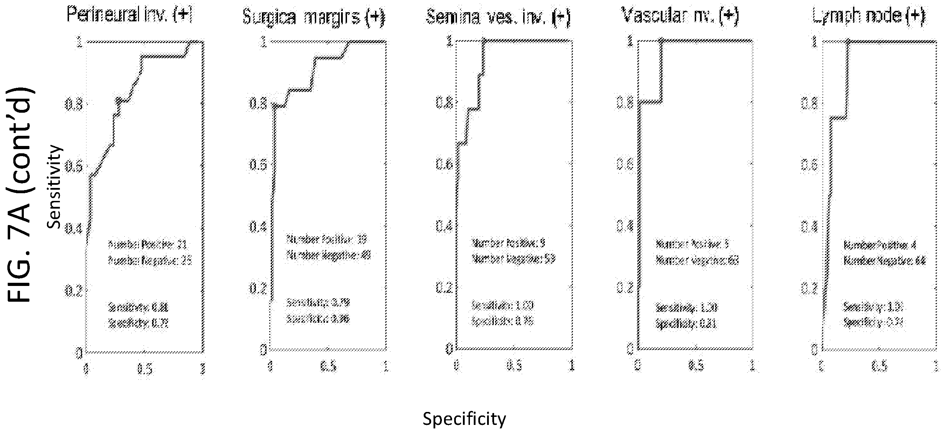

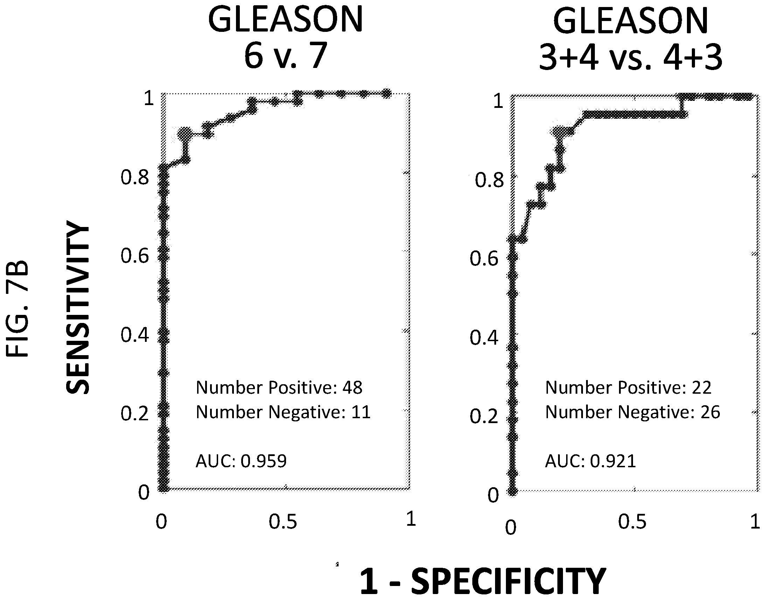

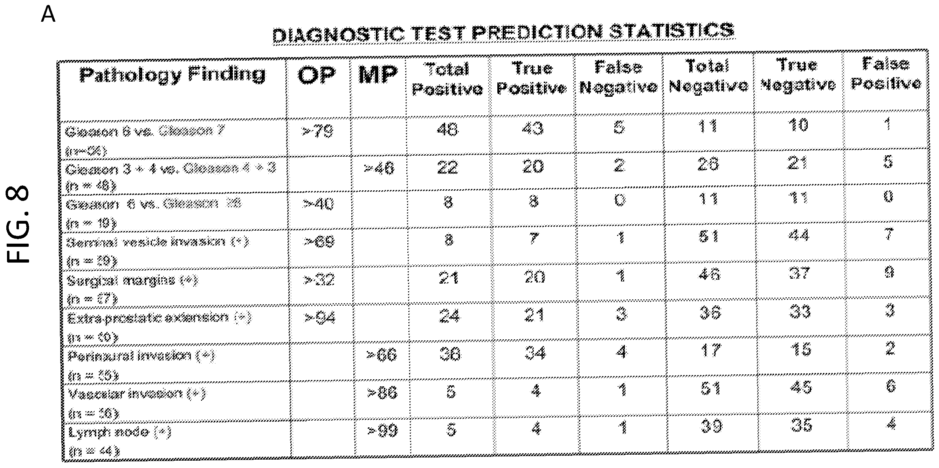

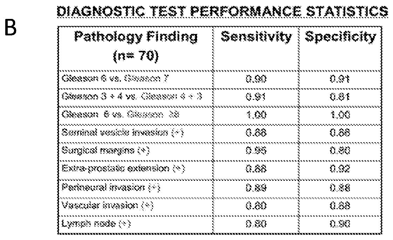

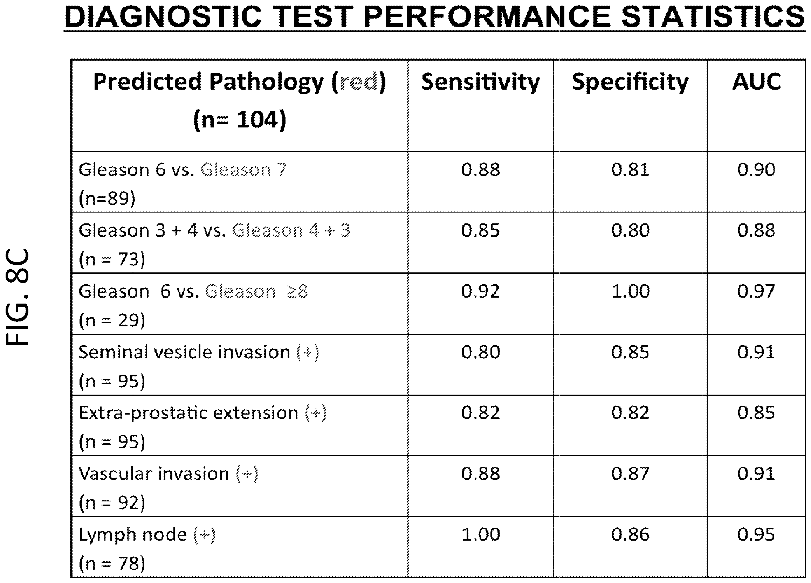

In a particularly preferred embodiment, a prostate cancer diagnostic having the capability to predict and/or adjust pathologic findings (i.e., Gleason Score and other established clinical nomogram, tumor grade, cancer staging or grading system, or pathological score) is provided herein. At least Figures 5-8 present clinical data generated using the methods, systems, and devices described herein. With regard to Figures 7 and 8, "Gleason 6 vs. Gleason 7" denotes predicting Gleason 7 patients from a set of Gleason 6 & Gleason 7 patients. In addition, "Gleason 3+4 vs. 4+3" denotes predicting Gleason 4+3 patients from the set of all Gleason 7 patients.

$$\text{sensitivity} = \frac{\text{true positives}}{(\text{true positives + false negatives})}$$

$$\text{specificity} = \frac{\text{true negatives}}{(\text{true negatives + false positives})}$$

The LAPP describes the extension of tumor in the prostate capsule and seminal vesicles, and the MAPP describes invasion into peripheral systems such as blood, lymph and/or bone. *See also* U.S. Patent Application Pub. No. 20130237453. LAPP & MAPP calculations, for example, are made using algorithms described herein. As depicted in Fig. 8, for example, LAPP and MAPP values represent predictive thresholds of disease status in connection with prostate cancer.

Although diagnostic and prognostic applications of the present methods, systems, and devices are described throughout the present disclosure, it is not intended to be so limited. In particular, the presently described systems and methods are useful for drug screening. In such applications, the activity of a composition or a formulation (e.g., small or large molecule drugs) on biomarkers in live cells is observed, analyzed, and the meaning of the effect is restructured into useable information for decisions related to the activity or expected activity of the composition or formulation. In a similar application, the presently described systems and methods are useful to evaluate the effect of a population of live cells in the presence of a diagnostic composition or device.

## *Drug Screening*

Depending on the candidate drug to be tested, the presently described methods, systems, and devices can be used to observe if the addition of drugs have an effect (intended or otherwise) on, for example, a tissue samples or other samples. For example, a prospective cancer drug can be added to cells as described herein to observe whether the drug affects cell metrics (e.g., biomarkers, prognostic indicators, etc.), that correlate to cancer staging (e.g., LAPP and MAPP), or other metrics, which are indicative of a change in single cell behavior or sample population dynamics (e.g., cell level or subject level).

Analytical methods, inclusion criteria, number of samples required and other test statistics for drug screening are similar to the setup for other methods described herein, e.g., prostate cancer. However, in drug screening the general outcome is not restricted to cancer or non-cancer; rather, it merely needs to be or include, for example, contrasting outcomes that are reflective of a drug's ability to effect a change on the samples. As described previously, the screening may utilize a suite of biomarkers and predicted outcomes that is similar or the same as described herein, or may be newly developed with the user in a separate process or as a result of the drug screening experiment.

## *Biomarkers and reagents*

A variety of biomarkers are detectable and measureable using the imaging and analysis methods and systems described herein. Available and contemplated biomarkers for use in the presently described systems and methods include those set forth in U.S. Patent Application Pub. No. 20130237453.

These biomarkers include native attributes of a cell that are identifiable using methods and systems described herein, with or without the use of additional reagents. Biomarkers also include attributes of a cell that are identifiable through subjecting the cell to a particular stimulus or reagent. Most frequently, the biomarkers detected and measured according to the methods and systems described herein are correlated in a regimented manner with a disease state such as cancer, or a specific cell transformative or cell proliferative disorder in a subject. Also often, the biomarkers detected and measured according to the methods and systems described herein are correlated in a regimented manner with the activity of a drug such as a small or large molecule drug on the cell being imaged.

One or more biomarkers may be evaluated when imaging a cell, particularly a live cell. These biomarkers are imaged over time to capture changes in these biomarkers over a measured time period. For example, imaging of one or more biomarkers present in a cell or collection of cells may occur periodically over the course of one, two, three, four, or five minutes, or more. In one embodiment, images of the one or more biomarkers occurs every fiofve seconds, but other time intervals may be utilized and are often dictated by the type of biomarker that is being imaged. For example, biomarkers that change relatively quickly over a period of time will occasionally be imaged more frequently than biomarkers that change relatively slowly over the same period of time.

In certain embodiments, images are taken of a cell (or a sample containing a population of cells) at 20-30 distinct time points (e.g., 26 time points). In these multiple images a variety of biomarkers are evaluated for each cell, for example, between 20-30 biomarkers noted herein. Often, the data pertaining to one or more of these biomarkers in each of the multiple images is reduced to create a single number representative of the entire timespan of observation. The data reduction and single number creation here often varies between averaging, standard deviation creation, top quartile selection, etc. The range of these single numbers for the population of observed cells is often normalized to enhance the functionality and results of machine learning and clustering.

Though additional biomarkers are still being discovered or evaluated, an exemplary list of biomarkers contemplated and tested according to the presently described methods and systems includes those set forth in the following Table 1:

Table 1

| No. | Biomarker | Details |
|---|---|---|
| 1 | Cell Area Mean/Median/ Standard Deviation: | Cell area at each time point. |
| 2 | Cell Perimeter Median/Standard Deviation: | Outer perimeter length of the cell at each time point. |
| 3 | Cell Tortuosity Median/ Standard Deviation: | A measurement of roughness of the cell contour. Mathematically defined as the length of the curve over the straight line distance between the two ends of the curve. Larger value means higher roughness. |
| 4 | Cell Aspect ratio Median/ Standard Deviation: | Ratio between the major and minor axis of the cell. An aspect ratio of 1 is a circle. |
| 5 | Nucleus area Median/ Standard Deviation: | Area of the cell nucleus. |
| 6 | Nucleus perimeter Median/ Standard Deviation: | Outer Perimeter length of the nucleus. |
| 7 | Nucleus Tortuosity Median/ Standard Deviation: | Roughness of the nucleus contour. |
| 8 | Nucleus aspect ratio Median/ Standard Deviation: | Ratio between the major and minor axis of the nucleus. An aspect ratio of 1 is a circle. |
| 9 | Mean Gray Scale Value (MGSV) median/Standard Deviation: | A measurement of the thickness of the cell. A higher MGSV value signifies a thicker cell. |
| 10 | Migration Velocity median/ Standard Deviation: | Distance the cell has traveled over time. |
| 11 | Retrograde flow velocity: | Velocity at which the outer perimeter of the cell membrane exhibit a retracting motion towards the nucleus. Both the median/standard deviation of all RFV lines measured for a given cell and those of the top 30% values of RFVs for a given cell are considered. |
| 12 | Retrograde flow velocity number: | Number of RFV lines detected per cell. |
| 13 | Focal Adhesion (FA): | Area of the fluorescently tagged focal adhesion. Both the median/standard deviation of all the FA and also the top 30% of the FA values may be considered. |
| 14 | Focal Adhesion numbers: | Number of distinct focal adhesion points measured in a cell. |
| 15 | Focal Adhesion distance: | Distance of each detected focal adhesion from the center of the cell. Both the median/standard deviation of all distances and the top 30% of distances are measured. |
| 16 | Focal Adhesion scaled distance: | Distance of each detected focal adhesion scaled to their cell's radius. Both the median/standard deviation of all distances and the top 30% of distances are measured. |
| 17 | Focal Adhesion intensity | Size and/or modification of sub-cellular protein complex termed "Focal Adhesion." |
| 18 | Spreading Velocity: | Expanding velocity of a cell's membrane. |
| 19 | Endoplasm Area | Area of cell excluding nucleus and cell edge. |
| 20 | Exoplasm Area | Area of the cell edge that is defined by distinct actin structures and dynamics. |
| 21 | Endo/Exoplasm Area ratio | Ratio of Endoplasm and Exoplasm area. |
| 22 | Microtubule density | The density of microtubule proteins and filaments within a cell. |

(continued)

| No. | Biomarker | Details |
|---|---|---|
| 23 | Microtubule orientation | The polarity and direction of microtubules as well as their subcellular morphology or shape. |
| 24 | Integrin-Linked Kinase (ILK) density | The density of sub-cellularly localized ILK. |
| 25 | Phospho-AKT | The protein modification state of AKT that may regulate its activity and localization. |
| 26 | poly(ADP-ribose) (PADPR) | Presence of a specific protein found in a cell termed "PADPR" |

In frequent embodiments, the selection of biomarkers may be adapted based on the machine learning model to incorporate or remove biomarkers based on the particular pathology that is being examined. In one example, biomarkers are selected an optimized for predictions relative to prostate cancer, including diagnosis, prognosis, treatment, or monitoring.

One or more biomarker can be utilized according to the present methods. For example, one biomarker is used to identify outlier cells or generate prognostic indicators. Often, between 2 to 5 biomarkers are used to identify outlier cells or generate prognostic indicators. Also often, 3 to 7 biomarkers are used to identify outlier cells or generate prognostic indicators. Also often, 5 to 10 biomarkers are used to identify outlier cells or generate prognostic indicators. Also often, 7 to 15 biomarkers are used to identify outlier cells or generate prognostic indicators. Also often, 10 to 17, or up to 17, biomarkers are used to identify outlier cells or generate prognostic indicators. Also often, 17 to 26 biomarkers are used to identify outlier cells or generate prognostic indicators. Also often, 26 or fewer biomarkers are used to identify outlier cells or generate prognostic indicators. Also often, 17 to 45 biomarkers are used to identify outlier cells or generate prognostic indicators. Also often, 20 to 30 biomarkers are used to identify outlier cells or generate prognostic indicators. Also often, 40 to 50 biomarkers are used to identify outlier cells or generate prognostic indicators. Also often, 45 or more biomarkers are used to identify outlier cells or generate prognostic indicators. The present methods and systems are not limited by the number of biomarkers that can be evaluated, which can include any relevant biomarker, particularly those generated or identified through the methods described herein.

Any of a variety of diagnostic reagents known in the art may be utilized to render a biomarker detectable. In addition, any of a variety of diagnostic reagents known in the art may be utilized to induce the expression of a biomarker that is or may be detectable. Contrast reagents, stains, chemiluminescent markers and probes, fluorescent markers and probes, and otherwise visually detectable marker reagents or systems, without limitation, are intended to be encompassed by the present disclosure. Vehicles for general or specific delivery of these reagents may vary and include primers, probes, amplification mechanisms, antibodies (including derivatives and fragments thereof), buffers, excipients, and other known reagent delivery mechanisms appropriate for the type of marker being utilized.

## ADDITIONAL ILLUSTRATIVE DATA

Illustration 1

Analytical validation study designed for proof of principle of cancer diagnostic platform and to demonstrate differentiation of cancer and non-cancer samples was conducted. Six sites collected fresh tissue from radical prostatectomy samples and overnight shipped patient samples at 4°C. Live cells were grown for 2 days on a microfluidic device described herein and biomarkers were measured within 72 hours of sample collection.

*Inclusion Criteria:* Males 40-80 years old with Gleason Scores 5-9. No prior treatment for prostate cancer. Plan for prostatectomy as primary treatment. Prior biopsy showed (1) one sextant with at least 10% tumor; (b) at least three sextants positive for tumor; or (c) Gleason score 8-9 with 5-10% biopsy. Exclusion criteria: non-prostate metastatic cancer diagnosis.

*Methods:* This proof of principle study was performed on 70 prostate cancer samples collected post radical prostatectomy according to methods described herein. The test was designed to sustain adhesion and survival of primary prostate tumor cells dissociated from fresh biopsy/surgical samples for up to three days prior to analysis of phenotypic characteristics.

In a related study, live cells from 70 radical prostatectomy procedures were analyzed according to the methods described herein.

*Results: See* FIGS. 7-8. Live normal and tumor cells were distinguished via a set of phenotypic, molecular, and biophysical biomarkers. The primary biomarkers were calculated using objective machine vision algorithms and were used to derive secondary metrics termed MAPP and LAPP. In comparing clinical measures with results of this assay, concordance analysis supports that LAPP and MAPP, were statistically significant in distinguishing between Gleason 6 and Gleason 7 with 90% sensitivity and 91% specificity, and Gleason 7 (4+3) vs. Gleason 7 (3+4) with 91% sensitivity and 81% specificity. Moreover, true positives and true negatives for early pathology and Gleason scores were predicted accurately at >80 percent.

*Conclusions:* This phenotypic diagnostic test generates scoring metrics of MAPP and LAPP that correlate with 1) aggressive Gleason 6 vs. indolent Gleason 6, 2) seminal vesicle invasion, 3) occurrence of margins after radical prostatectomy, 4) vascular invasion, 5) lymph node invasion. These results will further help stratify patient tumors to improve clinical decision-making in low to intermediate-risk prostate cancer populations, and potentially avoid unnecessary surgery or radiation, ultimately leading to improved patient outcomes. The assay strongly predicts Gleason grade in radical prostatectomy specimens and the proprietary predictive metrics for local tumor advancement and metastatic invasion can stratify patients with low and intermediate grade prostate cancer.

The test results demonstrate that the utilized quantitative and actionable phenotypic biomarker panel is applicable in risk stratification in men with, for example, Gleason 6 and Gleason 7 (3+4, 4+3) prostate cancer. The test results also provide results using biomarkers, devices, methods, and systems applicable to other disorders such as cancers, including bladder, lung, kidney, breast, ovarian, uterine, colon, thyroid, skin cancers.

As detailed in FIGS. 8B & 8C, "sensitivity" and "specificity" results describe the capability of the prostate cancer diagnostic test to predict pathologic (Gleason and adverse pathology) findings. Local Adverse Pathology Potential describes, for example, the extension of tumor in the prostate capsule and seminal vesicles. Metastatic Adverse Pathology Potential describes, for example, invasion into peripheral systems such as blood, lymph and/or bone. The LAPP & MAPP calculation was made with an algorithm described herein. LAPP and MAPP values in the first table of Fig. 8 represent predictive thresholds of disease status. Gleason 6 vs. Gleason 7 denotes predicting Gleason 7 patients from a set of Gleason 6 & Gleason 7 patients; and Gleason 3+4 vs. 4+3 denotes predicting Gleason 4+3 patients from the set of all Gleason 7 patients.

Illustration 2

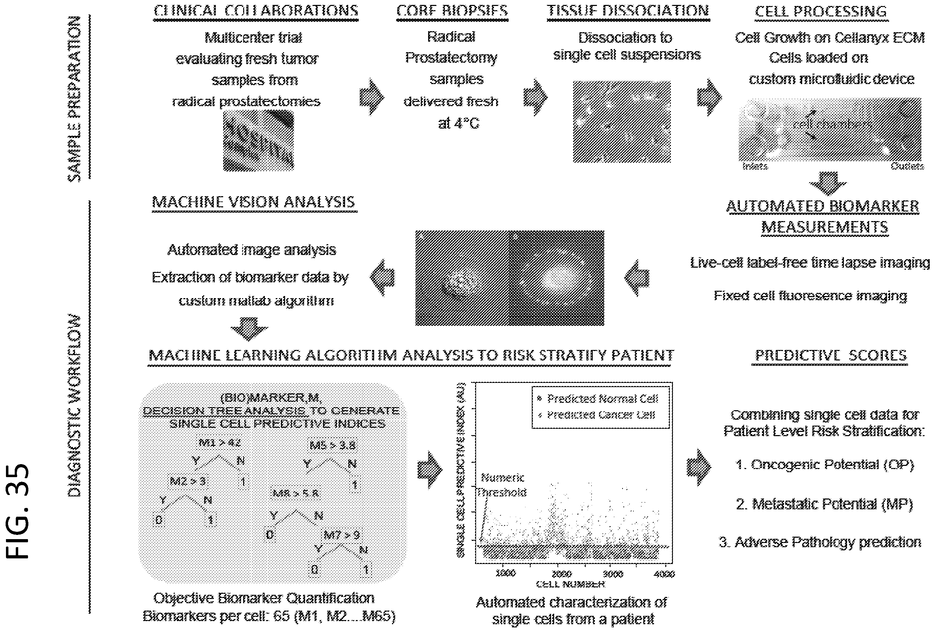

An exemplary study design is depicted in FIG. 35. Excised radical prostatectomy specimens were collected from males 55-69 years old in accordance with optimized prostate cancer detection protocols set forth by AUA 2013.

Once received, the tissue/biopsy samples are dissociated into a single cell suspension using mechanical agitation and treatment with a protease solution in prostate cell growth medium (Lonza®). Subsequently cells are collected by centrifugation and seeded onto culture plates with ECM (containing equal parts collagen and fibronectin, 10$\mu$g/ml each). The ECM is developed from purified sources and is therefore free of contaminants. Primary tissue-derived cells are maintained *in* vitro at 37 °C / 5% $CO_2$ for 48 hours prior to conducting the diagnostic assay. Single cell monolayers are disrupted by treatment with trypsin. Cells are washed with buffered prostate cell growth media containing HEPES, recovered by trypsinization and centrifugation and counted using a hemocytometer. Cells (up to 15,000) are transferred to a functionalized and ECM coated microfluidic device and maintained at 37 °C. Microfluidic devices described herein provide for monitoring of single cells in precise controlled-environments. Over the next 3 hours the cells are imaged via live-cell Differential Interference Contrast (DIC) microscopy to measure biophysical biomarkers in a label-free manner. The imaging routine captures multiple images of each cell over time to obtain information about each cell at a single time point and across multiple time points over the course of three hours. After observation the cells are fixed, stained for protein markers, and imaged using confocal fluorescence microscopy.

Measurements are automated using a motorized stage both for DIC and fluorescent microscopy, and a cooled CCD camera. Custom-developed machine vision MATLAB programs based on methods described previously are run on the cell images to measure 44 proprietary biomarkers and generate 11 additional aggregate biomarkers. These biomarkers are related to cell kinematics, morphology, and metabolic states. The computer vision algorithms operate by first locating and tracking each individual cell in each of the images. About 10,000 cells were tracked over the course of several hours in 4000 total images. The cells are identified and the proprietary metrics are calculated via methods described herein. The result of this process is a measurement of 65 biomarkers for each cell in the sample. The generated data is analyzed by a machine learning algorithm according to methods described herein. Using this algorithm, biomarker datasets from individual patient-derived cells are subjected to a decision-tree analysis protocol that characterizes each cell as normal or cancerous and its aggressiveness is graded (FIG. 35). The data from individual cells were then pooled to generate predictor scores, LAPP and MAPP for an individual sample and patient.

Results

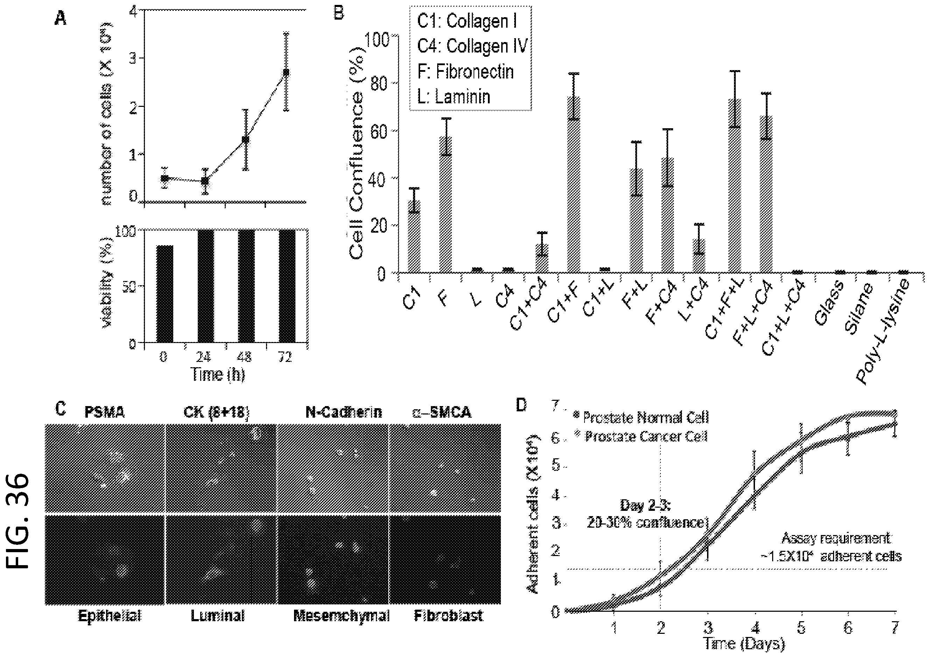

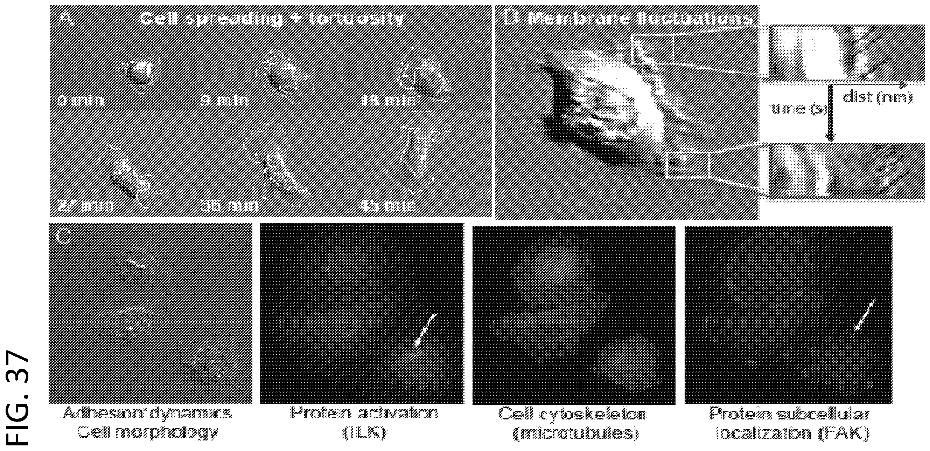

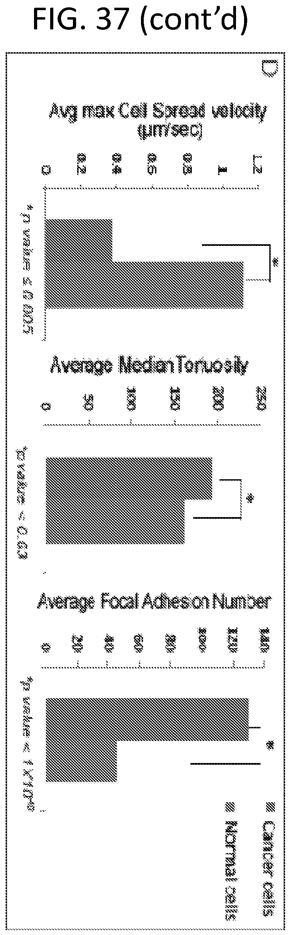

For all samples received under the present protocol, a greater than 95% viability was achieved (FIG. 36A). The ECM formulation allows cell adhesion, survival, and cell-type separation for primary prostate cells (FIG. 36B). Moreover, the various cell types that adhere onto our ECM using cell-type specific antibodies have been characterized, including basal and luminal epithelial cells, mesenchymal cells and fibroblasts, which incorporates all cell types normally found in the prostate tissue (FIG. 36C). Using the presently described culture conditions 20-30% confluence was achieved within 48 hours of culture as seen by the growth profiles of normal and prostate cancer cells (FIG. 36D). FIG. 37(A-C) shows representative images of single cells tracked over time for a representative selection of the herein described biomarkers, including rate of cell adhesion, spreading dynamics and cellular morphology, membrane fluctuations, protein expression, activation and subcellular localization. Also shown are graphs of biomarker quantification (FIG. 37D) demonstrating clear differences in cell spreading velocity, tortuosity and focal adhesion number between cancerous and normal cells.

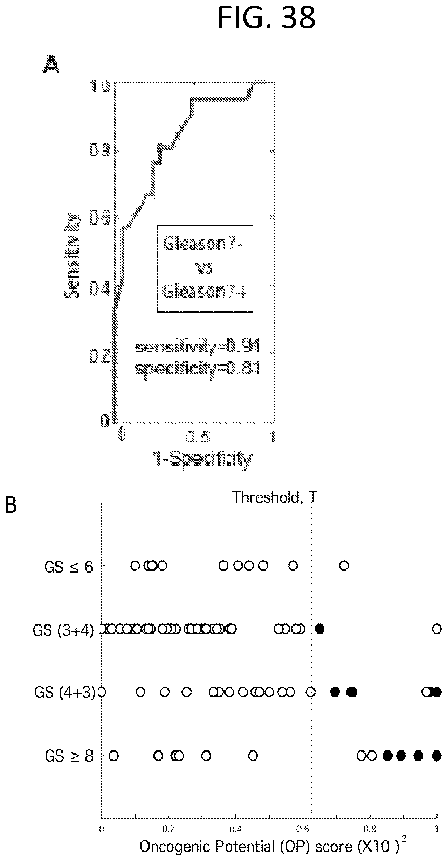

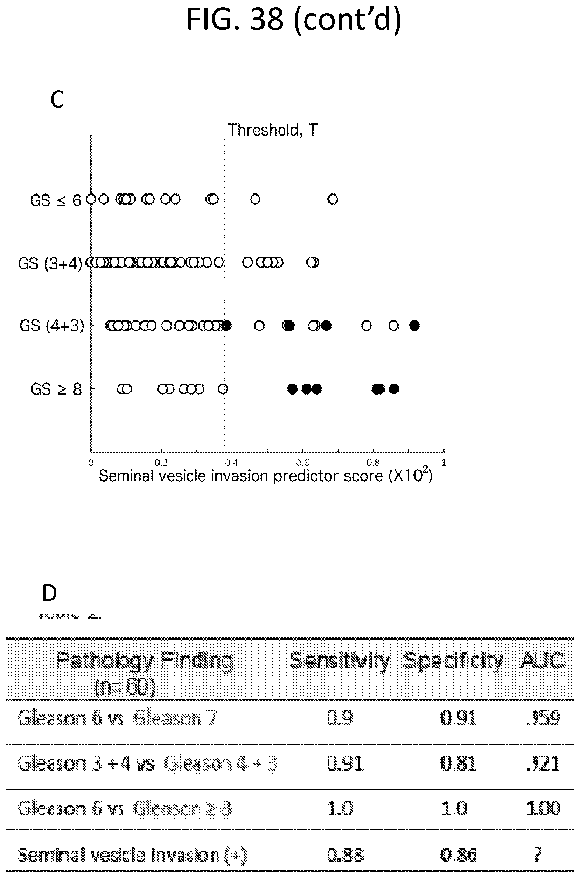

In order to make clinical predictions, the machine learning algorithm has been trained. For training, biomarker data from 70% cells of a particular sample (with known Gleason score and adverse pathology) is fed into the algorithm. Subsequently the algorithm analyzes data from the remaining cells (30%) to make predictions about the LAPP and MAPP of the population. To determine the accuracy of our assay, the predictions made by the algorithm were compared to known Gleason scores and adverse pathology data. FIG. 38D demonstrates high sensitivity and specificity for the present methods to predict Gleason score, and distinguishes between samples with different Gleason scores. Remarkably, Gleason 3+4 (marked 7-) from Gleason 4+3 (marked 7+) were discerned in samples with high confidence, as seen by the ROC curve and associated statistics (FIG. 38A). Moreover, these data demonstrate wide distribution of LAPP scores within the same Gleason group (FIG. 38B), indicating that the present diagnostic methods provide an evaluation of the tumorigenic potential of a sample that is more quantitative than, or is complementary to, the current Gleason scoring system.

These data demonstrate, for example, that: (i) it is feasible to isolate and maintain tumor-derived cells; (ii) a panel of phenotypic biomarkers may be accurately measured; (iii) it is possible to train the machine learning algorithm to achieve increased accuracy to predict LAPP and MAPP; and (iv) the methods are capable of risk stratifying samples with the same Gleason score with high accuracy. Additionally, the machine learning algorithm is demonstrated to predict seminal vesicle invasion (FIG. 38C & D).

*Drawings*

FIGS. 36A-D depicts cell growth, viability and characterization of primary biopsy derived cells. FIG. 36A depicts Growth and Viability of biopsy-derived cells 0, 24, 48 and 72 hours after seeding on ECM-coated plates. FIG. 36B provides a Graph demonstrating present ECM formula providing increased adhesion and survival of cells compared to traditionally non permissive glass surfaces or various other ECM formulations (95% confidence interval). FIG. 36C provides DIC (top) and fluorescence images (bottom) of cells stained with different cell-type specific markers, mentioned on the bottom (PSMA- prostate specific membrane antigen; CK (8+18)- cytokeratin(8+18); SMCA-smooth muscle cell actin). FIG. 36D provides cell growth and confluence profiles of normal and cancer cells. Cells were seeded on Day 1 (~5000 cells) and reached 20-30% confluence by day 2, when the diagnostic assay can be performed.

FIG. 37A-D depicts biomarkers quantified to identify and risk stratify tumor cells. FIG. 37A depicts a montage depicting cell spreading over time on an ECM coated microfluidic device. Algorithm is used to track the edge of a cell as it spreads, and determine changes in morphology and tortuosity. FIG. 37B depicts membrane fluctuations monitored by imaging the edge of the cell every 3 seconds. A machine vision algorithm defined the membrane edge and generated kymographs by plotting distance moved over time (offset images on right). The slope of the membrane folds were measured as retro grade flow velocity (RFV). As depicted in FIG. 37C, once cell morphology, adhesion dynamics and other biomarkers have been recorded in live cells, cells were fixed (in the microfluidic device) and stained with specific antibodies monitor protein activation (ILK staining), cell cytoskeletal network (microtubule staining) and protein subcellular localization (Focal Adhesion kinase staining). FIG. 37 (D) depicts representative bar graphs showing statistically significant differences in biomarker measurements between normal and cancer cells (n=136 and 112, respectively). Parameters plotted in this Figure are cell spreading velocity (left), median tortuosity (middle) and focal adhesion number (right). All biomarker data can be combined to generate the LAPP and MAPP scores.

FIGS. 38A-D depicts risk assessment plots demonstrating an ability to distinctly grade patient samples. FIG. 38A provides an ROC curve showing the hight sensitivity and specificity of our assay in ditinguishing Gleason 7- from Gleason 7 +. FIG. 38B provides a risk stratification scatter plot showing the predicted oncogenic potential of individual patients with clinically assigned Gleason scores (each dot represents an individual). Within each Gleason group there is a wide distribution of oncogenicity scores. The dotted red line is the algorithm-specified operation threshold. Individuals with LAPP values above this threshold (marked with red dots) are predicted to have locally aggressive disease and would be recommended for treatment. FIG. 38C provides a risk stratification scatter plot similar to (Fig. 38B), demonstrating the predicted risk for seminal vesicle invasion in different gleason groups. The dashed line is the algorithm-specified operation threshold. Hollow dots to the right of the threshold represent false positive predictions (samples that did not actually have this adverse pathology) while solid dots depict true positives (samples that were positive for this pathology in the path report). FIG. 38D provides sensitivity and specificity numbers demonstrating the capability of our assay to predict Gleason scores and seminal vesicle invasion (adverse pathology). Gleason 6 vs Gleason 7 denotes predicting Gleason 7 patients from a pooled set of Gleason 6 and 7).

Illustration 3

Biomarkers and are measured and the LAPP and MAPP of 150 clinically derived prostate samples using the automated live cell diagnostic platform are calculated. Tissue samples are dissociated into single cell suspensions and cycled through the diagnostic workflow detailed in Illustration 2. Thousands of cells are sampled per sample via image acquisition and machine vision software, thereby further training the machine learning software and predicting LAPP/MAPP metrics for each cell population.

Sensitivity and specificity are evaluated by positive predictive value (PPV) and negative predictive value (NPV) respectively, using standard equations. Optimal receiver operator curve area under the curve (ROC-AUC) is calculated to determine assay accuracy. Additionally, using Jaspen multiserial correlation, results are correlated with Gleason score.

An algorithm is developed to predict specific adverse pathologies with ~90% accuracy in clinical samples, defined as surgical margins, extra-prostatic extension (EPE), seminal vesicle invasion (SVI), perineural invasion (PI), vascular invasion (VI) and lymph node invasion (LNI). One of the parameters relied upon is Traction Force Index or TFI. TFI correlates with migration rate of cells and informs of associated metastatic pathologies, for example, vascular invasion and lymph node invasion. Nuclear tortuosity is also evaluated. Changes in nuclear tortuosity over time are evaluated to discern mechanical properties of various cells and improve the accuracy of predicting adverse pathologies.

Each of the herein described parameters are included individually and in combination in the described machine learning algorithm to evaluate their effect on the accuracy (sensitivity and specificity) of predictions of all six adverse pathologies related to prostate cancer. The basic workflow is as follows: Each patient is treated as a single clinical sample. For each sample, biomarker data from each single cell is fed into a trained random forest classifier. Each random forest classifier is trained based on study data to predict one of six different adverse pathologies related to prostate cancer. Therefore the likelihood of each of the adverse pathologies is predicted independently. The output from this random forest classifier is a predictor score for each cell in the sample. Finally, the proportion of cells that are above an operating threshold (determined at the time of training) and the predictor value of these cells is taken into account to generate final sample (patient level) predictor values. These final adverse pathology predictor values range from 0 to 1, where 0 represents no probability of adverse pathology, while 1 indicates 100% probability.

Illustration 4

Illustration 4 presents a variety of experimental results and data generated utilizing devices and methods described herein.

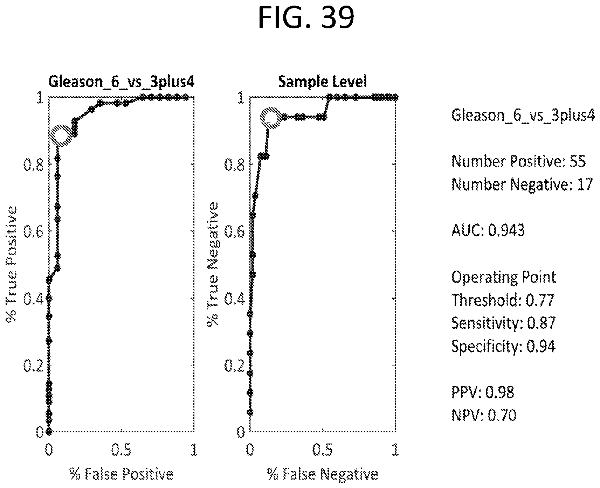

FIG. 39 provides an exemplary receiver operating characteristic (ROC) curves generated using methods described herein, and numerical representations of accuracy based on the ROC curves. ROC curves provide, for example, a way of representing the performance of a binary classifier. These ROC curves were generated as follows: The output from the binary classifier for each sample is a scalar value between 0 and 1 — 0 meaning that, for example, there is 0 likelihood that a cell should have a positive result from our evaluation, and 1 meaning that we are extremely confident that this cell had the outcome in question. The algorithm (described herein) output can, for example, be anywhere between 0 and 1. However, in the most frequent embodiments the ultimate output is purely binary (i.e.: cancer or non-cancer), so a threshold value is selected, above which indicates cancer and below which indicates no-cancer (see, e.g., FIGS. 38, 51, 52). To generate this threshold, performance is tested on a data set against multiple threshold ranges between 0 and 1 to 1). The percentage of false positive and percentage of true positive from these tests are utilized to generate exemplary ROC curves. Each dot depicted on the ROC curves is, for example, the result of the tests for one value of possible threshold value. The large dot on the ROC curves is one exemplary operating point, which represents a threshold value where we results improved. Information about the Figure is also provided on the side of the ROC curves, including information about the metric being evaluated (e.g., "Gleason 6 vs 3+4"), number of positive and negative samples, the AUC, sensitivity, specificity and selected threshold at the selected operating point, the Positive Predictive Value (PPV) and Negative Predictive Value (NPV).

As shown in FIG. 39, an algorithm was designed to determine the difference between samples that were graded as a Gleason 3+3 (6) or 3+4 (7-). This, for example, is a clinical grey area where the pathological difference may be slight but the treatment decisions may be great. Being able to differentiate these accurately is often complicated. To do so, an algorithm was designed, trained, and tested on a dataset of 72 samples that were either Gleason 6 or 7-. The AUC for the algorithm is 0.943. And, at the selected operating point, samples that were Gleason 6 vs. 7 were correctly differentiated with 87% sensitivity and 94% specificity.

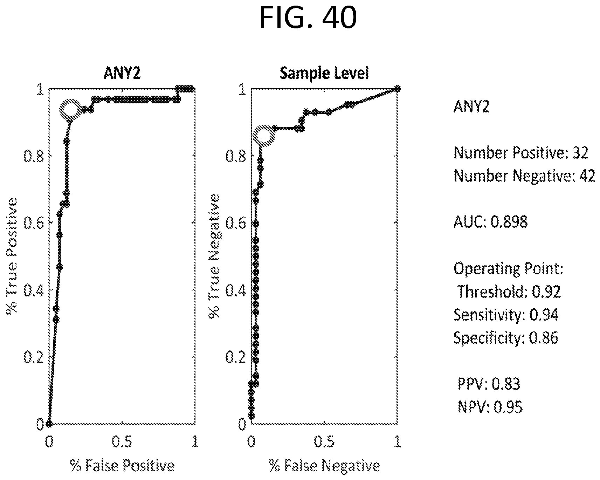

FIG. 40 provides another ROC curve, but for a different classification algorithm that can predict adverse pathologies. The algorithm used to generate FIG. 40 was designed to be a high-level algorithm that predicts if a sample will be positive for any one of the four listed adverse pathologies. A "Positive" result in this test was a sample that was listed by a surgeon as having any one of: Seminal Vesicle Invasion, Extraprostatic Extension, Positive Lymph Nodes, or Vascular Invasion. As indicated, an AUC of 0.898 is demonstrated at the selected operating point, achieving a sensitivity of 0.94 and specificity of 0.86.

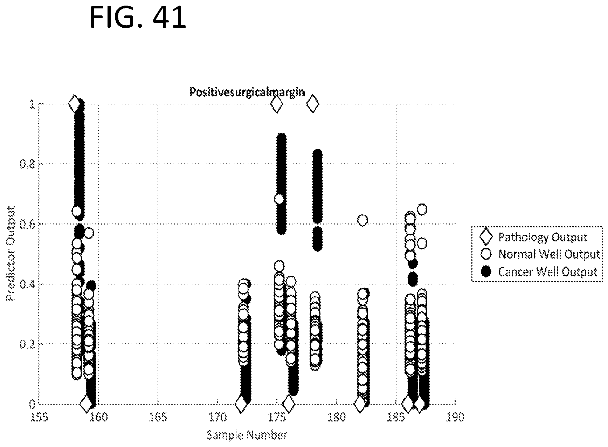

FIG. 41 depicts a representation of evaluating suspected cancerous and non-cancerous cells in the sample/analysis. The data in this plot evaluates difference, if any, between suspected cancer cells versus normal cells from the same person. Such an evaluation is useful as prostate tissue samples can be, and often are, heterogeneous tissues with respect to disease. The plot on FIG. 41 is a result of that analysis. Each data point on the Figure is output from the classification algorithm for a single cell. The x-value is the sample number - such that the cells for each sample that was analyzed in this manner are in a single column. The y-value for each point is the output from the classification algorithm (a value between 0 and 1). The cells that are from the suspected cancerous sample are solid circles (labeled "Cancer Well Output" in the legend) and the cells from the believed normal sample are hollow circles (labeled "Normal Well Output" in the legend). The diamonds provide an output value as indicated by the surgeon for that adverse pathology. If the y-value of the diamond is 1, then that sample was positive for that adverse pathology, and it is expected to see a difference between the "normal" cells and the "cancer" cells. If the y-value of the blue dot is 0, then we may expect there to be no difference between the cancer cells and the normal cells for this metric.

The graph on FIG. 41 is for the adverse pathology "Positive Surgical Margins." In this plot, there is a noticeable difference in classifier output between the suspected cancer cells and the suspected normal cells for samples where the patient had that adverse pathology. This shows that the difference in the predicted values for this metric is sensitive enough to discriminate cancer cells from normal cells - even if they are from the same patient. Also, it shows that the evaluation is specific enough such that a difference between the "cancerous" cells and the "normal" cells is not reported when the patient did not have this adverse pathology.

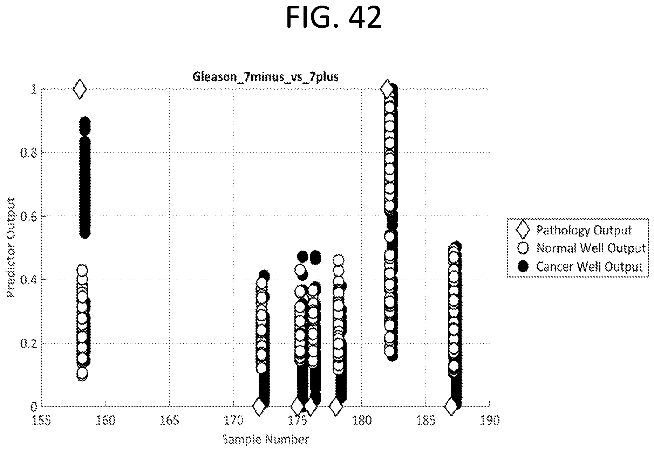

FIG. 42 is the same type of plot as on FIG. 41, but for another metric. This metric is a differentiation between Gleason 7- (3 + 4) and 7+ (4+3). This is another pathologically and clinically grey area. For sample 157, a clear distinction is generated/observed between the suspected cancer cells and the normal cells. However, for sample 182, there does not appear to be a significant difference in spread between the suspected cancer cells and the reported normal cells. Though not wishing to be bound by any particular theory, this indicates that for this sample, the cancer may have spread more than the surgeon had thought, and this "normal" sample actually had cancerous cells in it. Alternatively, these results may also indicate that the presently described metrics are so sensitive, that they can accurately discriminate Gleason 7- vs Gleason 7+, even in locations that are believed to be cancer-free.

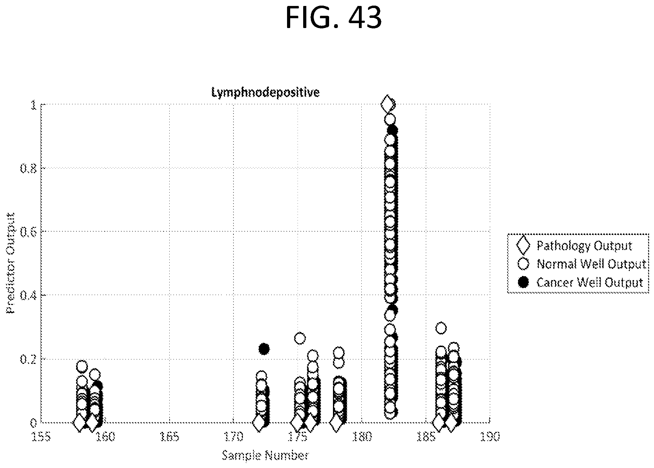

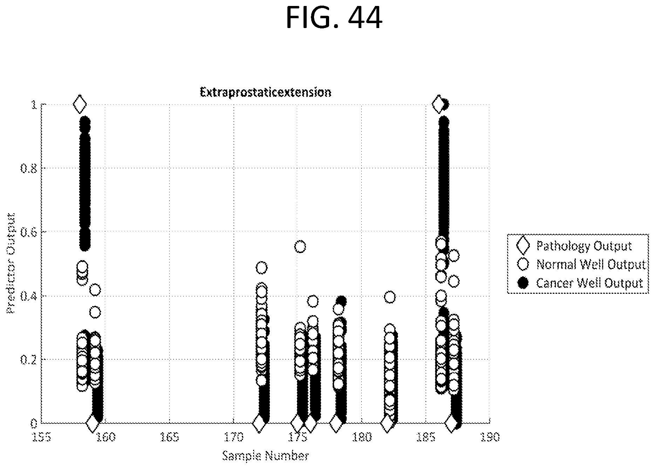

FIGS. 43 and 44 are similar to FIGS. 41 and 42. As with FIG. 42, in FIGS. 43 and 44 there is at least one sample that is positive for the evaluated metric (i.e., Lymph Node Positive and Extraprostatic Extension) where the utilized algorithm does not provide a significant difference between the suspected cancer cells and the normal cells.

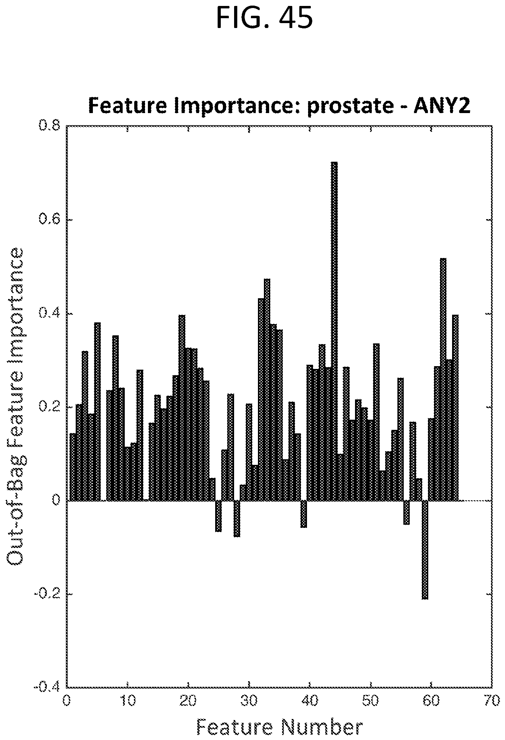

As depicted in FIG. 45, utilizing the presently described machine learning algorithms, various selected biomarkers (i.e., feature number) have been ranked in terms of importance to contemplated prognostic outputs.

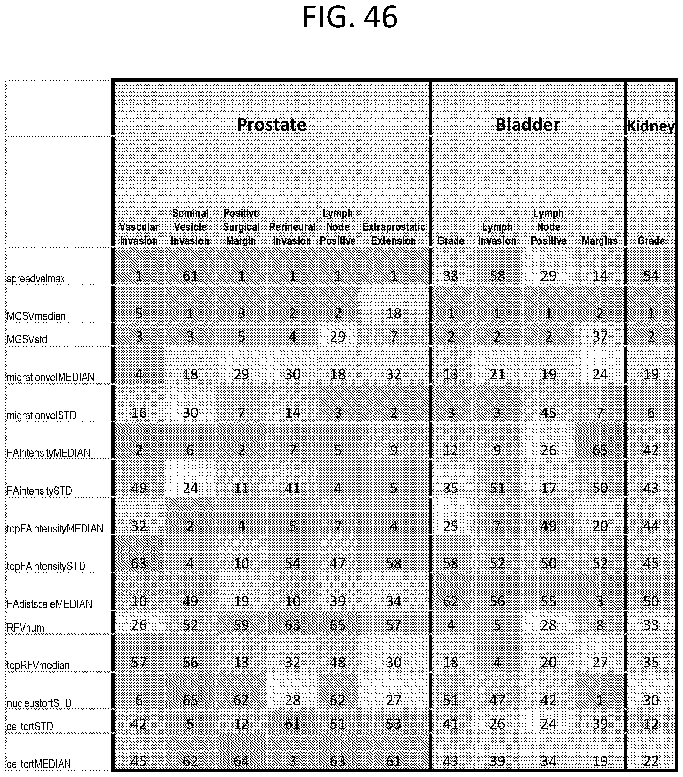

As depicted in FIG. 46, certain classification metrics are provided based on a suite of 65 biomarkers (quantified biophysical characteristics of the cells). This Figure provides an example of a calculation of the relative importance of each biomarker to an exemplary algorithm output. In particular, in FIG. 46 the relative importance of certain selected biomarkers for each adverse pathology prediction algorithm are provided. The number in each box represents the relative importance (1 is the most important, 65 is the least). This table provides an exemplary relative output ranking of different predictors. In certain embodiments, a relative ranking of biomarkers is performed when training a classifier. Optionally, in certain embodiments a similar ranking or weighted ranking is performed when evaluating a patient sample, before or after biomarker measurement. In certain embodiments, each biomarker is measured and its value is identified as a proxy indicator of a cell behavior or changing cell behavior. As explained herein, an exemplary biomarkers consists of at least two parts: (1) the physical property being measured, and 2) the way that these measurements over time are combined. In an exemplary embodiment, a number of images are captured of each individual cell during an evaluation, and for each image, a suite (e.g., up to 65 different markers) of biomarkers are calculated. Therefore, for each cell, a time-series of multiple values are provided for each biomarker. These values are often combined or collected in several ways: taking the maximum, the median, the standard deviation, or taking the mean for one or more of the biomarkers. Exemplary biomarkers are provided in the following table (which can be read together with the Table 1 above for added detail):

**Table 2**

| Name | Equation / description |
|---|---|
| 'cellareaMEAN' | Cellarea: area of the cell |
| 'cellareaMEDIAN' | |

(continued)

| Name | Equation / description |
|---|---|
| 'cellareaSTD' | |
| 'cellperimMEDIAN' | Cellperim: length of the cell perimeter |
| 'cellperimSTD' | |
| 'celltortMEDIAN' | Celltort: tortuosity of the cell outline |
| 'celltortSTD' | |
| 'cellaspectMEDIAN' | Cellaspect: aspect ratio of the cell outline |
| 'cellaspectSTD' | |
| 'nucleusareMEDIAN' | Nucleusarea: area of the nucleus |
| 'nucleusareaSTD' | |
| 'nucleusperimMEDIAN' | Nucleusperim: length of the neuclecus perimeter |
| 'nucleusperimSTD' | |
| 'nucleustortMEDIAN' | Nucleustort: tortuosity of the nucleus outline |
| 'nucleustortSTD' | |
| 'nucleusaspectMEDIAN' | Nucleusaspect: aspect ratio of the nucleus |
| 'nucleusaspectSTD' | |
| 'MGSVmedian' | MGSV: Mean grey scale value |
| 'MGSVstd' | |
| 'migrationvelMEDIAN' | Migrationvel: migration velocity |
| 'migrationvel STD' | |
| 'RFVnum' | RFV: Retrograde flow velocity<br>RFVnum: Number of Retrograde flow velocity values |
| 'RFVmedian' | |
| 'RFVstd' | |
| 'topRFVmedian' | topRFV: highest RFV value |
| 'topRFV std' | |
| 'FAnum' | FA: Focal adhesion |
| 'FAmedian' | |
| 'FAstd' | |
| 'topFAmedian' | |
| 'topFAstd' | |
| 'FAintensityMEDIAN' | FAintensity: Focal adhesion intensity |
| 'FAintensitySTD' | |
| 'topFAintensityMEDIAN' | |
| 'topFAintensitySTD' | |
| 'FAdistMEDIAN' | FAdist: distance of the FA from the center of the cell |
| 'FAdistSTD' | |
| 'topFAdistMEDIAN' | |
| 'topFAdistSTD' | |

(continued)

| Name | Equation / description |
|---|---|
| 'FAdistscaleMEDIAN' | FAdistscale: scaled distance of the FA from the center of the cell as a fraction of the distance from the cell center to the edge. |
| 'FAdistscaleSTD' | |
| 'topFAdistscaleMEDIAN' | |
| 'topFAdistscaleSTD' | |
| 'spreadvelmax' | Spreadvel: spreading velocity of the cell. |
| 'LAPP1' | LAPP1 = Tortuosity / RFV |
| 'LAPP2' | LAPP2 = Tortuosity * Perimeter / RFV |
| 'LAPP3' | LAPP3 = Area * RFV / Tortuosity |
| 'LAPP4' | LAPP4 = FASize / RFV |
| 'MAPP2' | MAPP2 = LAPP3 * Migration Velocity |
| 'P4' | P4 = Area / RFV |
| 'P5' | P5 = RFV / Area |
| 'P6' | P6 = FASize / Area |
| 'P7' | P7 = Area / FASize |
| 'P8' | P8 = Area / (RFV * Tortuosity) |
| 'P9' | P9 = RFV * Tortuosity / Area |
| 'P10' | P10 = Area * MGSV / (RFV * Tortuosity) |
| 'P11' | P11 = Area / (RFV * Tortuosity * MGSV) |
| 'P12' | P12 = FASize * Tortuosity / Area |
| 'P13' | P13 = Area * FASize / Tortuosity |
| 'P14' | P14 = Area / MigrationVelocity |
| 'P15' | P15 = FASize / Tortuosity |
| 'P16' | P16 = Migrationvelocity * Tortuosity |
| 'P17' | P17 = Migrationvelocity / Tortuosity |
| T18' | P18 = Tortuosity / FASize |
| T19' | P19 = Area * Migration Velocity |

The table above lists and/or defines a selection of 65 biomarkers contemplated herein. Certain of these exemplary biomarkers are further described elsewhere herein. Relations of these biomarkers to each other and to the status of a sample, a cell, and/or a subject in terms of diagnosis, prognosis, supplementary information, or confirmation are described throughout the present disclosure.

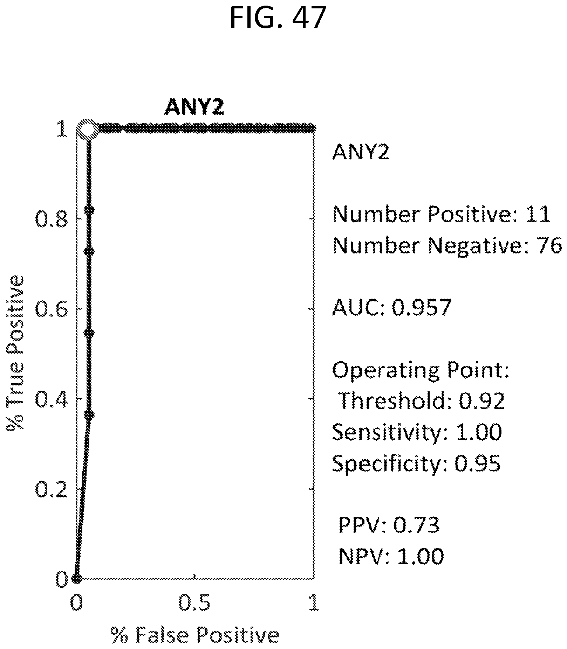

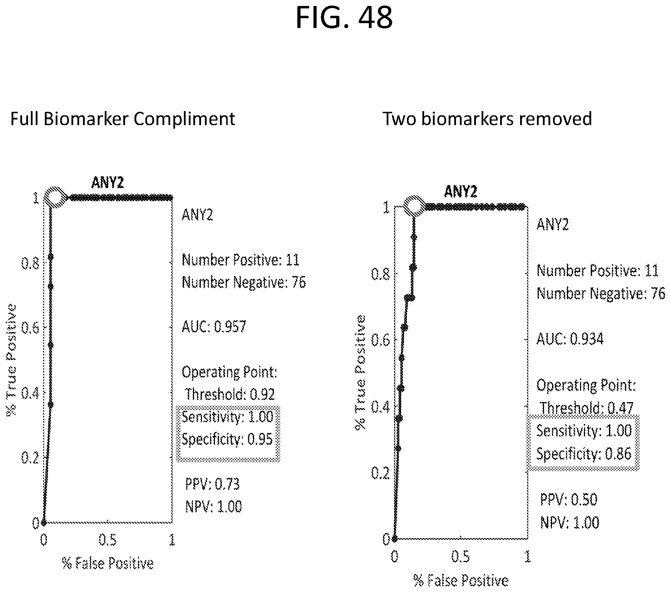

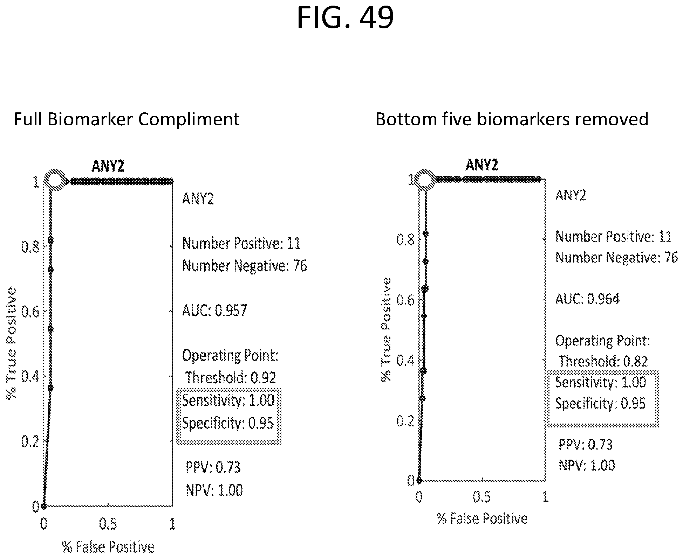

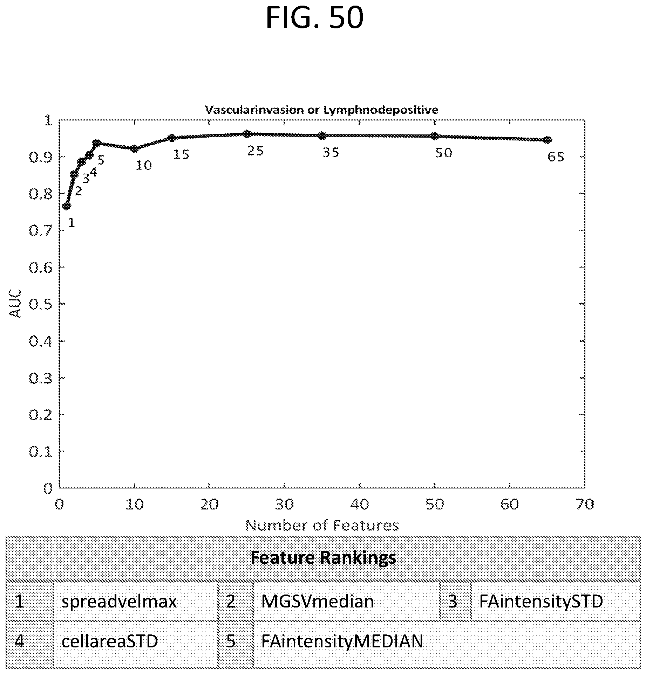

FIGs. 47-50 depict ROC Curves. FIG. 47 depicts a baseline ROC curve. Performance is: AUC 0.957, Sensitivity, 1.00, specificity: 0.95. A machine learning algorithm is provided to, for example in this Figure, predict metastasis, defined by having a pathology report positive for Vascular Invasion or Lymph Node Positive. In connection with FIG. 48, an example of an alteration in performance output is provided if two of the three "top" biomarkers are removed. Alternatively, in connection with FIG. 49, an example of an alteration in performance output is provided if five of the "lower" ranked biomarkers (see, e.g., FIG. 46) are removed. With reference to FIGS. 48 and 49, algorithm training and testing was performed with a varying number of biomarkers available, from one to all available biomarkers. In FIG. 50, a selected number of biomarkers are evaluated between one marker and 65 markers, and performance is evaluated.

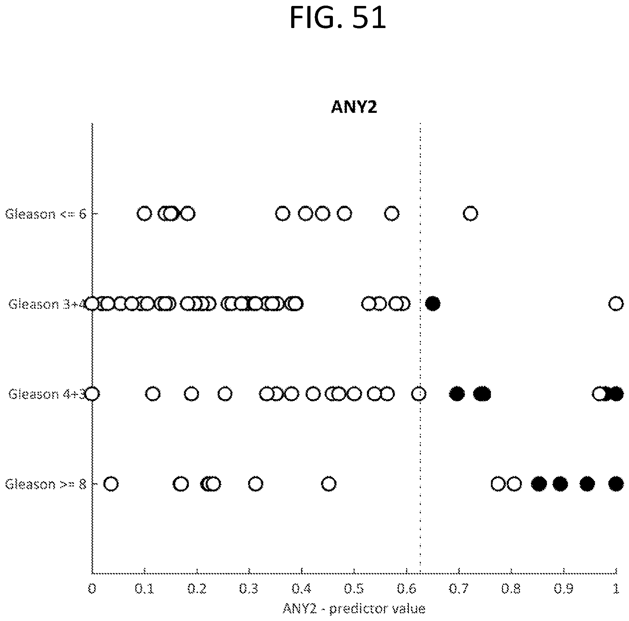

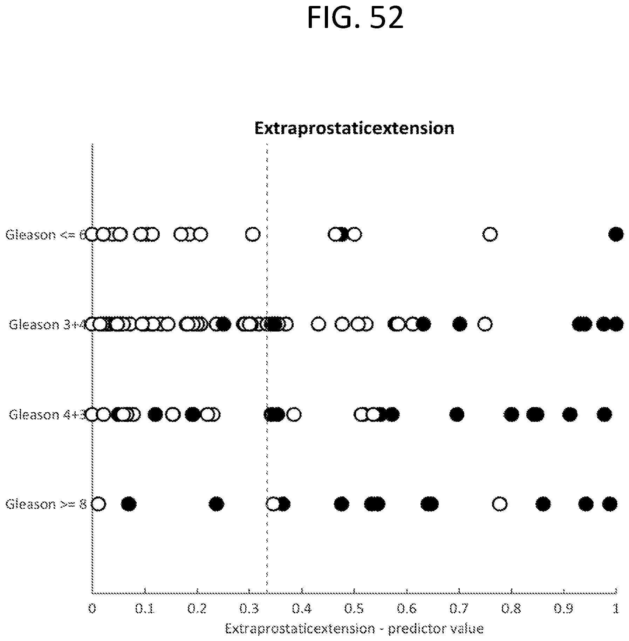

FIG. 51 provides an exemplary representation of how the Gleason score can, in certain embodiments, be included in the metrics described herein. In these plots, the x-axis is the output from a classifier. In FIG. 51 the adverse pathology testing for is the "ANY2" metric, which is any one of: Seminal Vesicle Invasion, Extraprostatic Extension, Positive Lymph Nodes, or Vascular Invasion. The Y axis is the Gleason score for each sample. The solid circles represent samples that are actually positive for this adverse pathology, and the open circles are ones that are not positive for this pathology. The dotted line is the exemplary selected operating point threshold for this metric. Any sample with a higher output number (further right) than the threshold (dotted red line) would be flagged as positive by the algorithm. Any solid circles to the right of the line are true positives, any open circles to the right of the line are false positives. This plot shows that Gleason scores can be taken into account during an exemplary process. In one implementation, the single dotted threshold line could be replaced with several different thresholds (one for each Gleason score). Doing this could achieve sensitivity and specificity. Separating samples by Gleason score, it can be seen how incorporating clinical surrogate biomarkers may, for example, provide enhanced data analysis. FIG. 52 provides a similar plot to FIG. 51, but evaluating Extraprostatic Extension.

Illustration 5

Illustration 5 describes clinical analysis of a live-cell phenotypic biomarker based diagnostic assay for the prediction of adverse pathology in prostate cancer.

Introduction and Objective: Prostate cancer accounts for over 28% of total cancer cases in the United States. Current screening and diagnostic approaches lack the sensitivity to objectively assess the tumors' aggressiveness. To address this issue, a diagnostic assay was developed to differentiate indolent from aggressive tumors, objectively risk stratify patients and predict adverse pathology. Here we describe a diagnostic platform that is based on the measurement of a panel of phenotypic and molecular biomarkers in live biopsy-derived cells. Combining microfluidics, automated imaging and image analysis described herein above, the assay provides predictive scores for local aggressiveness, invasiveness and the presence of adverse clinical pathologies.

Methods: This clinical study was done on fresh prostate cancer samples (n=325) obtained at the time of radical prostatectomy. Patient cells were grown ex vivo (up to 72 h) to enable live-cell, label-free imaging of multiple phenotypic biomarkers. Cells were then stained & imaged for molecular markers. Data were objectively quantified by machine vision to evaluate cellular behavior, and machine learning analysis to generate predictive metrics.

Results: The developed predictive dynamic biomarker metrics of adverse pathology: LAPP and MAPP, report on the local aggressiveness and invasiveness, respectively, are able to distinguish benign from malignant cells, risk stratify fresh tumor samples, and predict adverse pathology. Comparing our results with known clinical pathology data, we can distinguish Gleason 6 from Gleason 7 and Gleason (3+4) from Gleason (4+3) with greater than 90% sensitivity and specificity. LAPP and MAPP metrics can also predict the likelihood of six different adverse clinical pathologies with high accuracy as characterized by Receiver Operator Curves with Area Under the Curve (AUC) values >0.80.

Table 3 below pertains to the 'field effect', described as changes in tissues (including benign tissues) surrounding cancer lesions (i.e., adjacent tissue) and their association with development of tumors in prostate tissue. ROC curves for prediction of extra prostatic extension (EPE) using normal tissue found adjacent to a cancer lesion were generated (as represented by the data in the Table), analyzed by a classifier algorithm specifically trained to detect field effect using benign tissue. For EPE, an AUC of 0.96 was obtained at a selected operating point, achieving a sensitivity of 0.93 and specificity of 0.94. For PSM prediction, a sensitivity of 0.91, specificity of 0.95, and an AUC of 0.959 was achieved. For SVI prediction, a sensitivity of 1.0, specificity of 0.85, and AUC of 0.923 was achieved. For PNI prediction, with a sensitivity, specificity, and AUC of 1.0 was achieved. For VI prediction, a sensitivity, specificity, and AUC of 1.0 was achieved. For LNI prediction, a sensitivity, specificity, and AUC of 1.0 was achieved. As also represented in the Table, another ROC curve was regenerated for prediction of overall local growth potential in patients (LAPP) using normal adjacent tissue and application of a field effect algorithm. An AUC of 0.932 was obtained at a selected operating point, achieving a sensitivity of 0.89 and specificity of 0.92. As also represented in the Table, another ROC curve was generated for prediction of overall Invasion potential in patient samples (MAPP) using normal adjacent tissue and a field effect algorithm. An AUC of 1.0 was obtained at a selected operating point, achieving a sensitivity, specificity, and AUC of 1.0.

**Table 3**

| Pathology Finding | Total #(n) | Number Positive | Number Negative | Operating Point Threshold | Sensitivity | Specificity | Area Under Curve (AUC) |
|---|---|---|---|---|---|---|---|
| Extra Prostatic Extension (EPE) | 31 | 14 | 17 | 0.30 | 0.93 | 0.94 | 0.96 |
| Positive Surgical Margin (PSM) | 31 | 11 | 20 | 0.36 | 0.91 | 0.95 | 0.959 |

(continued)

| Pathology Finding | Total #(n) | Number Positive | Number Negative | Operating Point Threshold | Sensitivity | Specificity | Area Under Curve (AUC) |
|---|---|---|---|---|---|---|---|
| Seminal Versicle Invasion (SVI) | 30 | 4 | 26 | 0.96 | 1.00 | 0.85 | 0.923 |
| Perineural Invasion (PNI) | 29 | 28 | 1 | 0.98 | 1.00 | 1.00 | 1.00 |
| Vascular Invasion (VI) | 31 | 3 | 28 | 0.92 | 1.00 | 1.00 | 1.00 |
| Lymph Node Positive (LNP) | 27 | 1 | 26 | 0.95 | 1.00 | 1.00 | 1.00 |
| Any Local Adverse Pathology Potential (LAPP) | 31 | 18 | 13 | 0.74 | 0.89 | 0.92 | 0.932 |
| Any Metastatic Adverse Pathology Potential (MAPP) | 31 | 28 | 3 | 0.62 | 1.00 | 1.00 | 1.00 |

Conclusions: This live-cell phenotypic assay can quantitatively risk stratify patients with similar Gleason scores. Moreover, this diagnostic can predict adverse clinical pathologies, namely 1) seminal vesicle invasion, 2) positive surgical margins, 3) extra prostatic extension, 4) perineural invasion, 5) vascular invasion and 6) lymph node invasion. These results indicate that this assay can accurately stratify low & intermediate risk cases and aid clinical decision-making to improve treatment outcomes.

Illustration 6

Certain and additional predictive criteria have been generated in accordance with methodologies, reagents, and devices described herein above in connection with breast cancer, kidney cancer, and bladder cancer samples and patients.

Table 4 provides a tabular representation of exemplary ROC curves generated to assess the sensitivity and specificity of the diagnostic assay in distinguishing malignant vs. benign breast tissue. Table 4 also provides exemplary tabular representations of ROC curves generated by a classification algorithm that can predict adverse pathologies in breast tissue. The algorithm used to generate these figures was designed to predict if a sample will be positive for any one of the listed adverse pathologies. At a selected operating point threshold, determined using methods described herein, the algorithm demonstrated high accuracy and precision, as demonstrated by the AUC, sensitivity, and specificity data below for the prediction adverse clinical pathologies in breast tissues or samples containing breast tissue cells, namely: positive for Her 2, cancer or tumor grade, lympho-vascular invasion, lymph node invasion, ductal carcinoma *in situ* (DCIS), lobular carcinoma *in situ* (LCIS), extra-nodal extension, positive surgical margins, LAPP, and/or MAPP. As such, the presently described methods and devices can quantitatively risk stratify breast cancer patients or patients suspected of having or being at risk for breast cancer.

**Table 4**

| Pathology Finding | Total #(n) | Number Positive | Number Negative | Operating Point Threshold | Sensitivity | Specificity | Area Under Curve (AUC) |
|---|---|---|---|---|---|---|---|
| Her 2 positive | 33 | 8 | 25 | 0.88142 | 1 | 0.96 | 0.99 |
| Grade | 33 | 13 | 20 | 0.21756 | 1 | 0.9 | 0.96923 |
| Lympho-vascular invasion | 33 | 16 | 17 | 0.79139 | 1 | 0.94118 | 0.97059 |

(continued)

| Pathology Finding | Total #(n) | Number Positive | Number Negative | Operating Point Threshold | Sensitivity | Specificity | Area Under Curve (AUC) |
|---|---|---|---|---|---|---|---|
| Lymph node invasion | 33 | 17 | 16 | 0.97029 | 0.94118 | 0.875 | 0.91544 |
| DCIS | 33 | 23 | 10 | 0.20511 | 0.95652 | 1 | 0.98696 |
| LCIS | 32 | 6 | 26 | 0.66667 | 1 | 0.96154 | 0.96795 |
| Extra-nodal extension | 33 | 9 | 24 | 0.87071 | 0.88889 | 0.91667 | 0.91898 |
| Positive surgical margins | 33 | 2 | 31 | 0.57383 | 1 | 1 | 1 |
| Any of the above adverse pathologies | 33 | 29 | 4 | 0.98011 | 1 | 1 | 1 |
| LAPP | 33 | 29 | 4 | 0.81818 | 1 | 1 | 1 |
| MAPP | 33 | 21 | 12 | 0.68726 | 0.95238 | 1 | 0.99206 |

Table 5 provides a tabular representation of an exemplary ROC curve generated by a classification algorithm that can predict grade of the cancer in kidney tissue. An AUC of 1.0 was obtained at a selected operating point, achieving a sensitivity and specificity of 1.0.

**Table 5**

| Pathology Finding | Total number (n) | Total Positive | Total negative | Operating Point Threshold | Sensitivity | Specificity | Area Under Curve (AUC) |
|---|---|---|---|---|---|---|---|
| Grade (kidney cancer) | 4 | 1 | 3 | 0.03 | 1.00 | 1.00 | 1.00 |

Table 6 provides a tabular representation of exemplary ROC curves generated by a classification algorithm that can predict adverse pathologies in bladder tissue. The algorithm used to generate these figures was designed to predict if a sample will be positive for any one of the listed adverse pathologies. As is shown, the ROC curve for prediction of the grade of the cancer demonstrated a high accuracy of assay prediction, with an AUC of 1.0 at a selected operating point, achieving a sensitivity and specificity of 1.0. Also, the ROC curve for prediction of lymph node positive demonstrated a high accuracy of assay prediction, with an AUC of 1.0 at a selected operating point, achieving a sensitivity and specificity of 1.0. Also, the ROC curve for prediction of squamous differentiation demonstrated a high accuracy of assay prediction, with an AUC of 1.0 at a selected operating point, achieving a sensitivity and specificity of 1.0. Also, the ROC curve for prediction of glandular differentiation is provided with an AUC of 0.833 at a selected operating point, achieving a sensitivity of 1.0 and specificity of 0.67. Moreover, the ROC curve for prediction of lymph invasion provided an AUC of 1.0 at a selected operating point, achieving a sensitivity and specificity of 1.0.

**Table 6**

| Pathology Finding | Total #(n) | Number Positive | Number Negative | Operating Point Threshold | Sensitivity | Specificity | Area Under Curve (AUC) |
|---|---|---|---|---|---|---|---|
| Grade | 4 | 3 | 1 | 0.11 | 1.00 | 1.00 | 1.00 |
| Lymph Node Positive (LNP) | 4 | 1 | 3 | 0.24 | 1.00 | 1.00 | 1.00 |

(continued)

| Pathology Finding | Total #(n) | Number Positive | Number Negative | Operating Point Threshold | Sensitivity | Specificity | Area Under Curve (AUC) |
|---|---|---|---|---|---|---|---|
| Squamous Differentiation | 4 | 1 | 3 | 0.1 | 1.00 | 1.00 | 1.00 |
| Glandular Differentiation | 4 | 1 | 3 | 0.0 | 1.00 | 0.67 | 0.833 |
| Lymph Invasion (LI) | 4 | 2 | 2 | 0.0 | 1.00 | 1.00 | 1.00 |

Table 6 lists an indication of an exemplary "feature importance" for grade predictor output in bladder tissue/cells, which refers to a rank order of the importance of various biomarkers in generating the algorithm output. The number associated with the biomarker represents an exemplary relative importance for the specific pathology.

**Table 6**

| Rank order: | Grade | Lymph Node Positive | Pathology Finding Squamous Differentiation | Glandular Differentiation | Lymph Invasion |
|---|---|---|---|---|---|
| 1 | MGSVmedian (0.91) | MGSVmedian (0.8) | CellAreaMean (0.59) | topFAdistMedian (0.28) | topFAdistscaleSTD (0.36) |
| 2 | P16 (0.67) | P17 (0.69) | OP2 (0.54) | FAdistscaleMedian (0.2) | SpreadVelMax (0.36) |
| 3 | CellAreaMedian (0.5) | CellAreaMedian (0.61) | MGSVmedian (0.51) | topFAdistSTD (0.2) | MGSVmedian (0.34) |
| 4 | P14 (0.48) | P19 (0.45) | migrationVelMedia n (0.45) | FAdistMedian (0.2) | CellAreaMedian (0.29) |
| 5 | OP2 (0.48) | P14 (0.45) | CellAreaMedian (0.43) | topFAdistscaleSTD (0.2) | topFAdistSTD (0.29) |
| 6 | CellAreaMean (0.42) | CellPerimMedian (0.43) | P19 (0.42) | OP2 (0.2) | CellAreaSTD (0.29) |
| 7 | P17 (0.41) | MGSVstd (0.37) | P17 (0.37) | P17 (0.2) | topFAdistMedian (0.28) |
| 8 | P5 (0.38) | P10 (0.35) | | MGSVmedian (0.2) | topFAdistscaleMedian (0.2) |
| 9 | CellPerimMedian (0.36) | OP2 (0.34) | OP1 (0.35) | FAdistscaleSTD (0.2) | P17 (0.2) |
| 10 | P19 (0.33) | OP1 (0.33) | P14 (0.34) | migrationVelMedian (-0.52) | FAdistSTD (0.2) |
| 65 | CellAreaSTD (-0.03) | RFVmedian (-0.18) | RFVmedian (-0.08) | P19 (-0.74) | P19 (0.0) |
| 64 | P18 (0.00) | P18 (0.0) | P18 (0.0) | P16 (-0.74) | P18 (0.0) |
| 63 | P15 (0.0) | P15 (0.0) | P15 (0.0) | P15 (-0.74) | P15 (0.0) |
| 62 | P13 (0.0) | P13 (0.0) | P13 (0.0) | P14 (-0.74) | P14 (0.0) |
| 61 | P12 (0.0) | P12 (0.0) | P12 (0.0) | P13 (-0.74) | P13 (0.0) |
| 60 | P7 (0.0) | P7 (0.0) | P11 (0.0) | P12 (-0.74) | P12 (0.0) |
| 59 | P6 (0.0) | P6 (0.0) | P7 (0.0) | P11 (-0.74) | P11(O.O) |
| 58 | OP4 (0.0) | OP4 (0.0) | P6 (0.0) | P10 (-0.74) | P10(0.0) |
| 57 | SpreadVelMax (0.0) | FAdistscaleMedian (0.0) | OP4 (0.0) | P9 (-0.74) | P9 (0.0) |
| 56 | topFAdistscaleSTD (0.0) | topFAdistscaleSTD (0.0) | SpreadVelMax (0.0) | P8 (-0.74) | P8 (0.0) |

One skilled in the art will appreciate further features and advantages of the presently disclosed methods, systems and devices based on the above-described embodiments. Accordingly, the presently disclosed methods are not to be limited by what has been particularly shown and described, except as indicated by the appended claims.

**Claims**

1. A method for evaluating the status of a cell in a sample, comprising:

   disposing the cell on an extracellular matrix (ECM) in an imaging chamber
   capturing multiple images of the cell within images of a plurality of cells as the cells interact with the ECM over a pre-defined time period in a sample obtained from a subject;
   combining multiple images or different portions of the imaging chamber into a single larger image;
   evaluating the multiple images of the cell to identify or measure a pre-selected biomarker;
   identifying the cell as normal or an outlier within the plurality of cells based on the identification or measurement of the pre-selected biomarker; wherein if the cell is identified as an outlier, subjecting the identified cell or measured biomarker in the outlier to a machine learning analysis thereby creating a cell level output indicator; and combining two or more cell level output indicators to create a prognostic indicator for the sample.

2. The method of claim 1, wherein the pre-selected biomarker comprises a plurality of biomarkers.

3. The method of claim 2, wherein two or more of the pre-selected biomarkers are used in the identification of the cell as normal or an outlier.

4. The method of any of claims 1-3, wherein two or more of the pre-selected biomarkers are subjected to the machine learning analysis.

5. The method of claim 4, wherein five or more of the pre-selected biomarkers are subjected to the machine learning analysis.

6. The method of claim 4, wherein 17 to 26 of the pre-selected biomarkers are subjected to the machine learning analysis.

7. The method of claim 4, wherein 17 or more of the pre-selected biomarkers are subjected to the machine learning analysis.

8. The method of claim 4, wherein up to 45 of the pre-selected biomarkers are subjected to the machine learning analysis.

9. The method of claim 1, wherein the sample comprises a plurality of live cells obtained from culturing live cells present in a sample obtained from the subject.

10. The method of claim 1, wherein the prognostic indicator comprises a diagnosis of the subject.

11. The method of claim 1, wherein the prognostic indicator comprises a prognosis for the subject.

12. The method of claim 1, wherein the prognostic indicator comprises a confirmation or adjustment of a diagnosis of the subject or prognosis for the subject.

13. The method of claim 1, wherein the prognostic indicator is used to modify or confirm a pathological determination for the sample.

14. The method of claim 1, wherein the prognostic indicator is used to modify, confirm, or deny an established clinical nomogram, tumor grade, cancer staging or grading system, or pathological score used for diagnosis and /or prognosis.

15. The method of claim 1, wherein the sample comprises a sample of cells from a prostate tissue, a bladder tissue, a lung tissue, a kidney tissue, a breast tissue, an ovarian tissue, a uterine tissue, a colon tissue, a thyroid tissue, a skin tissue.

**Patentansprüche**

1. Verfahren zur Bewertung des Status einer Zelle in einer Probe, umfassend:

   Anordnen der Zelle auf einer extrazellulären Matrix (ECM) in einer Abbildungskammer
   Aufnehmen mehrerer Bilder der Zelle innerhalb von Bildern einer Vielzahl von Zellen, während die Zellen mit der ECM über eine vordefinierte Zeitspanne in einer von einem Subjekt erhaltenen Probe interagieren
   Kombinieren mehrerer Bilder oder verschiedener Teile der Abbildungskammer zu einem einzigen größeren Bild
   Auswerten der mehreren Bilder der Zelle, um einen vorgewählten Biomarker zu identifizieren oder zu messen;
   Identifizieren der Zelle als normal oder als Ausreißer innerhalb der Vielzahl von Zellen auf der Grundlage der Identifizierung oder Messung des vorgewählten Biomarkers
   Identifizierung der Zelle als normal oder als Ausreißer innerhalb der Vielzahl von Zellen auf der Grundlage der Identifizierung oder Messung des vorgewählten Biomarkers; wobei, wenn die Zelle als Ausreißer identifiziert wird, die identifizierte Zelle oder der gemessene Biomarker in dem Ausreißer einer Machine-Learning-Analyse unterzogen wird, wodurch ein Ausgangsindikator auf Zellebene erzeugt wird; und
   Kombinieren von zwei oder mehr Zellniveau-Ausgangsindikatoren, um einen prognostischen Indikator für die Probe zu erzeugen.

2. Verfahren nach Anspruch 1, wobei der vorausgewählte Biomarker eine Vielzahl von Biomarkern umfasst.

3. Verfahren nach Anspruch 2, wobei zwei oder mehr der vorausgewählten Biomarker bei der Identifizierung der Zelle als normal oder als Ausreißer verwendet werden.

4. Verfahren nach einem der Ansprüche 1 bis 3, wobei zwei oder mehr der vorausgewählten Biomarker der Machine-Learning-Analyse unterzogen werden.

5. Verfahren nach Anspruch 4, bei dem fünf oder mehr der vorausgewählten Biomarker der Machine-Learning-Analyse unterzogen werden.

6. Verfahren nach Anspruch 4, wobei 17 bis 26 der vorausgewählten Biomarker der Machine-Learning-Analyse unterzogen werden.

7. Verfahren nach Anspruch 4, wobei 17 oder mehr der vorausgewählten Biomarker der Machine-Learning-Analyse unterzogen werden.

8. Verfahren nach Anspruch 4, wobei bis zu 45 der vorausgewählten Biomarker der maschinellen Learning-Analyse unterzogen werden.

9. Verfahren nach Anspruch 1, wobei die Probe eine Vielzahl von lebenden Zellen umfasst, die aus der Kultivierung von lebenden Zellen gewonnen werden, die in einer von der Person erhaltenen Probe vorhanden sind.

10. Verfahren nach Anspruch 1, wobei der prognostische Indikator eine Diagnose des Patienten umfasst.

11. Verfahren nach Anspruch 1, wobei der prognostische Indikator eine Prognose für das Subjekt umfasst.

12. Verfahren nach Anspruch 1, wobei der prognostische Indikator eine Bestätigung oder Anpassung einer Diagnose des Patienten oder einer Prognose für den Patienten umfasst.

13. Verfahren nach Anspruch 1, wobei der prognostische Indikator verwendet wird, um eine pathologische Bestimmung für die Probe zu modifizieren oder zu bestätigen.

14. Verfahren nach Anspruch 1, wobei der prognostische Indikator verwendet wird, um ein etabliertes klinisches Nomogramm, einen Tumorgrad, ein Krebs-Staging- oder Grading-System oder einen pathologischen Score, der für die Diagnose und/oder Prognose verwendet wird, zu modifizieren, zu bestätigen oder zu verneinen.

15. Verfahren nach Anspruch 1, wobei die Probe eine Probe von Zellen eines Prostatagewebes, eines Blasengewebes, eines Lungengewebes, eines Nierengewebes, eines Brustgewebes, eines Eierstockgewebes, eines Gebärmuttergewebes, eines Dickdarmgewebes, eines Schilddrüsengewebes, eines Hautgewebes umfasst.

**Revendications**

1. Procédé d'évaluation de l'état d'une cellule dans un échantillon, comprenant les étapes consistant à :

disposer la cellule sur une matrice extracellulaire (ECM) dans une chambre d'imagerie

la capture d'images multiples de la cellule dans des images d'une pluralité de cellules lorsque les cellules interagissent avec la MEC sur une période de temps prédéfinie dans un échantillon obtenu à partir d'un sujet ;

la combinaison d'images multiples ou de différentes parties de la chambre d'imagerie en une seule image de plus grande taille.

l'évaluation des images multiples de la cellule pour identifier ou mesurer un biomarqueur présélectionné ;

l'identification de la cellule comme étant normale ou aberrante au sein de la pluralité de cellules sur la base de l'identification

ou la mesure du biomarqueur présélectionné ; dans lequel, si la cellule est identifiée comme aberrante, soumettre la cellule identifiée ou le biomarqueur mesuré dans l'aberrante à une analyse par apprentissage automatique, créant ainsi un indicateur de sortie au niveau de la cellule ; et

combiner deux ou plusieurs indicateurs de sortie au niveau cellulaire pour créer un indicateur de pronostic pour l'échantillon.

2. Procédé selon revendication 1, dans lequel le biomarqueur présélectionné comprend une pluralité de biomarqueurs.

3. Procédé selon revendication 2, dans lequel deux ou plusieurs des biomarqueurs présélectionnés sont utilisés dans l'identification de la cellule comme normale ou aberrante.

4. Procédé selon l'une quelconque des revendications 1 à 3, dans lequel deux ou plusieurs des biomarqueurs présélectionnés sont soumis à l'analyse par apprentissage machine.

5. Méthode selon la revendication 4, dans laquelle cinq ou plus des biomarqueurs présélectionnés sont soumis à l'analyse par apprentissage machine.

6. Procédé selon la revendication 4, dans lequel 17 à 26 des biomarqueurs présélectionnés sont soumis à l'analyse par apprentissage machine.

7. Procédé selon la revendication 4, dans lequel 17 ou plus des biomarqueurs présélectionnés sont soumis à l'analyse par apprentissage machine.

8. Procédé selon la revendication 4, dans lequel jusqu'à 45 des biomarqueurs présélectionnés sont soumis à l'analyse par apprentissage machine.

9. Procédé selon la revendication 1, dans lequel l'échantillon comprend une pluralité de cellules vivantes obtenues à partir de la culture de cellules vivantes présentes dans un échantillon obtenu à partir du sujet.

10. Procédé selon la revendication 1, dans lequel l'indicateur pronostique comprend un diagnostic du sujet.

11. La méthode selon la revendication 1, dans laquelle l'indicateur de pronostic comprend un pronostic pour le sujet.

12. Méthode selon la revendication 1, dans laquelle l'indicateur de pronostic comprend une confirmation ou un ajustement d'un diagnostic du sujet ou d'un pronostic pour le sujet.

13. La méthode selon la revendication 1, dans laquelle l'indicateur de pronostic est utilisé pour modifier ou confirmer une détermination pathologique pour l'échantillon.

14. Méthode selon la revendication 1, dans laquelle l'indicateur de pronostic est utilisé pour modifier, confirmer ou infirmer un nomogramme clinique établi, un grade tumoral, un système de classification ou de mise en scène du cancer, ou un score pathologique utilisé pour le diagnostic et/ou le pronostic.

15. Procédé selon la revendication 1, dans lequel l'échantillon comprend un échantillon de cellules provenant d'un tissu de prostate, d'un tissu de vessie, d'un tissu de poumon, d'un tissu de rein, d'un tissu de sein, d'un tissu d'ovaire, d'un tissu d'utérus, d'un tissu de côlon, d'un tissu de thyroïde, d'un tissu de peau.

# FIG. 1

# SAMPLE PROCESSING & *IN VITRO* MICRO-ENVIRONMENT

AUTOMATED BIOMARKER IMAGING

A device substrate

B adhesion dynamics

C morphology

D spreading dynamics

E cytoskeletal dynamics

F protein localization

G protein modification

H protein expression

I metabolic state

FIG. 3

EP 3 262 417 B1

40

## FIG. 4

# FIG. 5

A

B

# FIG. 6

## 1. BIOMARKER QUANTIFICATION AND ANALYTICAL ALGORITHM OUTPUT

**A** (BIO)MARKERS, M

**B** (BIO)MARKER,M DECISION TREE ANALYSIS TO GENERATE SINGLE CELL PREDICTIVE INDEX

SCPI(M1, M2, M3, ...)

## 2. CHARACTERIZATION OF SINGLE NORMAL & POTENTIAL CANCER CELLS

**C**

PPI(T, SCPI(M1, M2, ...)

# FIG. 6 (cont'd)

# FIG. 7A

# FIG. 7A (cont'd)

FIG. 7B

**SENSITIVITY**

**1 - SPECIFICITY**

GLEASON
6 v. 7

Number Positive: 48
Number Negative: 11

AUC: 0.959

GLEASON
3+4 vs. 4+3

Number Positive: 22
Number Negative: 26

AUC: 0.921

EP 3 262 417 B1

# FIG. 8

## DIAGNOSTIC TEST PREDICTION STATISTICS

| Pathology Finding | OP | MP | Total Positive | True Positive | False Negative | Total Negative | True Negative | False Positive |
|---|---|---|---|---|---|---|---|---|
| Gleason 6 vs. Gleason 7 (n=56) | >79 | | 48 | 43 | 5 | 11 | 10 | 1 |
| Gleason 3 + 4 vs. Gleason 4 + 3 (n = 48) | | >46 | 22 | 20 | 2 | 26 | 21 | 5 |
| Gleason 6 vs. Gleason ≥8 (n = 19) | >40 | | 8 | 8 | 0 | 11 | 11 | 0 |
| Seminal vesicle invasion (+) (n = 19) | >69 | | 8 | 7 | 1 | 51 | 44 | 7 |
| Surgical margins (+) (n = 67) | >32 | | 21 | 20 | 1 | 46 | 37 | 9 |
| Extra-prostatic extension (+) (n = 50) | >94 | | 24 | 21 | 3 | 36 | 33 | 3 |
| Perineural invasion (+) (n = 55) | | >66 | 38 | 34 | 4 | 17 | 15 | 2 |
| Vascular invasion (+) (n = 56) | | >86 | 5 | 4 | 1 | 51 | 45 | 6 |
| Lymph node (+) (n = 44) | | >99 | 5 | 4 | 1 | 39 | 35 | 4 |

## DIAGNOSTIC TEST PERFORMANCE STATISTICS

B

| Pathology Finding (n= 70) | Sensitivity | Specificity |
|---|---|---|
| Gleason 6 vs. Gleason 7 | 0.90 | 0.91 |
| Gleason 3 + 4 vs. Gleason 4 + 3 | 0.91 | 0.81 |
| Gleason 6 vs. Gleason ≥8 | 1.00 | 1.00 |
| Seminal vesicle invasion (+) | 0.66 | 0.66 |
| Surgical margins (+) | 0.95 | 0.80 |
| Extra-prostatic extension (+) | 0.66 | 0.92 |
| Perineural invasion (+) | 0.89 | 0.88 |
| Vascular invasion (+) | 0.80 | 0.68 |
| Lymph node (+) | 0.80 | 0.90 |

# DIAGNOSTIC TEST PERFORMANCE STATISTICS

| Predicted Pathology (red) (n= 104) | Sensitivity | Specificity | AUC |
|---|---|---|---|
| Gleason 6 vs. Gleason 7 (n=89) | 0.88 | 0.81 | 0.90 |
| Gleason 3 + 4 vs. Gleason 4 + 3 (n = 73) | 0.85 | 0.80 | 0.88 |
| Gleason 6 vs. Gleason ≥8 (n = 29) | 0.92 | 1.00 | 0.97 |
| Seminal vesicle invasion (+) (n = 95) | 0.80 | 0.85 | 0.91 |
| Extra-prostatic extension (+) (n = 95) | 0.82 | 0.82 | 0.85 |
| Vascular invasion (+) (n = 92) | 0.88 | 0.87 | 0.91 |
| Lymph node (+) (n = 78) | 1.00 | 0.86 | 0.95 |

FIG. 8C

EP 3 262 417 B1

# FIG. 9

Cell Processing → Cell Culturing → Cell Imaging → Transformation of Cell Images into biophysical metrics → Transformation of biophysical metrics into prognostic indications

# FIG. 10

Start

Input images of cells across growth chamber over time

Set up cell coordinate system for tracking

Montage of multiple imaging spots → Mask out background to isolate cells → Split up groups of cells → Record Cell migration positions

Biomarker Measurement

Live Cell Biomarker Measurement

Fixed Cell Fluorescent Marker Measurements

Output chart with biomarker matched to each cell

End

# FIG. 11

Input: Multiple images

Montage images by stitching based on coordinates

Calculate/Apply Illumination Correction to even out brightness

Example Output: Illumination-Corrected Montage

# FIG. 12

A

B

Before

After

# FIG. 13

Input: Illumination-Corrected Montage

→

Clean Up Image by stretching pixel values

→

Find and apply initial threshold for edge detection

Adjust/Apply threshold

No

→

Detect edges of "objects" and count

→

Is there an acceptable number of "objects" detected and percent of image covered by the "objects"?

Yes →

Dilate "object", remove small objects, close structures

→

Keep objects that are above a certain morphology to consider as a cell (or group of cells)

→

Ex Output: Images with only "objects" that are at least the morphology of a cell present and the background blacked out

FIG. 14

FIG. 15

A

B

FIG. 16

FIG. 17

# FIG. 18

# FIG. 19

Input: Images with only "objects" that are at least the morphology of a cell present and the background blacked out

Identify "Object" edges by watershedding, record results

Employ stricter thresholding of "objects" to narrow search for nucleus edge

Adjust/Apply stricter threshold

No

Watershedding to find new edge

Is the new "object" defined by the edge indicative of a nucleus?

Yes

Combine results of "object" edge and nucleus

Reject locations with no nucleus

Identify locations with one "object" area but multiple nucleus

(Cont.)

# FIG. 20

Adjust/Apply local threshold

No

Local watershedding to find new edge

Are there the same number of "object" areas as identified nucleus?

Yes

Apply segmentation

Output: Images with correctly identified and segmented cells, background that is blacked out

FIG. 21

FIG. 22

# FIG. 23

Object with multiple nuclei:
further attempts to split

# FIG. 24

# FIG. 25

Input: Images with properly segmented cells

Find location for cells in image at time t

Find location for cells in image at time t-1

Calculate distance of all cells at time t-1 to a cell at time t

Find cell of minimum distance

Is the minimum distance within an acceptable threshold?

Cont.

# FIG. 26

Calculate and find distance of all cells at t-2 to a cell at time t

**No**

Is the minimum distance within an acceptable threshold?

**No** → New cell has emerged, assigned new cell ID

**Yes** → Record location of time t, and estimate location of time t-1

**Yes** → Record Cell location at time t

Example Output:
- Sequence of Images with cell ID attached
- Array of X-Y location of each cell at each timepoint

# FIG. 27

Input: Images with properly segmented cells

Find Center of Cell using previously recorded migration track

Draw 8 lines extending radially outwards to generate kymograph

Find edge of cell on kymograph, analyze only in section within cell

Find local peaks in intensity in kymograph

Trace line linking peaks in intensity (if applicable)

Measure Slope of line, and back calculate for velocity

Example Output: Kymographs with measured retrograde flow velocity values

# FIG. 28

A

B

center of cell ⟶ outside of cell

Time 0

Later time

# FIG. 29

Input: Fluorescent Images of various targets

Use Microtubule staining to identify cell locations

Utilize microtubule staining to subtract common background noise from focal adhesion images

Measure various properties of stained focal adhesion

Output: Highlighted Focal adhesion as identified by program, array of focal adhesion properties

# FIG. 30

A

Current cell in focus

B

Filtered objects

# FIG. 31

Cell Level Biophysic al Metrics

Identify outlier cells

- Example Output: cell-level metrics for outlier cells

Put cell metrics through machine learning algorithm

- Example Output: prognostic indicator for each individual cell

Combine cell-level results to get patient-level output

- Example Output: prognostic indicator for sample

# FIG. 32

Represent each cell as a unique data point described by the totality of its individual metrics

• Example Input: Approximately 2000 cells per sample, with 45 biomarkers per cell

Examples of unsupervised machine learning methods
k-means
hierarchical clustering
fuzzy clustering
expectation-maximizing clustering

Identify outlier cells using unsupervised machine learning on those metrics.

• Example Output: Approximately 100 cells per patient that are outliers

# FIG. 33

Example Input (from prior step): 100 cells per patient, each represented by 45 biomarkers

Run each individual outlier cell through a trained machine-learning classifier.

Example Output: For each cell – the likelihood that this cell came from someone with the specified pathological endpoint.

- Examples of machine learning classifiers:
  - Decision Tree
  - Bootstrap aggregated decision tree
  - Neural network
  - Linear discriminator
  - Non-linear discriminator

# FIG. 34

Biophysical
metrics

Find
outlier
cells

Machine learning
classifier

Combine cell
level
predictors
through
trained
algorithms

Example
Output: For
all the
abnormal
cells – the
combined
likelihood
that this
person has
the specified
pathological
endpoint.

Processing of each individual cell

FIG. 35

EP 3 262 417 B1

**DIAGNOSTIC WORKFLOW**

**SAMPLE PREPARATION**

Objective Biomarker Quantification
Biomarkers per cell: 65 (M1, M2...M65)

DECISION TREE ANALYSIS TO GENERATE
SINGLE CELL PREDICTIVE INDICES
(BIOMARKER M)

$M1 < 42$   $M2 < 3$   $M7 > 9$
$M5 > 3.8$   $M8 > 5.8$
Y   N   0   1

Automated characterization of
single cells from a patient

CELL NUMBER
1000   2000   3000   4000
SINGLE CELL PREDICTIVE INDEX (AU)
Numeric Threshold
• Predicted Normal Cell
• Predicted Cancer Cell

PREDICTIVE SCORES

Combining single cell data for
Patient Level Risk Stratification:
1. Oncogenic Potential (OP)
2. Metastatic Potential (MP)
3. Adverse Pathology prediction

MACHINE LEARNING ALGORITHM ANALYSIS TO RISK STRATIFY PATIENT

MACHINE VISION ANALYSIS

Automated image analysis
Extraction of biomarker data by
custom matlab algorithm

AUTOMATED BIOMARKER
MEASUREMENTS

Live-cell label-free time lapse imaging
Fixed cell fluorescence imaging

CELL PROCESSING

Cell Growth on Cellanyx ECM
Cells loaded on
custom microfluidic device

TISSUE DISSOCIATION

Dissociation to
single cell suspensions

CORE BIOPSIES

Radical
Prostatectomy
samples
delivered fresh
at 4°C

CLINICAL COLLABORATIONS

Multicenter trial
evaluating fresh tumor
samples from
radical prostatectomies

# FIG. 36

## FIG. 37

# FIG. 37 (cont'd)

# FIG. 38

# FIG. 38 (cont'd)

C

Threshold, T

GS ≤ 6

GS (3+4)

GS (4+3)

GS ≥ 8

Seminal vesicle invasion predictor score (X10$^2$)

D

| Pathology Finding (n= 60) | Sensitivity | Specificity | AUC |
|---|---|---|---|
| Gleason 6 vs Gleason 7 | 0.9 | 0.91 | .959 |
| Gleason 3 +4 vs Gleason 4 + 3 | 0.91 | 0.81 | .921 |
| Gleason 6 vs Gleason ≥ 8 | 1.0 | 1.0 | 1.00 |
| Seminal vesicle invasion (+) | 0.88 | 0.86 | ? |

# FIG. 39

**Gleason_6_vs_3plus4**

**Sample Level**

Gleason_6_vs_3plus4

Number Positive: 55
Number Negative: 17

AUC: 0.943

Operating Point
Threshold: 0.77
Sensitivity: 0.87
Specificity: 0.94

PPV: 0.98
NPV: 0.70

# FIG. 40

ANY2

Number Positive: 32
Number Negative: 42

AUC: 0.898

Operating Point:
 Threshold: 0.92
Sensitivity: 0.94
Specificity: 0.86

PPV: 0.83
NPV: 0.95

# FIG. 41

# FIG. 42

# FIG. 43

# FIG. 44

# FIG. 45

**Feature Importance: prostate - ANY2**

# FIG. 46

| | Prostate | | | | | | Bladder | | | | Kidney |
|---|---|---|---|---|---|---|---|---|---|---|---|
| | Vascular Invasion | Seminal Vesicle Invasion | Positive Surgical Margin | Perineural Invasion | Lymph Node Positive | Extraprostatic Extension | Grade | Lymph Invasion | Lymph Node Positive | Margins | Grade |
| spreadvelmax | 1 | 61 | 1 | 1 | 1 | 1 | 38 | 58 | 29 | 14 | 54 |
| MGSVmedian | 5 | 1 | 3 | 2 | 2 | 18 | 1 | 1 | 1 | 2 | 1 |
| MGSVstd | 3 | 3 | 5 | 4 | 29 | 7 | 2 | 2 | 2 | 37 | 2 |
| migrationvelMEDIAN | 4 | 18 | 29 | 30 | 18 | 32 | 13 | 21 | 19 | 24 | 19 |
| migrationvelSTD | 16 | 30 | 7 | 14 | 3 | 2 | 3 | 3 | 45 | 7 | 6 |
| FAintensityMEDIAN | 2 | 6 | 2 | 7 | 5 | 9 | 12 | 9 | 26 | 65 | 42 |
| FAintensitySTD | 49 | 24 | 11 | 41 | 4 | 5 | 35 | 51 | 17 | 50 | 43 |
| topFAintensityMEDIAN | 32 | 2 | 4 | 5 | 7 | 4 | 25 | 7 | 49 | 20 | 44 |
| topFAintensitySTD | 63 | 4 | 10 | 54 | 47 | 58 | 58 | 52 | 50 | 52 | 45 |
| FAdistscaleMEDIAN | 10 | 49 | 19 | 10 | 39 | 34 | 62 | 56 | 55 | 3 | 50 |
| RFVnum | 26 | 52 | 59 | 63 | 65 | 57 | 4 | 5 | 28 | 8 | 33 |
| topRFVmedian | 57 | 56 | 13 | 32 | 48 | 30 | 18 | 4 | 20 | 27 | 35 |
| nucleustortSTD | 6 | 65 | 62 | 28 | 62 | 27 | 51 | 47 | 42 | 1 | 30 |
| celltortSTD | 42 | 5 | 12 | 61 | 51 | 53 | 41 | 26 | 24 | 39 | 12 |
| celltortMEDIAN | 45 | 62 | 64 | 3 | 63 | 61 | 43 | 39 | 34 | 19 | 22 |

# FIG. 47

**ANY2**

ANY2

Number Positive: 11
Number Negative: 76

AUC: 0.957

Operating Point:
 Threshold: 0.92
Sensitivity: 1.00
Specificity: 0.95

PPV: 0.73
NPV: 1.00

# FIG. 48

**Full Biomarker Compliment**

**Two biomarkers removed**

ANY2

Number Positive: 11
Number Negative: 76

AUC: 0.957

Operating Point:
 Threshold: 0.92
Sensitivity: 1.00
Specificity: 0.95

PPV: 0.73
NPV: 1.00

ANY2

Number Positive: 11
Number Negative: 76

AUC: 0.934

Operating Point:
 Threshold: 0.47
Sensitivity: 1.00
Specificity: 0.86

PPV: 0.50
NPV: 1.00

# FIG. 49

Full Biomarker Compliment

Bottom five biomarkers removed

ANY2

Number Positive: 11
Number Negative: 76

AUC: 0.957

Operating Point:
 Threshold: 0.92
 Sensitivity: 1.00
 Specificity: 0.95

PPV: 0.73
NPV: 1.00

ANY2

Number Positive: 11
Number Negative: 76

AUC: 0.964

Operating Point:
 Threshold: 0.82
 Sensitivity: 1.00
 Specificity: 0.95

PPV: 0.73
NPV: 1.00

# FIG. 50

| Feature Rankings | | | | | |
|---|---|---|---|---|---|
| 1 | spreadvelmax | 2 | MGSVmedian | 3 | FAintensitySTD |
| 4 | cellareaSTD | 5 | FAintensityMEDIAN | | | |

# FIG. 51

# FIG. 52

**Extraprostaticextension**

# FIG. 53

**Microfluidic Device**

A    Direction of flow

Cell Imaging
Chambers

B

# FIG. 54

1 ) Collagen I (CI)
2 ) Fibronectin (F)
3) Laminin (L)
4) Collagen IV (CIV)
5) CI + F
6) CI + L
7) CI + CIV
8) F + L

9) F + CIV
10) L + CIV
11) CI + F + L
12)CI + F + CIV
13) CIV + F + L
14)L + CI + CIV
15) Silane Glass
16)Polylysine

Error Bars=95% CI

# FIG. 55

1 ) Collagen I (CI)
2 ) Fibronectin (F)
3) Laminin (L)
4) Collagen IV (CIV)
5) CI + F
6) CI + L
7) CI + CIV
8) F + L

9) F + CIV
10) L + CIV
11) CI + F + L
12)CI + F + CIV
13) CIV + F + L
14)L + CI + CIV
15) Silane Glass
16)Polylysine

Error Bars=95% CI

# FIG. 56

## A

## B

Prostate cells
    ECM/Silane p=4.91e-5
    ECM/ polylysine p=1.9e-3

Kidney cells
    ECM/Silane p=7.21e-5
    ECM/polylysine p=1.71e-4

Bladder cells
    ECM/Silane p=1.51e-4
    ECM/polylysine p=7.5e-3

# FIG. 57

1 ) Collagen I (CI)
2 ) Fibronectin (F)
3) Laminin (L)
4) CI + F
5) Silane Glass
6) CI + F + L
7) F + L
8) CI + L

# FIG. 58

**A**

Surgical
Margin (SM)

**B**

Seminal Vesicle
Invasion (SVI)

**C**

Extra prostatic
extension (EPE)

**D**

Perineural
Invasion (PNI)

**E**

Lymph Node
invasion (LI)

**F**

ANY 2
pathologies

# FIG. 59

# FIG. 59 (cont'd)

**Feature Importance – LAPP Score**

C

# FIG. 59 (cont'd)

## C (cont'd)

Feature Importance - Most Important

1: MGSVmedian - Importance: 2.35

2: spreadVelmax - Importance: 2.06

3: FAintensityMEDIAN - Importance: 2.04

4: topFAintensityMEDIAN - Importance: 1.32

5: MGSVstd - Importance: 1.12

6: P11 - Importance: 0.82

7: P10 - Importance: 0.75

8: MP2 - Importance: 0.75

9: nucleusperimMEDIAN - Importance: 0.74

10: FAdistSTD - Importance: 0.73

Feature Importance - Least Important

65: RRstd - Importance: 0.28

64: RRnum - Importance: -0.15

63: RRmedian - Importance: -0.05

62: P6 - Importance: 0.12

61: topRFVmedian - Importance: 0.19

60: nucleusaspectSTD - Importance: 0.19

59: FAintensitySTD - Importance: 0.21

58: topRFVstd - Importance: 0.24

57: P12 - Importance: 0.28

56: cellaspectMEDIAN - Importance: 0.28

# FIG. 59D

Feature Importance –MAPP Score

# FIG. 59D

## D (cont'd)

| Feature Importance - Most Important | Feature Importance - Least Important |
|---|---|
| 1 spreadveimar - Importance: 2.08 | 65: P5 - Importance: -0.19 |
| 2 MGSVmedian - Importance: 1.35 | 64: topRRstd - Importance: -0.09 |
| 3 FAintensityMEDIAN - Importance: 1.28 | 63: topFAdistSTD - Importance: 0.07 |
| 4 MGSVstd - Importance: 1.16 | 62: RRstd - Importance: 0.07 |
| 5 FAdistscaleMEDIAN - Importance: 1.15 | 61: cellaspectSTD - Importance: 0.10 |
| 6 topFAintensityMEDIAN - Importance: 1.00 | 60: FAintensitySTD - Importance: 0.17 |
| 7 P13 - Importance: 0.72 | 59: nucleusareaSTD - Importance: 0.17 |
| 8 P15 - Importance: 0.69 | 58: FAdistscaleSTD - Importance: 0.18 |
| 9 P10 - Importance: 0.64 | 57: FAdistSTD - Importance: 0.19 |
| 10: cellperimMEDIAN - Importance: 0.53 | 56: FAstd - Importance: 0.20 |

# FIG. 60

# FIG. 60 (cont'd)

B

# FIG. 61A

All Data
outputsSELECT1942 frozen repeat.mat
outputsSELECT194.mat

# FIG. 61B

outputsSELECT1942 frozen repeat.mat
outputsSELECT194.mat

# FIG. 61C

## Sample level output-Binary

# FIG. 61C
## (cont'd)

Sample level output

outputSELECT194.mat
outputSELECT1942 frozen repeat.mat

## FIG. 62A

# FIG. 62B

# FIG. 62C

Sample level output-Binary

# FIG. 62C
# (cont'd)

Sample level output

## FIG. 63A

## FIG. 63B

# FIG. 63C

### Sample level output-Binary

# FIG. 63C (cont'd)

Sample level output

# FIG. 64

A

Metastatic Adverse Pathology Potential (MAPP)

Local Adverse Pathology Potential (LAPP)

Legend:
- Negative for all adverse pathologies
- Positive for any adverse pathology
- Negative for BCR (6 month PSA < 0.2 ng/mL) NN=15
- Positive for BCR (6 month PSA >= 0.2 ng/mL) NP=1

B

| Path. Finding | Total number (n) | Total Positive | Total negative | Operating Point Threshold | Sensitivity | Specificity | Area Under Curve (AUC) |
|---|---|---|---|---|---|---|---|
| BCR (6 month_PSA) | 25 | 2 | 23 | 0.85 | 1.00 | 1.00 | 1.00 |

EP 3 262 417 B1

# FIG. 64 (cont'd)

C

**noBCR6mo**

% True Positive

% False Positive

**Sample Level**

% True Negative

% False Negative

noBCR6mo

Number Positive: 21
Number Negative: 2

AUC: 0.952

Operating Point:
 Threshold: 0.89
Sensitivity: 0.90
Specificity: 1.00

PPV: 1.00
NPV: 0.50

122

# FIG. 65

| Rank order: Most important Features | Local Adverse Pathology Potential (LAPP) | Biochemical Recurrence (BCR) Potential | Metastatic Adverse Pathology Potential (MAPP) |
|---|---|---|---|
| 1 | MGSV median | MGSV median | spreadvelMax |
| 2 | spreadvelMax | FAintensity Median | MGSV median |
| 3 | FAintensity Median | P10 | FAintensity Median |
| 4 | topFAintensityMedian | P11 | MGSVstd |
| 5 | MGSVstd | OP2 | FAdistscaleMedian |
| 6 | P11 | cellareaMedian | topFAintensityMedian |
| 7 | P10 | P14 | P13 |
| 8 | MP2 | P9 | P19 |
| 9 | NucleusperimMedian | MigrationvelSTD | P10 |
| 10 | FAdistSTD | P4 | cellperimMedian |

# FIG. 66

A

B

grade

Full Trained classification method

grace

Number Positive: 72
Number Negative: 218

AUC: 0.996

Operating Point
Threshold: 0.39
Sensitivity: 0.97
Specificity: 0.98

PPV: 0.93
NPV: 0.99

% True Positive

% False Positive

% True Negative

% False Negative

# FIG. 66 (cont'd)

C

1= Predicted Normal cell
2= Predicted Cancer cell

# FIG. 67

Field Biopsy

Cancer

Biopsy

PROSTATE

# FIG. 68

# FIG. 69

Number Positive: 148

Number Negative: 29

AUC: 0.923

GAPP = 33

Sensitivity: 0.84

Specificity: 0.97

PPV: 0.99

NPV: 0.55

# FIG. 70A

| Predicted Adverse Pathology | (n) number + of 178 | LAPP | MAPP | Sensitivity | Specificity | AUC |
|---|---|---|---|---|---|---|
| ANY | 148 | 33 (GAPP) | - | 0.84 | 0.97 | 0.92 |
| ANY OP | 91 | 80 | | 0.78 | 0.81 | 0.84 |
| ANY MP | 129 | - | 59 | 0.83 | 0.88 | 0.91 |
| Gleason | 24 | 53 | - | 0.92 | 0.91 | 0.94 |
| PSM | 55 | 54 | - | 0.87 | 0.79 | 0.85 |
| SVI | 23 | 51 | - | 0.96 | 0.84 | 0.92 |
| EPE | 66 | 36 | - | 0.80 | 0.78 | 0.85 |
| PNI | 126 | - | 67 | 0.83 | 0.96 | 0.95 |
| VI | 14 | - | 69 | 1.0 | 0.89 | 0.93 |
| LNP | 11 | - | 85 | 1.0 | 0.98 | 0.99 |

# FIG. 70B

| Predicted Adverse Pathology | (n) number + Of 31 | LAPP | MAPP | Sensitivity | Specificity | AUC |
|---|---|---|---|---|---|---|
| ANY | 30 | 87 | - | 1.00 | 1.00 | 1.00 |
| ANY OP | 18 | 74 | | 0.89 | 0.92 | 0.93 |
| ANY MP | 28 | - | 62 | 1.00 | 1.00 | 1.00 |
| PSM | 11 | 36 | - | 0.91 | 0.95 | 0.96 |
| SVI | 4 | 96 | - | 1.0 | 0.85 | 0.92 |
| EPE | 14 | 30 | - | 0.93 | 0.94 | 0.96 |
| PNI | 28 | - | 98 | 1.0 | 1.0 | 1.0 |
| VI | 3 | - | 92 | 1.0 | 1.0 | 1.0 |
| LNI | 1 | - | 95 | 1.0 | 1.0 | 1.0 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012048269 A2
- WO 20131811127 A1
- US 20130149724
- US 20130237453
- US 1461782 W
- WO 2015061458 A1

**Non-patent literature cited in the description**

- **C. M. BISHOP.** Pattern Recognition and Machine Learning. Springer, 2007